(19) Europäisches Patentamt / European Patent Office / Office européen des brevets

(11) **EP 4 714 953 A1**

(12) **EUROPEAN PATENT APPLICATION**
published in accordance with Art. 153(4) EPC


(43) Date of publication:
**25.03.2026 Bulletin 2026/13**

(21) Application number: **24807217.5**

(22) Date of filing: **15.05.2024**

(51) International Patent Classification (IPC):
***C07D 487/04*** (2006.01) ***A61K 31/4985*** (2006.01)
***A61P 3/00*** (2006.01) ***A61P 3/04*** (2006.01)
***A61P 3/10*** (2006.01) ***A61P 5/00*** (2006.01)
***A61P 5/38*** (2006.01) ***A61P 9/00*** (2006.01)
***A61P 9/10*** (2006.01) ***A61P 9/12*** (2006.01)
***A61P 15/00*** (2006.01) ***A61P 19/10*** (2006.01)
***A61P 25/00*** (2006.01) ***A61P 25/24*** (2006.01)
***A61P 27/02*** (2006.01) ***A61P 27/06*** (2006.01)
***A61P 27/12*** (2006.01) ***A61P 31/00*** (2006.01)
***A61P 43/00*** (2006.01)

(52) Cooperative Patent Classification (CPC):
**A61K 31/4985; A61P 3/00; A61P 3/04; A61P 3/10; A61P 5/00; A61P 5/38; A61P 9/00; A61P 9/10; A61P 9/12; A61P 15/00; A61P 19/10; A61P 25/00; A61P 25/24; A61P 27/02; A61P 27/06;** (Cont.)

(86) International application number:
**PCT/JP2024/017921**

(87) International publication number:
**WO 2024/237266 (21.11.2024 Gazette 2024/47)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **16.05.2023 JP 2023080580**



(71) Applicant: **Scohia Pharma, Inc.**
**Fujisawa-shi, Kanagawa 251-8555 (JP)**

(72) Inventors:
- **MATSUMOTO, Shigemitsu**
  **Fujisawa-shi, Kanagawa 251-8555 (JP)**
- **NOGUCHI, Naoyoshi**
  **Fujisawa-shi, Kanagawa 251-8555 (JP)**
- **SASAKI, Satoshi**
  **Fujisawa-shi, Kanagawa 251-8555 (JP)**
- **IKOMA, Minoru**
  **Fujisawa-shi, Kanagawa 251-8555 (JP)**
- **FUKUSHI, Hideto**
  **Fujisawa-shi, Kanagawa 251-8555 (JP)**
- **HARA, Ryoma**
  **Fujisawa-shi, Kanagawa 251-8555 (JP)**
- **ASANO, Kohei**
  **Fujisawa-shi, Kanagawa 251-8555 (JP)**
- **WATANABE, Koji**
  **Fujisawa-shi, Kanagawa 251-8555 (JP)**
- **OGINO, Masaki**
  **Fujisawa-shi, Kanagawa 251-8555 (JP)**
- **KASAI, Shizuo**
  **Fujisawa-shi, Kanagawa 251-8555 (JP)**
- **MAEKAWA, Tsuyoshi**
  **Fujisawa-shi, Kanagawa 251-8555 (JP)**
- **HIROSE, Hideki**
  **Fujisawa-shi, Kanagawa 251-8555 (JP)**

(74) Representative: **J A Kemp LLP**
**80 Turnmill Street**
**London EC1M 5QU (GB)**


(54) **MELANOCORTIN 2-TYPE RECEPTOR ANTAGONIST**


(57) The present invention discloses, as one embodiment of the invention, heterocyclic compound (I) represented by the following formula (I):


EP 4 714 953 A1

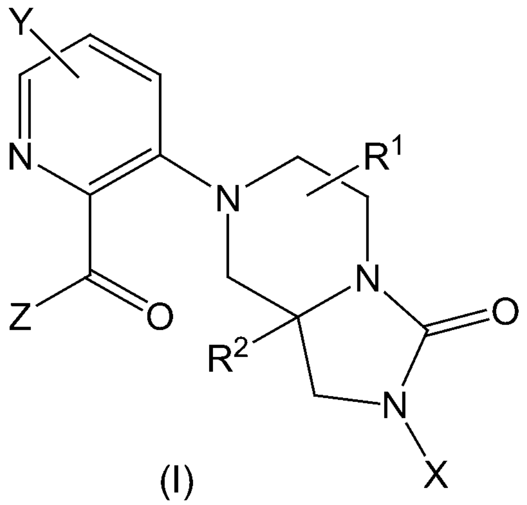

(I)

wherein each symbol is as defined in the specification, or a salt thereof, which has an antagonist activity against melanocortin 2 receptor and useful for the treatment or prophylaxis of endocrine diseases and the like.

(52) Cooperative Patent Classification (CPC): (Cont.)
**A61P 27/12; A61P 31/00; A61P 43/00; C07D 487/04**

## Description

[Technical Field]

The present invention relates to heterocyclic compounds, as an embodiment thereof, having antagonist activity against melanocortin 2 receptor (MC2R) and useful for the treatment or prevention of conditions, diseases, and disorders in response to MC2R antagonistic action, such as endocrine diseases (e.g., Cushing's disease, ectopic ACTH syndrome, ectopic CRH syndrome, congenital adrenal hyperplasia, polycystic ovary syndrome), metabolic diseases (e.g., obesity, type 2 diabetes, dyslipidemia, osteoporosis, or sarcopenia), cardiovascular diseases (e.g., hypertension, arteriosclerosis), central nervous system diseases (e.g., depression, dementia, insomnia), eye diseases (e.g., cataract, glaucoma), infectious diseases, and the like, and is useful in the pharmaceutical field.

[Background Art]

The hypothalamic-pituitary-adrenal (HPA) system is responsible for the biological response to stress, and is an essential neuroendocrine mechanism for protecting the living body from stress and maintaining homeostasis. When corticotropin-releasing factor (CRF) is secreted from CRF-producing neurons in the paraventricular nucleus of the hypothalamus in response to stress stimulation, CRF passes through the pituitary portal vein, acts on adrenocorticotropic hormone-producing cell to promote the synthesis and secretion of adrenocorticotropic hormone (ACTH), which consists of 39 amino acids. ACTH secreted from the anterior pituitary gland acts on the adrenal cortex via the bloodstream, enhances the secretion of adrenal cortical hormones such as glucocorticoids, and regulates the metabolic and immune responses of the whole body, thereby inducing biological responses to stress. Furthermore, glucocorticoids also play a role in terminating the activation of the HPA system through a negative feedback mechanism that acts on the hypothalamus and anterior pituitary gland to suppress ACTH secretion. The HPA system is an important physiological response for the body to adapt to external environmental stimulation. On the other hand, failure of its function, such as excessive activation of the HPA system, is considered to be deeply involved in the manifestation of various systemic pathologies.

ACTH secreted from the anterior pituitary gland exerts its physiological actions by selectively binding to ACTH receptors. ACTH receptor is composed of MC2R, which is a seven-transmembrane G protein-coupled receptor, and melanocortin-2 receptor accessory protein-1 (MRAP). When ACTH binds to the receptor, adenylate cyclase is activated via G$\alpha$s and intracellular cyclic AMP increases, thereby transmitting a downstream signal into the cell. Since the expression of MC2R is localized to the adrenal glands, and the only endogenous ligand for MC2R is ACTH, the ACTH-MC2R/MRAP signal is considered to be a specific and essential pathway for steroid synthesis in the adrenal glands. Therefore, selective antagonists of this receptor can selectively block ACTH signal in the adrenal glands, and are expected to exert therapeutic or preventive effects on conditions, diseases, and disorders caused by excessive ACTH signals.

Under such circumstances, attempts have been made in this field to create MC2R antagonist (Patent Literature 1; Non Patent Literature 1). However, the effectiveness and safety thereof are still not sufficient, and the creation of a more useful MC2R antagonist has been awaited.

[Citation List]

[Patent Literature]

[Patent Literature 1]
WO 2019/236699

[Non Patent Literature]

[Non Patent Literature 1]
Front Endocrinol (Lausanne). 2016 Aug 5; 7:101. doi: 10.3389/fendo.2016.00101. PMID: 27547198; PMCID: PMC4974254.

[Summary of Invention]

[Technical Problem]

In an attempt to solve the above-mentioned problems, the present invention aims to provide, as an embodiment thereof, novel heterocyclic compounds having MC2R antagonist activity, and the like.

[Solution to Problem]

The present inventors have found that a heterocyclic compound represented by the following formula (I) or a salt thereof has MC2R antagonist activity, conducted further studies, and completed the present invention. Specific embodiments of the present invention are as follows; however, the present invention is not limited to them.

[1] A compound represented by the following formula (I):

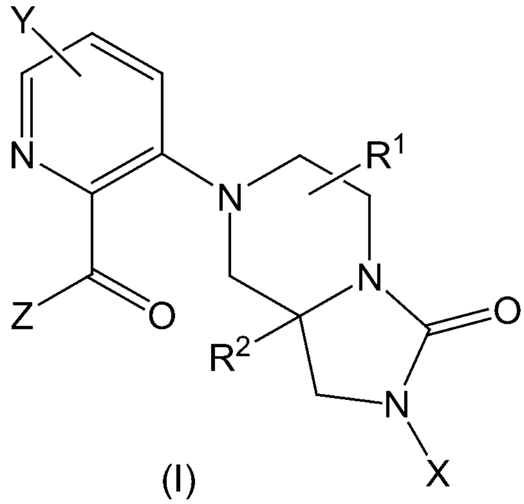

(I)

wherein

$R^1$ is a hydrogen atom or an optionally substituted $C_{1-6}$ alkyl group,
$R^2$ is a hydrogen atom or an optionally substituted $C_{1-6}$ alkyl group,
X is an optionally substituted aryl group, an optionally substituted aromatic heterocyclic group, or an optionally substituted saturated hydrocarbon ring group,
Y is an optionally substituted aromatic heterocyclic group or an optionally substituted aryl group, and
Z is a hydrogen atom, a hydroxy group, $NH_2$, or $NHR^3$
wherein $R^3$ is an optionally substituted $C_{1-6}$ alkyl group or an optionally substituted saturated ring group (hereinafter to be also denoted as "compound (I)"),
or a salt thereof.

[2] The compound of the above-mentioned [1], wherein X is an optionally substituted aryl group, an optionally substituted nitrogen-containing aromatic heterocyclic group, or an optionally substituted saturated hydrocarbon ring group, and Y is an optionally substituted nitrogen-containing aromatic heterocyclic group or an optionally substituted aryl group, or a salt thereof.
[3] The compound of the above-mentioned [1] or [2], wherein X is an optionally substituted $C_{6-14}$ aryl group, an optionally substituted 5- to 14-membered (preferably, 5- or 6-membered) nitrogen-containing aromatic heterocyclic group, or an optionally substituted saturated hydrocarbon ring group, and Y is an optionally substituted 5- to 14-membered (preferably, 5- or 6-membered) nitrogen-containing aromatic heterocyclic group or an optionally substituted $C_{6-14}$ aryl group, or a salt thereof.
[4] The compound of any of the above-mentioned [1] to [3], wherein X is an optionally substituted phenyl group, an optionally substituted pyridyl group, or an optionally substituted saturated hydrocarbon ring group, and

Y is an optionally substituted pyridyl group or an optionally substituted phenyl group,
or a salt thereof.

[5] The compound of any of the above-mentioned [1] to [4],

wherein X is an optionally substituted phenyl group or an optionally substituted saturated hydrocarbon ring group, and
Y is an optionally substituted pyridyl group or an optionally substituted phenyl group,
or a salt thereof.

[6] The compound of any of the above-mentioned [3] to [5],
wherein the "optionally substituted saturated hydrocarbon ring group" for X is an "optionally substituted $C_{3-10}$ cycloalkyl group", or a salt thereof.
[7] The compound of any of the above-mentioned [1] to [6], wherein X is an optionally substituted phenyl group or an optionally substituted $C_{3-10}$ cycloalkyl group,
or a salt thereof.
[8] The compound of any of the above-mentioned [1] to [7], wherein Z is a hydrogen atom, a hydroxy group, $NH_2$, or $NHR^3$ wherein $R^3$ is an optionally substituted $C_{1-6}$ alkyl group or an optionally substituted 3- to 8-membered saturated ring group, or a salt thereof.
[9] The compound of any of the above-mentioned [1] to [8], wherein $R^1$ is a hydrogen atom or a $C_{1-6}$ alkyl group,

$R^2$ is a hydrogen atom or a $C_{1-6}$ alkyl group,
X is a phenyl group optionally substituted by 1 to 3 substituents selected from 1) a halogen atom, 2) a $C_{1-6}$ alkyl group optionally substituted by 1 to 3 halogen atoms, and 3) a cyano group, or
a $C_{3-10}$ cycloalkyl group optionally substituted by 1 to 3 $C_{1-6}$ alkyl groups optionally substituted by 1 to 3 halogen atoms,
Y is a pyridyl group optionally substituted by 1 to 3 $C_{1-6}$ alkoxy groups, or a phenyl group optionally substituted by 1 to 3 $C_{1-6}$ alkoxy groups, and
Z is a hydrogen atom, a hydroxy group, $NH_2$, or
$NHR^3$
wherein $R^3$ is

(i) a $C_{1-6}$ alkyl group optionally substituted by 1 to 3 substituents selected from

a hydroxy group,
a $C_{1-6}$ alkoxy group,
a (mono- or di-$C_{1-6}$ alkyl)amino group,
a $C_{3-10}$ cycloalkyl group,
a 5- or 6-membered monocyclic aromatic heterocyclic group,
a 3- to 8-membered monocyclic non-aromatic heterocyclic group optionally substituted by a $C_{1-6}$ alkoxy-carbonyl group,
a $C_{1-6}$ alkyl-carbonylamino group,
a (mono- or di-$C_{1-6}$ alkylamino)carbonyl group, and
a $C_{1-6}$ alkyl-sulfonyl group, or

(ii) a 3- to 8-membered monocyclic saturated ring group optionally substituted by 1 to 3 substituents selected from

a $C_{1-6}$ alkyl group,
a $C_{1-6}$ alkoxy-carbonyl group, and
an oxo group
(preferably, the optionally substituted 3- to 8-membered monocyclic saturated heterocyclic group),
or a salt thereof.

[10] The compound of any of the above-mentioned [1] to [9],

wherein X is a phenyl group optionally substituted by 1 to 3 substituents selected from 1) a halogen atom, 2) a $C_{1-6}$ alkyl group optionally substituted by 1 to 3 halogen atoms, and 3) a cyano group, and
Y is a pyridyl group optionally substituted by 1 to 3 $C_{1-6}$ alkoxy groups,
or a salt thereof.

[11] The compound of any of the above-mentioned [1] to [10], wherein $R^1$ is a $C_{1-6}$ alkyl group, and

$R^2$ is a hydrogen atom,
or a salt thereof.

[12] The compound of any of the above-mentioned [1] to [11],

wherein Z is a hydrogen atom, a hydroxy group, $NH_2$, or $NHR^3$
wherein $R^3$ is

(i) a $C_{1-6}$ alkyl group optionally substituted by 1 to 3 substituents selected from

a hydroxy group,
a $C_{1-6}$ alkoxy group,
a (mono- or di-$C_{1-6}$ alkyl)amino group,
a $C_{3-10}$ cycloalkyl group,
a 5- or 6-membered monocyclic nitrogen-containing aromatic heterocyclic group,
a 3- to 8-membered monocyclic oxygen-containing non-aromatic heterocyclic group,
a 3- to 8-membered monocyclic nitrogen-containing non-aromatic heterocyclic group optionally substituted by a $C_{1-6}$ alkoxy-carbonyl group,
a $C_{1-6}$ alkyl-carbonylamino group,
a (mono- or di-$C_{1-6}$ alkylamino)carbonyl group, and
a $C_{1-6}$ alkyl-sulfonyl group, or

(ii)

(a) a 3- to 8-membered monocyclic oxygen-containing saturated heterocyclic group, or
(b) a 3- to 8-membered monocyclic nitrogen-containing saturated heterocyclic group optionally substituted by 1 to 3 substituents selected from

a $C_{1-6}$ alkyl group,
a $C_{1-6}$ alkoxy-carbonyl group, and
an oxo group,
or a salt thereof.

[13] The compound of any of the above-mentioned [1] to [12],

wherein Z is a hydroxy group or $NHR^3$
wherein $R^3$ is

(a) a 3- to 8-membered monocyclic oxygen containing saturated heterocyclic group, or
(b) a 3- to 8-membered nitrogen-containing saturated heterocyclic group optionally substituted 1 to 3 substituents selected from

a $C_{1-6}$ alkyl group,
a $C_{1-6}$ alkoxy-carbonyl group, and
an oxo group,
or a salt thereof.

[14] The compound of any of the above-mentioned [1] to [13],

wherein $R^1$ is a $C_{1-6}$ alkyl group,
$R^2$ is a hydrogen atom,
X is a phenyl group optionally substituted by 1 to 3 substituents selected from 1) a halogen atom, 2) a $C_{1-6}$ alkyl group optionally substituted by 1 to 3 halogen atoms, and 3) a cyano group,
Y is a pyridyl group optionally substituted by 1 to 3 $C_{1-6}$ alkoxy groups, and
Z is a hydroxy group, or $NHR^3$
wherein $R^3$ is a pyrrolidinyl group optionally substituted by 1 to 3 substituents selected from a $C_{1-6}$ alkyl group and an oxo group,
or a salt thereof.

[15] The compound of any of the above-mentioned [1] to [14], wherein compound (I) has a structure represented by the following formula (Ia):

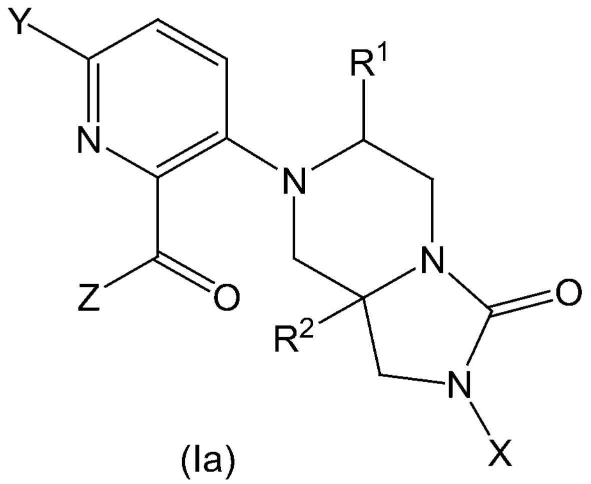

(Ia)

,

or a salt thereof.

[16] The compound of the above-mentioned [1] or a salt thereof, which is selected from

3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-(2-ethoxypyridin-3-yl)-N-[(3R)-1-methyl-5-oxopyrrolidin-3-yl]pyridine-2-carboxamide (compound of Example 49), 3-[(6R,8aS)-6-ethyl-2-[4-fluoro-2-(trifluoromethyl)phenyl]-3-oxo-5,6,8,8a-tetrahydro-1H-imidazo[1,5-a]pyrazin-7-yl]-6-(2-ethoxypyridin-3-yl)-N-[(3R)-1-methyl-5-oxopyrrolidin-3-yl]pyridine-2-carboxamide (compound of Example 52), 3-[(6R,8aS)-2-(4-chloro-2-cyanophenyl)-6-ethyl-3-oxo-5,6,8,8a-tetrahydro-1H-imidazo[1,5-a]pyrazin-7-yl]-6-(2-ethoxypyridin-3-yl)-N-[(3R)-1-methyl-5-oxopyrrolidin-3-yl]pyridine-2-carboxamide (compound of Example 54), 3-[(6R,8aS)-2-[4-chloro-2-(trifluoromethyl)phenyl]-6-methyl-3-oxo-5,6,8,8a-tetrahydro-1H-imidazo[1,5-a]pyrazin-7-yl]-6-(2-methoxy-3-pyridinyl)-N-[(3R)-1-methyl-5-oxopyrrolidin-3-yl]pyridine-2-carboxamide (compound of Example 61), 3-[(6R,8aS)-2-[4-chloro-2-(trifluoromethyl)phenyl]-6-methyl-3-oxo-5,6,8,8a-tetrahydro-1H-imidazo[1,5-a]pyrazin-7-yl]-6-(2-methoxyphenyl)-N-[(3R)-1-methyl-5-oxopyrrolidin-3-yl]pyridine-2-carboxamide (compound of Example 64), or
a salt of each compound mentioned above.

[17] A medicament comprising the compound of any of the above-mentioned [1] to [16], or a salt thereof as an active ingredient.

[18] The medicament of the above-mentioned [17], which is an MC2R antagonist.

[19] The medicament of the above-mentioned [17] or [18], which is a prophylactic or therapeutic agent for endocrine disease, metabolic disease, cardiovascular disease, central nervous system disease, eye disease, and/or infectious disease.

[20] The medicament of any of the above-mentioned [17] to [19], which is a prophylactic or therapeutic agent for Cushing's disease, ectopic ACTH syndrome, ectopic CRH syndrome, congenital adrenal hyperplasia, polycystic ovary syndrome, obesity, type 2 diabetes, dyslipidemia, osteoporosis, sarcopenia, hypertension, arteriosclerosis, depression, dementia, insomnia, cataract, glaucoma and/or infectious disease.

[21] A method for the prophylaxis and/or treatment of endocrine disease, metabolic disease, cardiovascular disease, central nervous system disease, eye disease, and/or infectious disease, comprising administering an effective amount of the compound of any of the above-mentioned [1] to [16], or a salt thereof to a mammal in need of the administration.

[22] The compound of any of the above-mentioned [1] to [16] or a salt thereof, which is for use for the prophylaxis and/or treatment of endocrine disease, metabolic disease, cardiovascular disease, central nervous system disease, eye disease, and/or infectious disease.

[23] Use of the compound of any of the above-mentioned [1] to [16] or a salt thereof in the production of a medicament for the prophylaxis and/or treatment of endocrine disease, metabolic disease, cardiovascular disease, central nervous system disease, eye disease, and/or infectious disease.

[Advantageous Effects of Invention]

According to the present invention, heterocyclic compounds having antagonist activity against MC2R and useful for the treatment or prevention of conditions, diseases, or disorders in response to MC2R antagonistic action, such as

endocrine diseases (e.g., Cushing's disease, ectopic ACTH syndrome, ectopic CRH syndrome, congenital adrenal hyperplasia, polycystic ovary syndrome), metabolic diseases (e.g., obesity, type 2 diabetes, dyslipidemia, osteoporosis, or sarcopenia), cardiovascular diseases (e.g., hypertension, arteriosclerosis), central nervous system diseases (e.g., depression, dementia, insomnia), eye diseases (e.g., cataract, glaucoma), infectious diseases, and the like, and salts thereof are provided as one of the embodiments thereof.

[Description of Embodiments]

The present invention is described in detail below. Unless otherwise specified in the text, all technical terms and scientific terms used in the present specification have the same meaning as those generally understood by a person skilled in the art to which the present invention pertains. Any methods and materials similar or equivalent to those described in the present specification can be used in the practice or testing of the present invention, and preferred methods and materials are described below. All publications and patents mentioned in the present specification are incorporated herein by reference, for the purpose of, for example, describing and disclosing the constructs and methodologies described in the publications that may be used in connection with the described invention.

[Compound (I)]

According to the present invention, as one embodiment thereof, "a compound represented by the following formula (I):

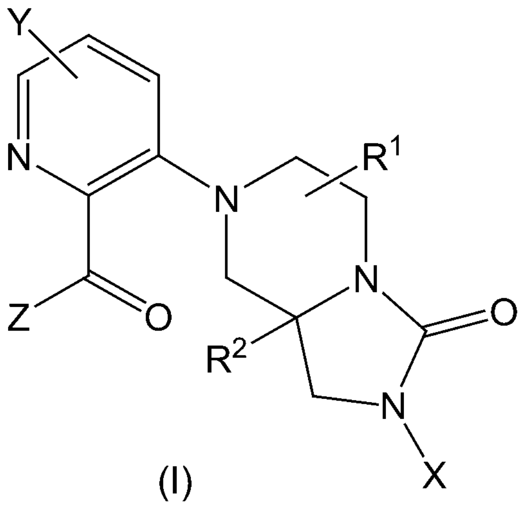

(I)

wherein

$R^1$ is a hydrogen atom or an optionally substituted $C_{1-6}$ alkyl group,
$R^2$ is a hydrogen atom or an optionally substituted $C_{1-6}$ alkyl group,
X is an optionally substituted aryl group, an optionally substituted aromatic heterocyclic group, or an optionally substituted saturated hydrocarbon ring group,
Y is an optionally substituted aromatic heterocyclic group or an optionally substituted aryl group, and
Z is a hydrogen atom, a hydroxy group, $NH_2$, or $NHR^3$
wherein $R^3$ is an optionally substituted $C_{1-6}$ alkyl group or an optionally substituted saturated ring group,
or a salt thereof"
is provided.

Each group of the above-mentioned compound (I) is explained below. In the following, each group (e.g., "$C_{1-6}$ alkyl group") is explained. When the group constitutes a part of another group (e.g., "$C_1$-$C_6$ alkyl-carbonyloxy group"), unless otherwise specified, the explanation for each group can be referred to for corresponding portions.
In the present specification, the notation "$C_{a-b}$" (e.g., $C_{1-6}$) or "$C_a$-$C_b$" (e.g., $C_1$-$C_6$) shows that the number of carbon atoms constituting the group is a to b (e.g., 1 to 6).
In the present specification, examples of the "halogen atom" include fluorine, chlorine, bromine, and iodine.
In the present specification, examples of the "$C_{1-6}$ alkyl group" include methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, and 2-ethylbutyl.

In the present specification, examples of the "$C_{1-6}$ alkoxy group" include methoxy, ethoxy, propoxy, isopropoxy, butoxy, isobutoxy, sec-butoxy, tert-butoxy, pentyloxy, and hexyloxy.

In the present specification, the "aryl group" means a monocyclic or polycyclic unsaturated hydrocarbon group, and examples thereof include $C_{6-14}$ aryl groups. As the specific example thereof, phenyl, 1-naphthyl, 2-naphthyl, 1-anthryl, 2-anthryl, and 9-anthryl can be mentioned. Among these, $C_{6-10}$ aryl groups are preferred.

In the present specification, examples of the "saturated hydrocarbon ring group" include monocyclic or bicyclic $C_{3-10}$ cycloalkyl groups. As specific example thereof, cyclopropyl, cyclobutyl, cyclopentyl, cyclohexyl, cycloheptyl, cyclooctyl, cyclononyl, cyclodecyl, bicyclo[2.2.1]heptyl, bicyclo[3.2.1]octyl, bicyclo[5.2.0]nonyl, decahydronaphthyl, and the like can be mentioned. Among these, a $C_{3-8}$ cycloalkyl group is preferred, and a $C_{3-6}$ cycloalkyl group is more preferred.

In the present specification, examples of the "heterocyclic group" include (i) an aromatic heterocyclic group and (ii) a non-aromatic heterocyclic group, each containing, as a ring-constituting atom besides carbon atom, 1 to 4 hetero atoms selected from a nitrogen atom, a sulfur atom, and an oxygen atom.

In the present specification, examples of the "aromatic heterocyclic group" include a 5- to 14-membered (preferably 5-to 10-membered) aromatic heterocyclic group containing, as a ring-constituting atom besides carbon atom, 1 to 4 hetero atoms selected from a nitrogen atom, a sulfur atom and an oxygen atom.

Preferred examples of the "aromatic heterocyclic group" include 5- or 6-membered monocyclic aromatic heterocyclic groups such as thienyl, furyl, pyrrolyl, imidazolyl, pyrazolyl, thiazolyl, isothiazolyl, oxazolyl, isoxazolyl, pyridyl, pyrazinyl, pyrimidinyl, pyridazinyl, 1,2,4-oxadiazolyl, 1,3,4-oxadiazolyl, 1,2,4-thiadiazolyl, 1,3,4-thiadiazolyl, triazolyl, tetrazolyl, triazinyl, and the like; and 8- to 14-membered fused polycyclic (preferably bi or tricyclic) aromatic heterocyclic groups such as benzothiophenyl, benzofuranyl, benzimidazolyl, benzoxazolyl, benzisoxazolyl, benzothiazolyl, benzisothiazolyl, benzotriazolyl, imidazopyridinyl, thienopyridinyl, furopyridinyl, pyrrolopyridinyl, pyrazolopyridinyl, oxazolopyridinyl, thiazolopyridinyl, imidazopyrazinyl, imidazopyrimidinyl, thienopyrimidinyl, furopyrimidinyl, pyrrolopyrimidinyl, pyrazolopyrimidinyl, oxazolopyrimidinyl, thiazolopyrimidinyl, pyrazolotriazinyl, naphtho[2,3-b]thienyl, phenoxathiinyl, indolyl, isoindolyl, 1H-indazolyl, purinyl, isoquinolyl, quinolyl, phthalazinyl, naphthyridinyl, quinoxalinyl, quinazolinyl, cinnolinyl, carbazolyl, β-carbolinyl, phenanthridinyl, acridinyl, phenazinyl, phenothiazinyl, phenoxazinyl, and the like.

In the present specification, examples of the "non-aromatic heterocyclic group" include a 3- to 14-membered (preferably 4- to 10-membered) non-aromatic heterocyclic group containing, as a ring-constituting atom besides carbon atom, 1 to 4 hetero atoms selected from a nitrogen atom, a sulfur atom and an oxygen atom.

Preferred examples of the "non-aromatic heterocyclic group" include 3- to 8-membered monocyclic non-aromatic heterocyclic groups such as aziridinyl, oxiranyl, thiiranyl, azetidinyl, oxetanyl, thietanyl, tetrahydrothienyl, tetrahydrofuranyl, pyrrolinyl, pyrrolidinyl, imidazolinyl, imidazolidinyl, oxazolinyl, oxazolidinyl, pyrazolinyl, pyrazolidinyl, thiazolinyl, thiazolidinyl, tetrahydroisothiazolyl, tetrahydrooxazolyl, tetrahydroisooxazolyl, piperidinyl, piperazinyl, tetrahydropyridinyl, dihydropyridinyl, dihydrothiopyranyl, tetrahydropyrimidinyl, tetrahydropyridazinyl, dihydropyranyl, tetrahydropyranyl, tetrahydrothiopyranyl, morpholinyl, thiomorpholinyl, azepanyl, diazepanyl, azepinyl, oxepanyl, azocanyl, diazocanyl, and the like; and 9- to 14-membered fused polycyclic (preferably bi or tricyclic) non-aromatic heterocyclic groups such as dihydrobenzofuranyl, dihydrobenzimidazolyl, dihydrobenzoxazolyl, dihydrobenzothiazolyl, dihydrobenzisothiazolyl, dihydronaphtho[2,3-b]thienyl, tetrahydroisoquinolyl, tetrahydroquinolyl, 4H-quinolizinyl, indolinyl, isoindolinyl, tetrahydrothieno[2,3-c]pyridinyl, tetrahydrobenzazepinyl, tetrahydroquinoxalinyl, tetrahydrophenanthridinyl, hexahydrophenothiazinyl, hexahydrophenoxazinyl, tetrahydrophthalazinyl, tetrahydronaphthyridinyl, tetrahydroquinazolinyl, tetrahydrocinnolinyl, tetrahydrocarbazolyl, tetrahydro-β-carbolinyl, tetrahydroacrydinyl, tetrahydrophenazinyl, tetrahydrothioxanthenyl, octahydroisoquinolyl, and the like.

In the present specification, as the "saturated heterocyclic group", a saturated heterocyclic group that does not contain an unsaturated bond can be mentioned from among the above-mentioned "heterocyclic groups".

When the above-mentioned "heterocyclic group" or "saturated heterocyclic group" is specified as "nitrogen-containing" or "oxygen-containing", it means that the "heterocyclic group" or "saturated heterocyclic group" contains, as a ring-constituting atom besides carbon atom, at least one nitrogen atom, or at least one oxygen atom.

In the present specification, as the "saturated ring group", those described above as the "saturated hydrocarbon ring group" and "saturated heterocyclic group" can be mentioned.

In the above, a 3- to 8-membered monocyclic group is preferred, and a "$C_{3-8}$ cycloalkyl group" and a "3- to 8-membered monocyclic saturated heterocyclic group" are more preferred, among which a "$C_{3-6}$ cycloalkyl group" and a "3- to 8-membered monocyclic saturated heterocyclic group containing nitrogen or oxygen as a ring-constituting atom" are more preferred.

In the present specification, examples of the "substituent" in the "optionally substituted group or ring" in the definition of compound (I) such as "optionally substituted $C_{1-6}$ alkyl group" and the like include substituents selected from the following [substituent group A].

The "substituents" in the number of 1 to 5 (preferably 1 to 3) may be present at substitutable positions, and when

the number of substituents is 2 or more, each substituent may be the same or different..

When a special explanation is given regarding the "substituent" for each group or each ring, the explanation should be followed.

[Substituent group A]

(1) a halogen atom,
(2) a nitro group,
(3) a cyano group,
(4) an oxo group,
(5) a hydroxy group,
(6) an optionally halogenated $C_1$-$C_6$ alkoxy group (e.g., methoxy, chloromethoxy, trifluoroethoxy),
(7) a $C_6$-$C_{14}$ aryloxy group (e.g., phenoxy, naphthoxy),
(8) a $C_7$-$C_{16}$ aralkyloxy group (e.g., benzyloxy),
(9) a $C_1$-$C_6$ alkyl-carbonyloxy group (e.g., acetoxy, propanoyloxy),
(10) a $C_6$-$C_{14}$ aryl-carbonyloxy group (e.g., benzoyloxy, 1-naphthoyloxy, 2-naphthoyloxy),
(11) a $C_1$-$C_6$ alkoxy-carbonyloxy group (e.g., methoxycarbonyloxy, ethoxycarbonyloxy, propoxycarbonyloxy, butoxycarbonyloxy),
(12) a 5- to 14-membered aromatic heterocyclic group (e.g., thienyl, furyl, pyrrolyl, imidazolyl, pyrazolyl, thiazolyl, isothiazolyl, oxazolyl, isoxazolyl, pyridyl, pyrazinyl, pyrimidinyl, pyridazinyl), in which a 5- or 6-membered monocyclic aromatic heterocyclic group is preferred, and a 5- or 6-membered monocyclic nitrogen-containing aromatic heterocyclic group is more preferred,
(13) a 3- to 14-membered non-aromatic heterocyclic group (e.g., aziridinyl, oxiranyl, thiiranyl, azetidinyl, oxetanyl, thietanyl, tetrahydrothienyl, tetrahydrofuranyl, pyrrolinyl, pyrrolidinyl, imidazolinyl, imidazolidinyl, oxazolinyl, oxazolidinyl, pyrazolinyl, pyrazolidinyl, thiazolinyl, thiazolidinyl, tetrahydroisothiazolyl, tetrahydrooxazolyl, tetrahydroisoxazolyl, piperidinyl, piperazinyl, tetrahydropyridinyl, dihydropyridinyl, dihydrothiopyranyl, tetrahydropyrimidinyl, tetrahydropyridazinyl, dihydropyranyl, tetrahydropyranyl, tetrahydrothiopyranyl, morpholinyl, thiomorpholinyl, azepanyl, diazepanyl, azepinyl, oxepanyl, azocanyl, diazocanyl), in which a 3- to 8-membered monocyclic non-aromatic heterocyclic group is preferred, a 3- to 8-membered monocyclic nitrogen containing non-aromatic heterocyclic group, or a 3- to 8-membered monocyclic oxygen containing non-aromatic heterocyclic group is more preferred,
(14) a formyl group,
(15) a carboxy group,
(16) an optionally halogenated $C_1$-$C_6$ alkyl-carbonyl group (e.g., acetyl, chloroacetyl, trifluoroacetyl),
(17) a $C_6$-$C_{14}$ aryl-carbonyl group (e.g., benzoyl, 1-naphthoyl, 2-naphthoyl),
(18) a $C_1$-$C_6$ alkoxy-carbonyl group (e.g., methoxycarbonyl, ethoxycarbonyl),
(19) a carbamoyl group,
(20) an amino group,
(21) a mono- or di-$C_1$-$C_6$ alkylamino group (e.g., methylamino, ethylamino, propylamino, isopropylamino, butylamino, dimethylamino, diethylamino, dipropylamino, dibutylamino, N-ethyl-N-methylamino),
(22) a mono- or di-$C_6$-$C_{14}$ arylamino group (e.g., phenylamino),
(23) a formylamino group,
(24) a $C_1$-$C_6$ alkyl-carbonylamino group (e.g., acetylamino, propanoylamino, butanoylamino),
(25) a $C_1$-$C_6$ alkoxy-carbonylamino group (e.g., methoxycarbonylamino, ethoxycarbonylamino, propoxycarbonylamino, butoxycarbonyl amino, tert-butoxycarbonyl amino),
(26) an optionally halogenated $C_1$-$C_6$ alkyl group (e.g., methyl, chloromethyl, difluoromethyl, trifluoromethyl, ethyl, 2-bromoethyl, 2,2,2-trifluoroethyl),
(27) a $C_2$-$C_6$ alkenyl group (e.g., ethenyl, 1-propenyl, 2-propenyl, 2-methyl-1-propenyl, 1-butenyl),
(28) a $C_2$-$C_6$ alkynyl group (e.g., ethynyl, 1-propynyl, 2-propynyl, 1-butynyl, 2-butynyl, 3-butynyl),
(29) a $C_3$-$C_{10}$ cycloalkyl group (e.g., cyclopropyl, cyclobutyl, cyclopentyl, cyclohexyl, cycloheptyl, cyclooctyl, bicyclo[2.2.1]heptyl, bicyclo[2.2.2]octyl, bicyclo[3.2.1]octyl, adamantyl),
(30) a $C_3$-$C_{10}$ cycloalkenyl group (e.g., cyclopropenyl, cyclobutenyl, cyclopentenyl, cyclohexenyl, cycloheptenyl, cyclooctenyl),
(31) a $C_6$-$C_{14}$ aryl group (e.g., phenyl, naphthyl),
(32) a (mono- or di-$C_1$-$C_6$ alkylamino)carbonyl group (e.g., methylamino-carbonyl group), and
(33) a $C_1$-$C_6$ alkyl-sulfonyl group (e.g., methylsulfonyl group).

Preferred embodiments of the groups represented by each symbol in compound (I) are described below.

$R^1$ is present at a substitutable position in the ring to which it is bonded, and is as defined above. It is preferably a hydrogen atom or a $C_{1-6}$ alkyl group, more preferably a $C_{1-6}$ alkyl group.

$R^2$ is as defined above and is preferably a hydrogen atom or a $C_{1-6}$ alkyl group, more preferably a hydrogen atom.

X is as defined above, and is preferably

(1) an optionally substituted aryl group (preferably optionally substituted by 1 to 3 substituents selected from 1) a halogen atom, 2) a $C_{1-6}$ alkyl group optionally substituted by 1 to 3 halogen atoms, and 3) a cyano group), an optionally substituted nitrogen-containing aromatic heterocyclic group (preferably optionally substituted by 1 to 3 substituents selected from 1) a halogen atom, 2) a $C_{1-6}$ alkyl group optionally substituted by 1 to 3 halogen atoms, and 3) a cyano group), or an optionally substituted saturated hydrocarbon ring group (preferably optionally substituted by 1 to 3 $C_{1-6}$ alkyl groups optionally substituted by 1 to 3 halogen atoms),
more preferably
(2) an optionally substituted $C_{6-14}$ aryl group (preferably optionally substituted by 1 to 3 substituents selected from 1) a halogen atom, 2) a $C_{1-6}$ alkyl group optionally substituted by 1 to 3 halogen atoms, and 3) a cyano group), an optionally substituted 5- to 14-membered (preferably, 5- or 6-membered) nitrogen-containing aromatic heterocyclic group (preferably optionally substituted by 1 to 3 substituents selected from 1) a halogen atom, 2) a $C_{1-6}$ alkyl group optionally substituted by 1 to 3 halogen atoms, and 3) a cyano group), or an optionally substituted saturated hydrocarbon ring group (preferably optionally substituted by 1 to 3 $C_{1-6}$ alkyl groups optionally substituted by 1 to 3 halogen atoms) (wherein the saturated hydrocarbon ring group is preferably a $C_{3-10}$ cycloalkyl group), further preferably
(3) an optionally substituted phenyl group (preferably optionally substituted by 1 to 3 substituents selected from 1) a halogen atom, 2) a $C_{1-6}$ alkyl group optionally substituted by 1 to 3 halogen atoms, and 3) a cyano group), an optionally substituted pyridyl group (preferably optionally substituted by 1 to 3 substituents selected from 1) a halogen atom, 2) a $C_{1-6}$ alkyl group optionally substituted by 1 to 3 halogen atoms, and 3) a cyano group), or an optionally substituted saturated hydrocarbon ring group (preferably optionally substituted by 1 to 3 $C_{1-6}$ alkyl groups optionally substituted by 1 to 3 halogen atoms) (wherein the saturated hydrocarbon ring group is preferably a $C_{3-10}$ cycloalkyl group),
still more preferably
(4) an optionally substituted phenyl group (preferably optionally substituted by 1 to 3 substituents selected from 1) a halogen atom, 2) a $C_{1-6}$ alkyl group optionally substituted by 1 to 3 halogen atoms, and 3) a cyano group) or

an optionally substituted saturated hydrocarbon ring group (preferably optionally substituted by 1 to 3 $C_{1-6}$ alkyl groups optionally substituted by 1 to 3 halogen atoms) (wherein the saturated hydrocarbon ring group is preferably a $C_{3-10}$ cycloalkyl group),
especially preferably

(5) an optionally substituted phenyl group (preferably optionally substituted by 1 to 3 substituents selected from 1) a halogen atom, 2) a $C_{1-6}$ alkyl group optionally substituted by 1 to 3 halogen atoms, and 3) a cyano group) or

an optionally substituted $C_{3-10}$ cycloalkyl group (preferably optionally substituted by 1 to 3 $C_{1-6}$ alkyl groups optionally substituted by 1 to 3 halogen atoms),
particularly preferably

(6) a phenyl group optionally substituted by 1 to 3 substituents selected from 1) a halogen atom, 2) a $C_{1-6}$ alkyl group optionally substituted by 1 to 3 halogen atoms, and 3) a cyano group, or
a $C_{3-10}$ cycloalkyl group optionally substituted by 1 to 3 $C_{1-6}$ alkyl groups optionally substituted by 1 to 3 halogen atoms,
most preferably
(7) a phenyl group optionally substituted by 1 to 3 substituents selected from 1) a halogen atom, 2) a $C_{1-6}$ alkyl group optionally substituted by 1 to 3 halogen atoms, and 3) a cyano group.

Y is present at a substitutable position in the ring to which it is bonded, and is as defined above. It is preferably

(1) an optionally substituted nitrogen-containing aromatic heterocyclic group (preferably optionally substituted by 1 to 3 $C_{1-6}$ alkoxy groups), or

an optionally substituted aryl group (preferably optionally substituted by 1 to 3 $C_{1-6}$ alkoxy groups),
more preferably

(2) an optionally substituted 5- to 14-membered (preferably, 5- or 6-membered) nitrogen-containing aromatic heterocyclic group (preferably optionally substituted by 1 to 3 $C_{1-6}$ alkoxy groups), or

an optionally substituted $C_{6-14}$ aryl group (preferably optionally substituted by 1 to 3 $C_{1-6}$ alkoxy groups), further preferably

(3) an optionally substituted pyridyl group (preferably optionally substituted by 1 to 3 $C_{1-6}$ alkoxy groups), or an optionally substituted phenyl group (preferably optionally substituted by 1 to 3 $C_{1-6}$ alkoxy groups), further more preferably
(4) a pyridyl group optionally substituted by 1 to 3 $C_{1-6}$ alkoxy groups, or

a phenyl group optionally substituted by 1 to 3 $C_{1-6}$ alkoxy groups, particularly preferably

(5) a pyridyl group optionally substituted by 1 to 3 $C_{1-6}$ alkoxy groups.

Z is as defined above, and is preferably

(1) a hydrogen atom, a hydroxy group, $NH_2$, or $NHR^3$
wherein $R^3$ is an optionally substituted $C_{1-6}$ alkyl group or an optionally substituted 3- to 8-membered saturated ring group, more preferably
(2) a hydrogen atom, a hydroxy group, $NH_2$, or $NHR^3$
wherein $R^3$ is

(i) a $C_{1-6}$ alkyl group optionally substituted by 1 to 3 substituents selected from

a hydroxy group,
a $C_{1-6}$ alkoxy group,
a (mono- or di-$C_{1-6}$ alkyl)amino group,
a $C_{3-10}$ cycloalkyl group,
a 5- or 6-membered monocyclic aromatic heterocyclic group,
a 3- to 8-membered monocyclic non-aromatic heterocyclic group optionally substituted by a $C_{1-6}$ alkoxy-carbonyl group,
a $C_{1-6}$ alkyl-carbonylamino group,
a (mono- or di-$C_{1-6}$ alkylamino)carbonyl group, and
a $C_{1-6}$ alkyl-sulfonyl group, or

(ii) a 3- to 8-membered saturated ring group optionally substituted by 1 to 3 substituents selected from

a $C_{1-6}$ alkyl group,
a $C_{1-6}$ alkoxy-carbonyl group, and
an oxo group,
further preferably

(3) a hydrogen atom, a hydroxy group, $NH_2$, or $NHR^3$
wherein $R^3$ is

(i) a $C_{1-6}$ alkyl group optionally substituted by 1 to 3 substituents selected from

a hydroxy group,
a $C_{1-6}$ alkoxy group,
a (mono- or di-$C_{1-6}$ alkyl)amino group,
a $C_{3-10}$ cycloalkyl group,
a 5- or 6-membered monocyclic aromatic heterocyclic group,
a 3- to 8-membered monocyclic non-aromatic heterocyclic group optionally substituted by a $C_{1-6}$ alkoxy-carbonyl group,
a $C_{1-6}$ alkyl-carbonylamino group,
a (mono- or di-$C_{1-6}$ alkylamino)carbonyl group, and

a $C_{1-6}$ alkyl-sulfonyl group, or

(ii) a 3- to 8-membered monocyclic saturated ring group optionally substituted by 1 to 3 substituents selected from

a $C_{1-6}$ alkyl group,
a $C_{1-6}$ alkoxy-carbonyl group, and
an oxo group,
further more preferably

(4) a hydrogen atom, a hydroxy group, $NH_2$, or $NHR^3$
wherein $R^3$ is

(i) a $C_{1-6}$ alkyl group optionally substituted by 1 to 3 substituents selected from

a hydroxy group,
a $C_{1-6}$ alkoxy group,
a (mono- or di-$C_{1-6}$ alkyl)amino group,
a $C_{3-10}$ cycloalkyl group,
a 5- or 6-membered monocyclic nitrogen-containing aromatic heterocyclic group,
a 3- to 8-membered monocyclic oxygen-containing non-aromatic heterocyclic group,
a 3- to 8-membered monocyclic nitrogen-containing non-aromatic heterocyclic group optionally substituted by a $C_{1-6}$ alkoxy-carbonyl group,
a $C_{1-6}$ alkyl-carbonylamino group,
a (mono- or di-$C_{1-6}$ alkylamino)carbonyl group, and
a $C_{1-6}$ alkyl-sulfonyl group, or

(ii)

(a) a 3- to 8-membered monocyclic oxygen-containing saturated heterocyclic group, or
(b) a 3- to 8-membered monocyclic nitrogen-containing saturated heterocyclic group optionally substituted by 1 to 3 substituents selected from

a $C_{1-6}$ alkyl group,
a $C_{1-6}$ alkoxy-carbonyl group, and
an oxo group,
particularly preferably

(5) a hydroxy group or $NHR^3$
wherein $R^3$ is

(a) a 3- to 8-membered monocyclic oxygen containing saturated heterocyclic group, or
(b) a 3- to 8-membered monocyclic nitrogen-containing saturated heterocyclic group optionally substituted 1 to 3 substituents selected from

a $C_{1-6}$ alkyl group,
a $C_{1-6}$ alkoxy-carbonyl group, and
an oxo group.

Specific preferred examples of compound (I) or a salt thereof include the following.

[Compound A-1]

Compound (I), wherein $R^1$ is a hydrogen atom or a $C_{1-6}$ alkyl group,

$R^2$ is a hydrogen atom or a $C_{1-6}$ alkyl group,
X is a phenyl group optionally substituted by 1 to 3 substituents selected from 1) a halogen atom, 2) a $C_{1-6}$ alkyl group optionally substituted by 1 to 3 halogen atoms, and 3) a cyano group,
a pyridyl group optionally substituted by 1 to 3 substituents selected from 1) a halogen atom, 2) a $C_{1-6}$ alkyl group

optionally substituted by 1 to 3 halogen atoms, and 3) a cyano group, or
a $C_{3-10}$ cycloalkyl group optionally substituted by 1 to 3 $C_{1-6}$ alkyl groups optionally substituted by 1 to 3 halogen atoms,
Y is a pyridyl group optionally substituted by 1 to 3 $C_{1-6}$ alkoxy groups, or a phenyl group optionally substituted by 1 to 3 $C_{1-6}$ alkoxy groups,
Z is a hydrogen atom, a hydroxy group, $NH_2$, or
$NHR^3$
wherein $R^3$ is

(i) a $C_{1-6}$ alkyl group optionally substituted by 1 to 3 substituents selected from

a hydroxy group,
a $C_{1-6}$ alkoxy group,
a (mono- or di-$C_{1-6}$ alkyl)amino group,
a $C_{3-10}$ cycloalkyl group,
a 5- or 6-membered monocyclic aromatic heterocyclic group,
a 3- to 8-membered monocyclic non-aromatic heterocyclic group optionally substituted by a $C_{1-6}$ alkoxy-carbonyl group,
a $C_{1-6}$ alkyl-carbonylamino group,
a (mono- or di-$C_{1-6}$ alkylamino)carbonyl group, and
a $C_{1-6}$ alkyl-sulfonyl group, or

(ii) a 3- to 8-membered monocyclic saturated ring group optionally substituted by 1 to 3 substituents selected from

a $C_{1-6}$ alkyl group,
a $C_{1-6}$ alkoxy-carbonyl group, and
an oxo group
(preferably, the optionally substituted 3- to 8-membered monocyclic saturated heterocyclic group),
or a salt thereof.

[Compound A-2]

The above-mentioned [Compound A-1], wherein X is a phenyl group optionally substituted by 1 to 3 substituents selected from 1) a halogen atom, 2) a $C_{1-6}$ alkyl group optionally substituted by 1 to 3 halogen atoms, and 3) a cyano group, or a $C_{3-10}$ cycloalkyl group optionally substituted by 1 to 3 $C_{1-6}$ alkyl groups optionally substituted by 1 to 3 halogen atoms, or a salt thereof.

[Compound A-3]

The above-mentioned [Compound A-1], wherein

$R^1$ is a hydrogen atom or a $C_{1-6}$ alkyl group,
$R^2$ is a hydrogen atom or a $C_{1-6}$ alkyl group,
X is a phenyl group optionally substituted by 1 to 3 substituents selected from 1) a halogen atom, 2) a $C_{1-6}$ alkyl group optionally substituted by 1 to 3 halogen atoms, and 3) a cyano group,
Y is a pyridyl group optionally substituted by 1 to 3 $C_{1-6}$ alkoxy groups, and
Z is a hydrogen atom, a hydroxy group, $NH_2$, or $NHR^3$
wherein $R^3$ is

(i) a $C_{1-6}$ alkyl group optionally substituted by 1 to 3 substituents selected from

a hydroxy group,
a $C_{1-6}$ alkoxy group,
a (mono- or di-$C_{1-6}$ alkyl)amino group,
a $C_{3-10}$ cycloalkyl group,
a 5- or 6-membered monocyclic aromatic heterocyclic group,
a 3- to 8-membered monocyclic non-aromatic heterocyclic group optionally substituted by a $C_{1-6}$ alkoxy-carbonyl group,
a $C_{1-6}$ alkyl-carbonylamino group,

a (mono- or di-$C_{1-6}$ alkylamino)carbonyl group, and
a $C_{1-6}$ alkyl-sulfonyl group, or

(ii) a 3- to 8-membered monocyclic saturated ring group optionally substituted by 1 to 3 substituents selected from

a $C_{1-6}$ alkyl group,
a $C_{1-6}$ alkoxy-carbonyl group, and
an oxo group (preferably, the optionally substituted 3- to 8-membered monocyclic saturated heterocyclic group),
or a salt thereof.

[Compound A-4]

The above-mentioned [Compound A-1], wherein

$R^1$ is a hydrogen atom or a $C_{1-6}$ alkyl group,
$R^2$ is a hydrogen atom or a $C_{1-6}$ alkyl group,
(wherein, preferably,
$R^1$ is a $C_{1-6}$ alkyl group,
$R^2$ is a hydrogen atom),
X is a phenyl group optionally substituted by 1 to 3 substituents selected from 1) a halogen atom, 2) a $C_{1-6}$ alkyl group optionally substituted by 1 to 3 halogen atoms, and 3) a cyano group, and
Y is a pyridyl group optionally substituted by 1 to 3 $C_{1-6}$ alkoxy groups,
Z is a hydrogen atom, a hydroxy group, $NH_2$, or $NHR^3$
wherein $R^3$ is

(i) a $C_{1-6}$ alkyl group optionally substituted by 1 to 3 substituents selected from

a hydroxy group,
a $C_{1-6}$ alkoxy group,
a (mono- or di-$C_{1-6}$ alkyl)amino group,
a $C_{3-10}$ cycloalkyl group,
a 5- or 6-membered monocyclic nitrogen-containing aromatic heterocyclic group (e.g., pyrazolyl group, pyridyl group),
a 3- to 8-membered monocyclic oxygen containing non-aromatic heterocyclic group (e.g., oxetanyl group, morpholinyl group),
a 3- to 8-membered monocyclic nitrogen containing non-aromatic heterocyclic group (e.g., azetidinyl group, piperidinyl group) optionally substituted by a $C_{1-6}$ alkoxy-carbonyl group,
a $C_{1-6}$ alkyl-carbonylamino group,
a (mono- or di-$C_{1-6}$ alkylamino)carbonyl group, and
a $C_{1-6}$ alkyl-sulfonyl group, or

(ii)

(a) a 3- to 8-membered monocyclic oxygen containing saturated heterocyclic group (e.g., tetrahydrofuranyl group), or
(b) a 3- to 8-membered monocyclic nitrogen containing saturated heterocyclic group (e.g., pyrrolidinyl group) optionally substituted by 1 to 3 substituents selected from

a $C_{1-6}$ alkyl group,
a $C_{1-6}$ alkoxy-carbonyl group, and
an oxo group,
or a salt thereof.

[Compound A-5]

The above-mentioned [Compound A-1] to [Compound A-4], wherein

Z is a hydroxy group or $NHR^3$
wherein $R^3$ is

(a) a 3- to 8-membered monocyclic oxygen containing saturated heterocyclic group, or
(b) a 3- to 8-membered nitrogen-containing saturated heterocyclic group optionally substituted by 1 to 3 substituents selected from

a $C_{1-6}$ alkyl group,
a $C_{1-6}$ alkoxy-carbonyl group, and
an oxo group,
or a salt thereof.

[Compound A-6]

The above-mentioned [Compound A-1], wherein

$R^1$ is a $C_{1-6}$ alkyl group,
$R^2$ is a hydrogen atom,
X is a phenyl group optionally substituted by 1 to 3 substituents selected from 1) a halogen atom, 2) a $C_{1-6}$ alkyl group optionally substituted by 1 to 3 halogen atoms, and 3) a cyano group,
Y is a pyridyl group optionally substituted by 1 to 3 $C_{1-6}$ alkoxy groups, and
Z is a hydroxy group or $NHR^3$
wherein $R^3$ is a pyrrolidinyl group optionally substituted by 1 to 3 substituents selected from a $C_{1-6}$ alkyl group and an oxo group,
or a salt thereof.

In the above [compound A-1] to [compound A-6], compound (I) more preferably has a structure represented by the following formula (Ia):

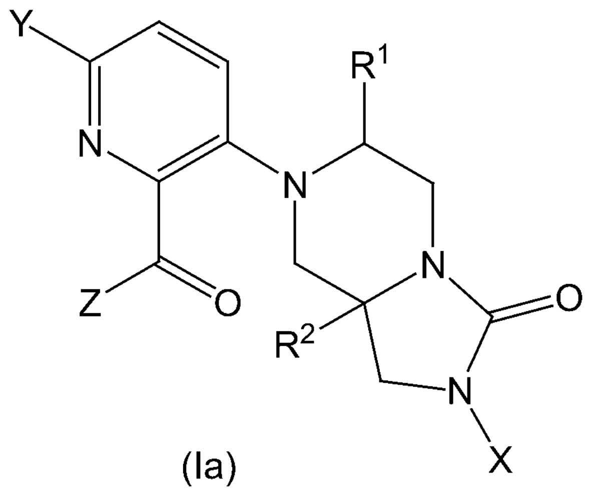

(Ia)

[Compound A-7]

The above-mentioned [Compound A-6] selected from 3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-(2-ethoxypyridin-3-yl)-N-[(3R)-1-methyl-5-oxopyrrolidin-3-yl]pyridine-2-carboxamide (compound of Example 49), 3-[(6R,8aS)-6-ethyl-2-[4-fluoro-2-(trifluoromethyl)phenyl]-3-oxo-5,6,8,8a-tetrahydro-1H-imidazo[1,5-a]pyrazin-7-yl]-6-(2-ethoxypyridin-3-yl)-N-[(3R)-1-methyl-5-oxopyrrolidin-3-yl]pyridine-2-carboxamide (compound of Example 52), 3-[(6R,8aS)-2-(4-chloro-2-cyanophenyl)-6-ethyl-3-oxo-5,6,8,8a-tetrahydro-1H-imidazo[1,5-a]pyrazin-7-yl]-6-(2-ethoxypyridin-3-yl)-N-[(3R)-1-methyl-5-oxopyrrolidin-3-yl]pyridine-2-carboxamide (compound of Example 54), 3-[(6R,8aS)-2-[4-chloro-2-(trifluoromethyl)phenyl]-6-methyl-3-oxo-5,6,8,8a-tetrahydro-1H-imidazo[1,5-a]pyrazin-7-yl]-6-(2-methoxy-3-pyridinyl)-N-[(3R)-1-methyl-5-oxopyrrolidin-3-yl]pyridine-2-carboxamide (compound of Example 61), 3-[(6R,8aS)-2-[4-chloro-2-(trifluoromethyl)phenyl]-6-methyl-3-oxo-5,6,8,8a-tetrahydro-1H-imidazo[1,5-a]pyrazin-7-yl]-6-(2-methoxyphenyl)-N-[(3R)-1-methyl-5-oxopyrrolidin-3-yl]pyridine-2-carboxamide (compound of Example 64), or
a salt thereof.

Other specific preferred examples of compound (I) include the following.

[Compound B-1]

Compound (I), wherein $R^1$ is a hydrogen atom or a $C_{1-6}$ alkyl group,

$R^2$ is a hydrogen atom or a $C_{1-6}$ alkyl group,
X is a phenyl group optionally substituted by 1 to 3 substituents selected from a halogen atom, a $C_{1-6}$ alkyl group optionally substituted by 1 to 3 halogen atoms, and a cyano group, or
a $C_{3-10}$ cycloalkyl group optionally substituted by 1 to 3 $C_{1-6}$ alkyl groups optionally substituted by 1 to 3 halogen atoms,
Y is a pyridyl group optionally substituted by 1 to 3 $C_{1-6}$ alkoxy groups, or a phenyl group optionally substituted by 1 to 3 $C_{1-6}$ alkoxy groups,
Z is a hydrogen atom, a hydroxy group, $NH_2$, or
$NHR^3$
wherein $R^3$ is

(i) a $C_{1-6}$ alkyl group optionally substituted by 1 to 3 substituents selected from

a hydroxy group,
a $C_{1-6}$ alkoxy group,
a (mono- or di-$C_{1-6}$ alkyl)amino group,
a $C_{3-10}$ cycloalkyl group,
a 5- or 6-membered monocyclic aromatic heterocyclic group,
a 3- to 8-membered monocyclic non-aromatic heterocyclic group optionally substituted by a $C_{1-6}$ alkoxy-carbonyl group,
a $C_{1-6}$ alkyl-carbonylamino group,
a (mono- or di-$C_{1-6}$ alkylamino)carbonyl group, and
a $C_{1-6}$ alkyl-sulfonyl group, or

(ii) a 3- to 8-membered monocyclic saturated ring group optionally substituted by 1 to 3 substituents selected from

a $C_{1-6}$ alkyl group,
a $C_{1-6}$ alkoxy-carbonyl group, and
an oxo group,
or a salt thereof.

[Compound B-2]

The above-mentioned [Compound B-1], wherein

$R^1$ is a $C_{1-6}$ alkyl group,
$R^2$ is a hydrogen atom,
X is a phenyl group optionally substituted by 1 to 3 substituents selected from a halogen atom, a $C_{1-6}$ alkyl group optionally substituted by 1 to 3 halogen atoms, and a cyano group,
Y is a pyridyl group optionally substituted by 1 to 3 $C_{1-6}$ alkoxy groups, and
Z is a hydrogen atom, a hydroxy group, $NH_2$, or $NHR^3$
wherein $R^3$ is

(i) a $C_{1-6}$ alkyl group optionally substituted by 1 to 3 substituents selected from

a hydroxy group,
a $C_{1-6}$ alkoxy group,
a (mono- or di-$C_{1-6}$ alkyl)amino group,
a $C_{3-10}$ cycloalkyl group,
a 5- or 6-membered monocyclic aromatic heterocyclic group,
a 3- to 8-membered monocyclic non-aromatic heterocyclic group optionally substituted by a $C_{1-6}$ alkoxy-

carbonyl group,
a $C_{1-6}$ alkyl-carbonylamino group,
a (mono- or di-$C_{1-6}$ alkylamino)carbonyl group, and
a $C_{1-6}$ alkyl-sulfonyl group, or

(ii) a 3- to 8-membered monocyclic saturated ring group optionally substituted by 1 to 3 substituents selected from

a $C_{1-6}$ alkyl group,
a $C_{1-6}$ alkoxy-carbonyl group, and
an oxo group,
or a salt thereof.

[Compound B-3]

The above-mentioned [Compound B-2], wherein

Z is a hydrogen atom, a hydroxy group, $NH_2$, or $NHR^3$
wherein $R^3$ is

(i) a $C_{1-6}$ alkyl group optionally substituted by 1 to 3 substituents selected from

a hydroxy group,
a $C_{1-6}$ alkoxy group,
a (mono- or di-$C_{1-6}$ alkyl)amino group,
a $C_{3-10}$ cycloalkyl group,
a 5- or 6-membered monocyclic nitrogen-containing aromatic heterocyclic group,
a 3- to 8-membered monocyclic oxygen containing non-aromatic heterocyclic group,
a 3- to 8-membered monocyclic nitrogen containing non-aromatic heterocyclic group optionally substituted by a $C_{1-6}$ alkoxy-carbonyl group,
a $C_{1-6}$ alkyl-carbonylamino group,
a (mono- or di-$C_{1-6}$ alkylamino)carbonyl group, and
a $C_{1-6}$ alkyl-sulfonyl group, or

(ii)

(a) a 3- to 8-membered monocyclic oxygen containing saturated heterocyclic group, or
(b) a 3- to 8-membered monocyclic nitrogen containing saturated heterocyclic group optionally substituted by 1 to 3 substituents selected from

a $C_{1-6}$ alkyl group,
a $C_{1-6}$ alkoxy-carbonyl group, and
an oxo group,
or a salt thereof.

[Compound B-4]

The above-mentioned [Compound B-3], wherein

Z is $NHR^3$
wherein $R^3$ is a 3- to 8-membered monocyclic nitrogen containing saturated heterocyclic group optionally substituted by 1 to 3 substituents selected from
a $C_{1-6}$ alkyl group,
a $C_{1-6}$ alkoxy-carbonyl group, and
an oxo group,
or a salt thereof.

[Compound B-5]

The above-mentioned [Compound B-4], wherein

$R^1$ is a $C_{1-6}$ alkyl group,
$R^2$ is a hydrogen atom,
X is a phenyl group optionally substituted by 1 to 3 substituents selected from a halogen atom, a $C_{1-6}$ alkyl group optionally substituted by 1 to 3 halogen atoms, and a cyano group,
Y is a pyridyl group optionally substituted by 1 to 3 $C_{1-6}$ alkoxy groups, and
$^Z$ is $NHR^3$
wherein $R^3$ is a pyrrolidinyl group optionally substituted by 1 to 3 substituents selected from a $C_{1-6}$ alkyl group and an oxo group,
or a salt thereof.

Compound (I) can be used either in a free form or in the form of a salt thereof (preferably a pharmaceutically acceptable salt thereof). Those skilled in the art can appropriately select from the both forms and carry out the present invention based on the characteristics of each compound (I) used.
Examples of salt acceptable as the medicament include salts with inorganic acid such as hydrochloride, hydrobromide, sulfate, phosphate, and the like, salts with organic acid such as acetate, fumarate, oxalate, citrate, methanesulfonate, benzenesulfonate, tosylate, maleate, and the like; and salts with base such as alkali metal salts such as sodium salt, potassium salt, and the like, alkaline earth metal salts such as calcium salt and the like, and the like; salts with amino acid such as glycinate, lysinate, argininate, ornithinate, glutamate, aspartate, and the like.

[Production method of compound (I) or a salt thereof]

The production methods of compound (I) or a salt thereof (hereinafter sometimes to be generically referred to as the present compound) are described below.
The starting materials and reagents used in each step in the following production method, and the obtained compounds each may form a salt. Examples of the salt include those similar to the aforementioned salts of the compound of the present invention and the like.
When the compound obtained in each step is a free compound, it can be converted to a desired salt by a method known per se. Conversely, when the compound obtained in each step is a salt, it can be converted to a free form or a desired other kind of salt by a method known per se.
The compound obtained in each step can also be used for the next reaction as a reaction mixture thereof or after obtaining a crude product thereof. Alternatively, the compound obtained in each step can be isolated and/or purified from the reaction mixture by a separation means such as concentration, crystallization, recrystallization, distillation, solvent extraction, fractionation, chromatography, and the like according to a conventional method. Racemic compounds can be separated and purified into chiral compounds by using a chiral column. Diastereomeric mixtures can be separated by silica gel column chromatography or a recrystallization method.
When the starting materials and reagent compounds of each step are commercially available, the commercially available products can be used as they are.
In the reaction of each step, while the reaction time varies depending on the reagents and solvents to be used, unless otherwise specified, it is generally 1 min to 4 days, preferably 10 min to 8 hr.
In the reaction of each step, while the reaction temperature varies depending on the reagents and solvents to be used, unless otherwise specified, it is generally -78°C to 300°C, preferably -78°C to 150°C.
In the reaction of each step, while the pressure varies depending on the reagents and solvents to be used, unless otherwise specified, it is generally 1 atm to 20 atm, preferably 1 atm to 3 atm.
In the reaction of each step, for example, microwave synthesizers such as Initiator manufactured by Biotage and the like are sometimes used. While the reaction temperature varies depending on the reagents and solvents to be used, unless otherwise specified, it is generally room temperature to 300°C, preferably 50°C to 250°C. While the reaction time varies depending on the reagents and solvents to be used, unless otherwise specified, it is generally 1 min to 48 hr, preferably 1 min to 8 hr.
In the reaction of each step, unless otherwise specified, a reagent is used in 0.5 equivalent to 20 equivalents, preferably 0.8 equivalent to 5 equivalents, relative to the substrate. When a reagent is used as a catalyst, the reagent is used in 0.001 equivalent to 1 equivalent, preferably 0.01 equivalent to 0.2 equivalent, relative to the substrate. When the reagent is also a reaction solvent, the reagent is used in a solvent amount.
In the reaction of each step, unless otherwise specified, it is performed without solvent or by dissolving or suspending in a suitable solvent. Specific examples of the solvent include those described in Examples and the following.

alcohols: methanol, ethanol, tert-butyl alcohol, 2-methoxyethanol, and the like;
ethers: diethyl ether, diisopropyl ether, diphenyl ether, tetrahydrofuran, 1,2-dimethoxyethane, and the like;
aromatic hydrocarbons: chlorobenzene, toluene, xylene, and the like;
saturated hydrocarbons: cyclohexane, hexane, heptane, and the like;
amides: N,N-dimethylformamide, N,N-dimethylacetamide, N-methylpyrrolidone, and the like;
halogenated hydrocarbons: dichloromethane, dichloroethane, carbon tetrachloride, and the like;
nitriles: acetonitrile, and the like;
sulfoxides: dimethyl sulfoxide, and the like;
aromatic organic bases: pyridine, and the like;
acid anhydrides: acetic anhydride, and the like;
organic acids: formic acid, acetic acid, trifluoroacetic acid, and the like;
inorganic acids: hydrochloric acid, sulfuric acid, and the like;
esters: ethyl acetate, and the like;
ketones: acetone, methyl ethyl ketone, and the like; and water.

Two or more kinds of the above-mentioned solvents may be used by mixing at an appropriate ratio.
When a base is used in the reaction of each step, for example, bases shown below or those described in Examples are used.

inorganic bases: sodium hydroxide, potassium hydroxide, magnesium hydroxide, potassium carbonate, sodium carbonate, cesium carbonate, calcium carbonate, sodium hydrogen carbonate, and the like;
organic bases: triethylamine, N,N-diisopropyl ethylamine, diethylamine, pyridine, 4-dimethylaminopyridine, N,N-dimethylaniline, 1,4-diazabicyclo[2.2.2]octane, 1,8-diazabicyclo[5.4.0]-7-undecene, imidazole, piperidine, and the like;
metal alkoxides: sodium ethoxide, potassium tert-butoxide, N-methylimidazole, and the like;
alkali metal hydrides: sodium hydride and the like;
metal amides: sodium amide, lithium diisopropyl amide, lithium hexamethyl disilazide, and the like;
organic lithiums: n-butyllithium and the like.

When an acid or acidic catalyst is used in the reaction of each step, for example, acids and acidic catalysts shown below or those described in Examples are used.

inorganic acids: hydrochloric acid, sulfuric acid, nitric acid, hydrobromic acid, phosphoric acid, and the like;
organic acids: acetic acid, trifluoroacetic acid, citric acid, p-toluenesulfonic acid, 10-camphorsulfonic acid, and the like; and
Lewis acids: boron trifluoride diethyl ether complex, zinc iodide, anhydrous aluminum chloride, anhydrous zinc chloride, anhydrous iron chloride, and the like.

Unless otherwise specified, the reaction of each step is performed according to a method known per se, for example, the methods described in Jikken Kagaku Kouza 5th edition, vol. 13 - vol. 19 (The Chemical Society of Japan ed.); Shinjikken Kagaku Kouza (Courses in Experimental Chemistry), vol. 14 - vol. 15 (The Chemical Society of Japan ed.); Fine Organic Chemistry rev. 2nd edition (L. F. Tietze, Th. Eicher, NANKODO); rev. Organic Name Reactions, Their Mechanism and Essence (Hideo Togo, Kodansha); ORGANIC SYNTHESES Collective Volume I - VII (John Wiley & Sons Inc); Modern Organic Synthesis in the Laboratory, A Collection of Standard Experimental Procedures (Jie Jack Li, OXFORD UNIVERSITY); Comprehensive Heterocyclic Chemistry III, Vol. 1 - Vol. 14 (Elsevier Japan KK); Strategic Applications of Named Reactions in Organic Synthesis (translation supervisor Kiyoshi Tomioka, KAGAKUDOJIN); Comprehensive Organic Transformations (VCH Publishers Inc.), 1989, and the like, or the methods described in the Examples.
When performing the Curtius rearrangement reaction in each step, diphenylphosphoryl azide and an organic base can be used as the reagents. Moreover, ethyl chloroformate, a base, sodium azide, and the like can also be used as the reagent.
When a reduction reaction is performed in each step, examples of the reducing agent to be used include metal hydrides such as lithium aluminum hydride, sodium triacetoxyborohydride, sodium cyanoborohydride, diisobutylaluminum hydride (DIBAL-H), sodium borohydride, tetramethylammonium triacetoxyborohydride, and the like; boranes such as borane tetrahydrofuran complex and the like; Raney nickel; Raney cobalt; hydrogen; formic acid; triethylsilane, and the like. When a carbon-carbon double bond or triple bond is reduced, a method using a catalyst such as palladium-carbon, Lindlar catalyst, and the like is used.
When the Mitsunobu reaction is performed in each step, azodicarboxylate esters (e.g., diethyl azodicarboxylate (DEAD), diisopropyl azodicarboxylate (DIAD), and the like) and triphenylphosphine, tributyl phosphine are used as the

reagents.

In each step, protection or deprotection reaction of a functional group is performed by the method known per se, for example, the methods described in "Protective Groups in Organic Synthesis, 4th Ed." (Theodora W. Greene, Peter G. M. Wuts) Wiley-Interscience, 2007; "Protecting Groups 3rd Ed." (P. J. Kocienski) Thieme, 2004, and the like, or the methods described in the Examples.

Examples of the protecting group of the hydroxy group of alcohol and the like and a phenolic hydroxy group include ether protecting groups such as methoxymethyl ether, benzyl ether, tert-butyldimethylsilyl ether, tetrahydropyranyl ether, and the like; carboxylate ester protecting groups such as acetate ester and the like; sulfonate ester protecting groups such as methanesulfonate ester and the like; carbonate ester protecting groups such as tert-butylcarbonate and the like, and the like.

Examples of the protecting group of the carbonyl group of aldehyde include acetal protecting groups such as dimethyl acetal and the like; cyclic acetal protecting groups such as 1,3-dioxane and the like, and the like.

Examples of the protecting group of the carbonyl group of ketone include ketal protecting groups such as dimethyl ketal and the like; cyclic ketal protecting groups such as 1,3-dioxane and the like; oxime protecting groups such as O-methyloxime and the like; hydrazone protecting groups such as N,N-dimethylhydrazone and the like, and the like.

Examples of the carboxyl protecting group include ester protecting groups such as methyl ester, benzylester, and the like; amide protecting groups such as N,N-dimethylamide and the like, and the like.

Examples of the protecting group of thiol include ether protecting groups such as benzyl thioether and the like; ester protecting groups such as thioacetate, thiocarbonate, thiocarbamate, and the like, and the like.

Examples of the protecting group of the amino group or the aromatic heterocycle of imidazole, pyrrole, indole, or the like include carbamate protecting groups such as benzylcarbamate, tert-butylcarbamate, and the like; amide protecting groups such as acetamide and the like; alkylamine protecting groups such as benzylamine, p-methoxybenzylamino, N-triphenyl methylamine, and the like, sulfonamide protecting groups such as methanesulfonamide and the like, and the like.

The protecting group can be removed by a method known per se, for example, a method using acid, base, ultraviolet light, hydrazine, phenylhydrazine, sodium N-methyldithiocarbamate, tetrabutylammonium fluoride, palladium acetate, trialkylsilyl halide (e.g., trimethylsilyl iodide, trimethylsilyl bromide), a reduction method (e.g., under hydrogen atmosphere, palladium/carbon), an oxidation method (e.g., using ammonium cerium(IV) nitrate or 2,3-dichloro-5,6-dicyano-1,4-benzoquinone), and the like. When converting an alkyl ester compound to a carboxylic acid compound, it can be performed using a strong base (sodium hydroxide or potassium trimethylsilanolate), a hydrogen-palladium catalyst, or a palladium(0) catalyst.

When an aromatic nucleophilic substitution reaction is performed in each step, a nucleophilic agent (e.g., amines, imidazole) and a base (e.g., organic bases, inorganic bases) are used as the reagent.

When a coupling reaction is performed in each step, as a metal catalyst to be used, palladium compounds such as palladium(II) acetate, tetrakis(triphenylphosphine)palladium(0), dichlorobis(triphenylphosphine)palladium(II), dichlorobis(triethylphosphine)palladium(II), tris(dibenzylideneacetone)dipalladium(O), 1,1'-bis(diphenylphosphino)ferrocene palladium(II) chloride, [1,1'-bis(di-tert-butylphosphino)ferrocene]dichloropalladium(II), and the like; nickel compounds such as tetrakis (triphenylphosphine) nickel(0) and the like; rhodium compounds such as chloro(1,5-cyclooctadiene) rhodium(I) (dimer), tris(triphenylphosphine) rhodium(III) chloride, and the like; cobalt compound; copper compounds such as copper oxide, copper(I) iodide, and the like; platinum compound, and the like can be mentioned. A base may be further added to the reaction, and as such base, organic bases, inorganic bases, and the like can be mentioned.

When an oxidation reaction is performed in each step, as an oxidant to be used, peroxy acids such as m-chloroperbenzoic acid (mCPBA), hydrogen peroxide, tert-butyl hydroperoxide, and the like; perchloric acid salts such as tetrabutylammonium perchlorate and the like; chlorine acid salts such as sodium chlorate and the like; chlorous acid salts such as sodium chlorite and the like; periodic acid salt such as sodium periodate and the like; high-valent iodine reagents such as iodosylbenzene and the like; reagents containing manganese such as manganese dioxide, potassium permanganate, and the like; leads such as lead tetraacetate and the like; reagents containing chrome such as pyridinium chlorochromate (PCC), pyridinium dichromate (PDC), Jones reagent, and the like; halogen compounds such as N-bromosuccinimide (NBS) and the like; oxygen; ozone; sulfur trioxide-pyridine complex; osmium tetraoxide; selenium dioxide; 2,3-dichloro-5,6-dicyano-1,4-benzoquinone (DDQ), and the like can be mentioned. The oxidation reaction can also be performed in an alcohol solvent in the presence of potassium hydroxide and iodine. Alternatively, the oxidation reaction can be performed by Pinnick oxidation using sodium chlorite and sodium dihydrogen phosphate in the presence of 2-methyl-2-butene.

When an esterification reaction, an amidation reaction, or a urea-formation reaction is performed in each step, as a reagent to be used, activated carboxylic acids such as halogenated acyl forms such as acid chloride, acid bromide, and the like; acid anhydride; active ester forms, and the like; and the like can be mentioned. As the activator of carboxylic acid, carbodiimide condensing agents such as 1-ethyl-3-(3-dimethylaminopropyl)carbodiimide hydrochloride (WSCD) and the like; triazine condensing agents such as 4-(4,6-dimethoxy-1,3,5-triazin-2-yl)-4-methylmorpholinium chloride-n-hydrate

(DMT-MM) and the like; carbonic acid ester condensing agents such as 1,1-carbonyldiimidazole (CDI) and the like; diphenylphosphoryl azide (DPPA); benzotriazol-1-yloxy-tris dimethylaminophosphonium salt (BOP reagent); 2-chloro-1-methyl-pyridinium iodide (Mukaiyama reagent); thionyl chloride; lower alkyl haloformates such as ethyl chloroformate and the like; O-(7-azabenzotriazol-1-yl)-N,N,N',N'-tetramethyluronium hexafluorophosphate (HATU); sulfuric acid; triphosgene; 2-bromo-1-ethylpyridinium tetrafluoroborate; chloro-N,N,N',N'-tetramethylformamidinium hexafluorophosphate(TCFH), these combination, and the like can be mentioned. When a carbodiimide condensing agent is used, additives such as 1-hydroxybenzotriazole (HOBt), N-hydroxysuccinimide (HOSu), dimethylaminopyridine (DMAP) and the like can be further added to the reaction. Furthermore, when the same molecule contains an amino group and an ester group or a carboxyl group, an intramolecular amidation reaction can also be performed under heating conditions. In addition, when performing a urea-formation reaction, triphosgene, 1,1-carbonyldiimidazole (CDI), and 2,2,2-trichloroethyl chloroformate can be used as reagents, and two different types of amines may also be used. The urea-formation reaction can be performed using isocyanates and amines.

When a hydrolysis reaction is performed in each step, an acid or a base is used as the reagent. In addition, when an acid hydrolysis reaction of tert-butyl ester is performed, formic acid, triethylsilane, and the like are sometimes added to reductively trap the by-produced tert-butyl cation.

When an alkylation reaction is performed in each step, nucleophilic agents (e.g., amines, alcohol, active methylene compound adjacent to electron-withdrawing group, and the like), electrophiles (e.g., alkyl halides, sulfonic acid esters), and bases (e.g., organic bases, alkali metal hydrides, metal alkoxides, inorganic bases, and the like) are used. In each step, the alkylation reaction includes converting alcohols into sulfonic acid ester compounds by using substituted sulfonyl chlorides (e.g., methanesulfonyl chloride, p-toluenesulfonyl chloride, etc.) and bases (e.g., organic bases, alkali metal hydrides, metal alkoxides, inorganic bases, etc.), after which the compounds may be reacted with amines or amides within the molecule.

When a Michael reaction is performed in each step, acrylates or acrylic acids (e.g., itaconic acid) and amines and the like are used. As the reaction conditions, conditions in which the reaction is performed in an acidic solvent such as acetic acid and the like, conditions in which a base (e.g., organic bases, metal alkoxides, inorganic bases) is added, and conditions in which only a solvent (N-methyl-2-pyrrolidone, etc.) is used can be mentioned.

Specific examples of the method for producing compound (I) include the following. However, the method is not limited to these.

Production method 1

Compound (I) can be produced by the following method.

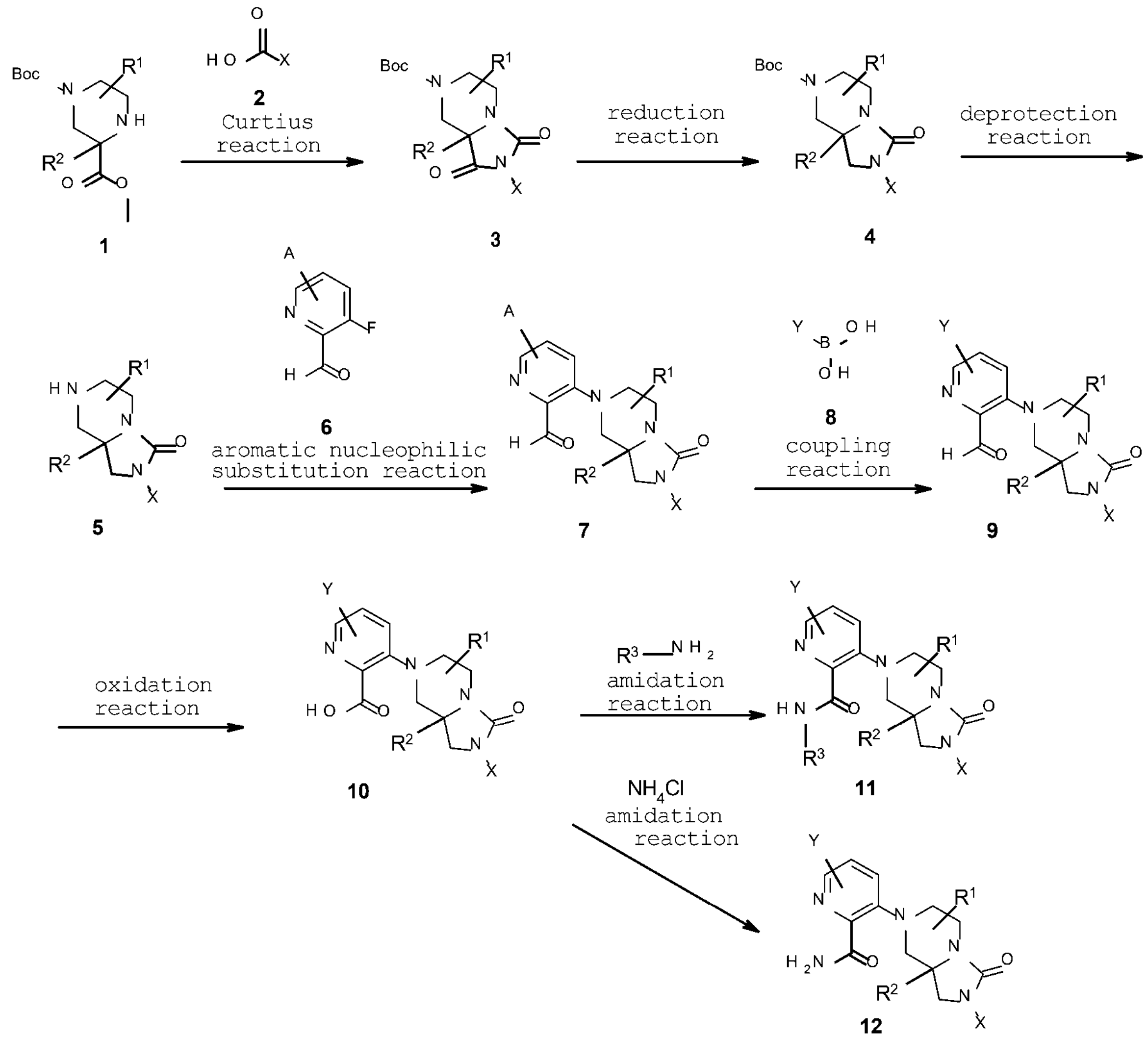


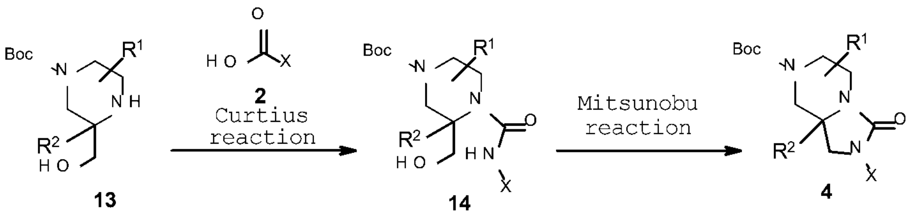


wherein A is a halogen atom (e.g., chlorine, iodine, bromine), and other symbols are as defined above.

Production method 2

Compound (I) can be produced by the following method.

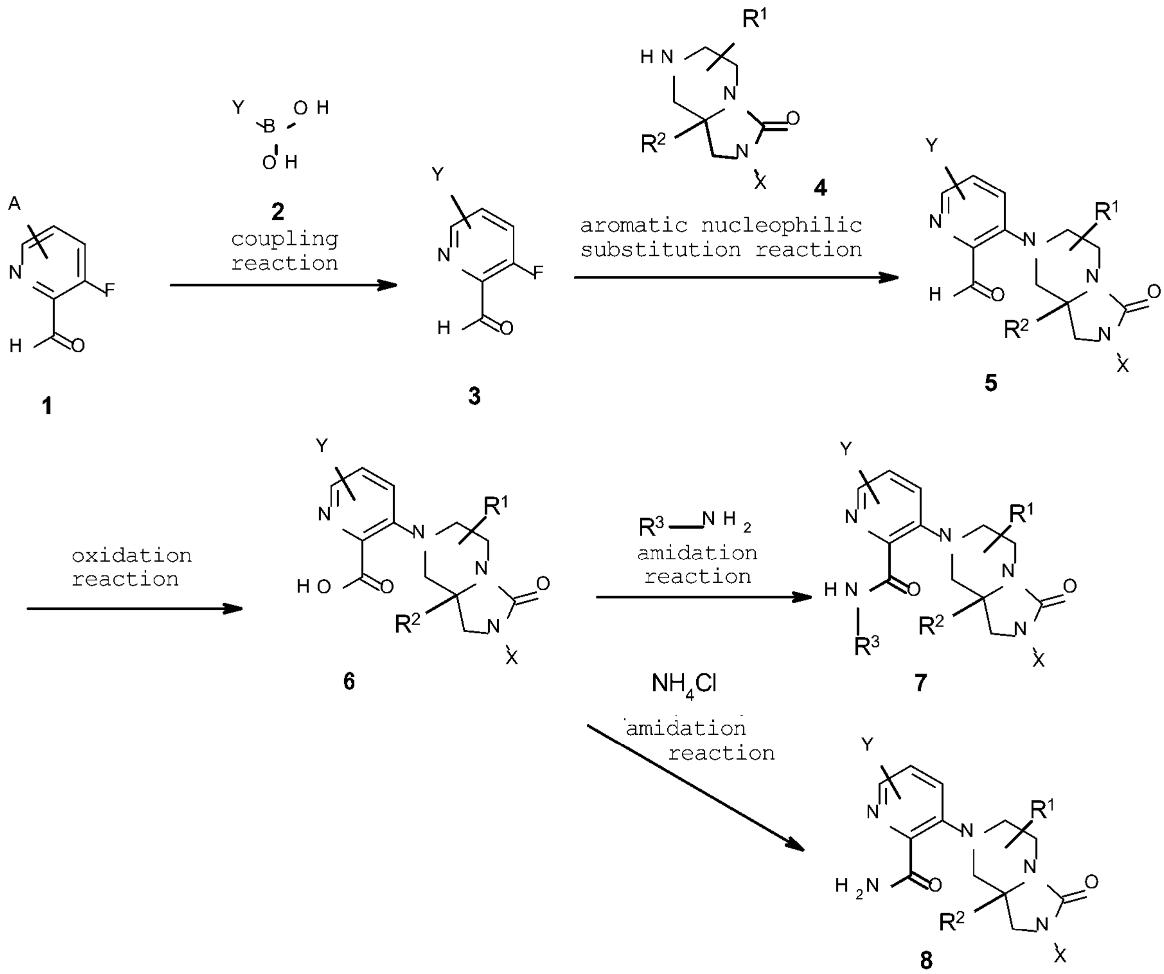


wherein A is a halogen atom (e.g., chlorine, iodine, bromine), and other symbols are as defined above.

Production method 3

Among compounds (I), compound (13), compound (14), compound (15), and compound (16) can be produced by the following methods.

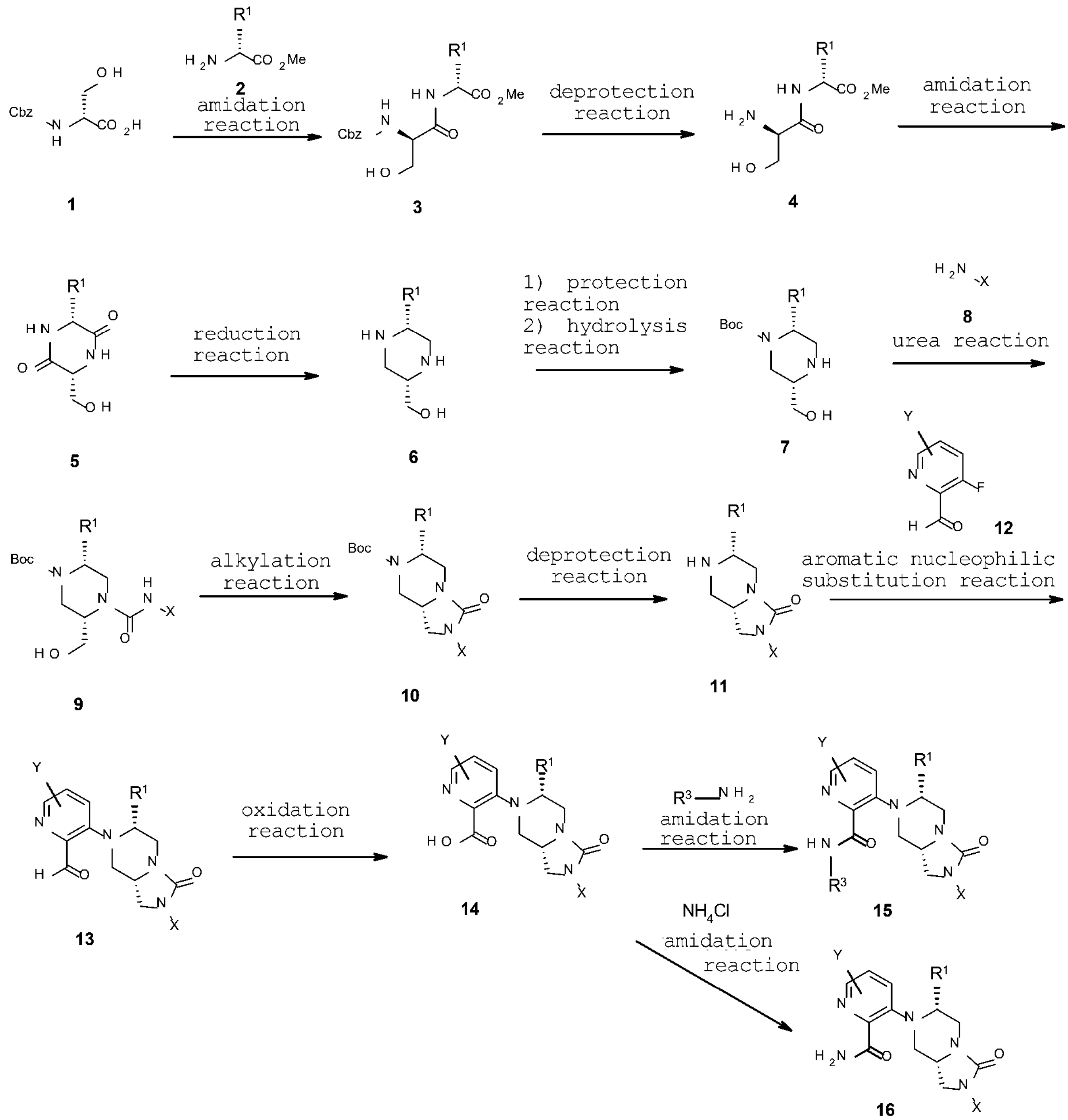


wherein symbols are as defined above.

Production method 4

Among compounds (I), compound (5), compound (6), compound (7), and compound (8) can be produced by the following methods.

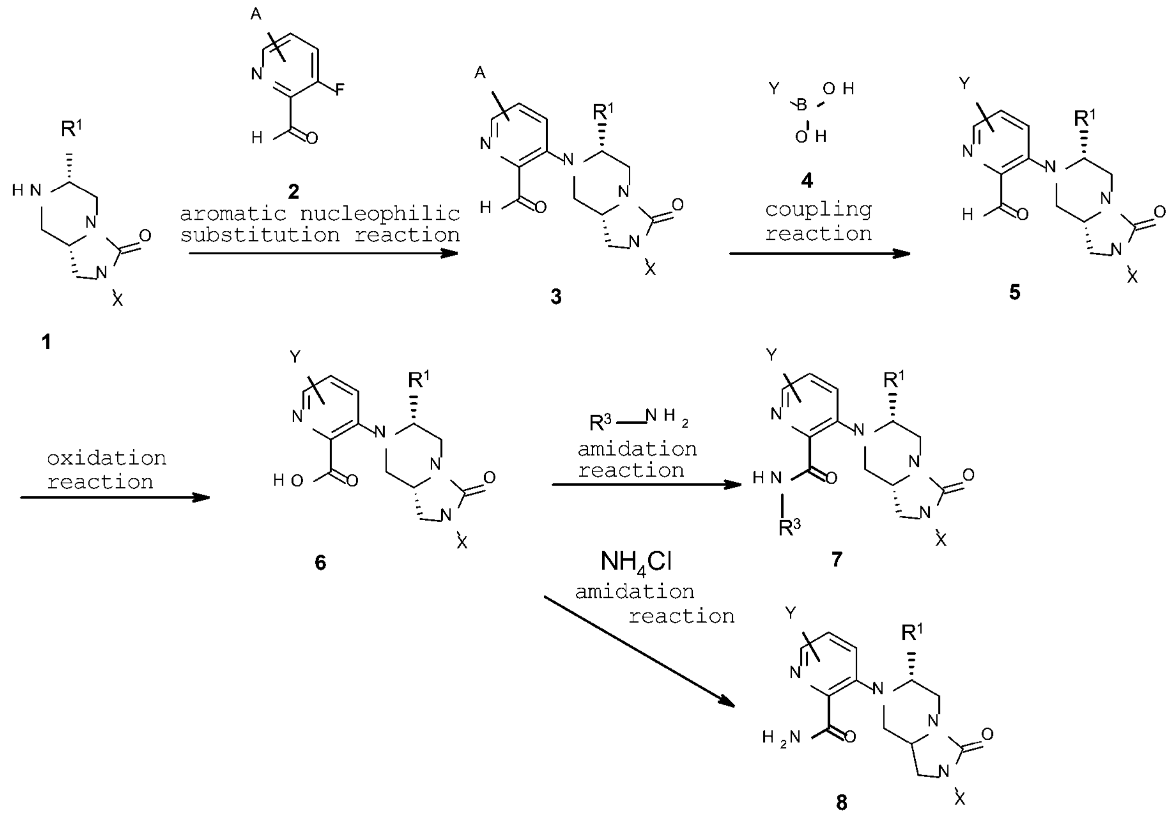


wherein A is a halogen atom (e.g., chlorine, iodine, bromine), and other symbols are as defined above.

In addition, in the step of producing compound (I) from the starting compound and/or production intermediate of compound (I), compound (I) can be synthesized by performing, when desired, Curtius reaction, reduction reaction, protection reaction, urea-formation reaction, deprotection reaction, aromatic nucleophilic substitution reaction, coupling reaction, oxidation reaction, hydrolysis reaction, alkylation reaction, amidation reaction, and Mitsunobu reaction either alone or in combination, depending on the various substituents that compound (I) can take.

The starting compounds and/or production intermediates of compound (I) may form a salt, and is not particularly limited as long as the reaction is achieved. For example, salts similar to the salts that compound (I) may form are used.

The starting compounds and/or production intermediates of compound (I) may generate a configurational isomer (E,Z form). Once the configurational isomer (E,Z form) is generated, it can be isolated and purified by general separation means such as extraction, recrystallization, distillation, chromatography, and the like, whereby a pure compound can be produced. In addition, it is possible to isomerize the double bond by heating, acid catalyst, transition metal complex, metal catalyst, radical species catalyst, light irradiation or strong base catalyst, and the like according to the method described in Jikken Kagaku Koza (Courses in Experimental Chemistry) 14 (edited by the Chemical Society of Japan), pages 251 to 253, and the method described in 4th Edition Jikken Kagaku Kouza 19 (edited by the Chemical Society of Japan), pages 273 to 274, and a method analogous thereto, and obtain the corresponding pure isomer.

When the target product is obtained in a free form by the above reaction, it may be converted into a salt according to a conventional method, and when it is obtained as a salt, it can also be converted into a free form or other salt according to a conventional method. Compound (I) thus obtained can be isolated and purified from the reaction solution by known means such as phase transfer, concentration, solvent extraction, fractionation, crystallization, recrystallization, chromatography, and the like.

Compound (I) thus obtained, other reaction intermediate and starting compounds thereof can be isolated and purified from the reaction mixture by a method known per se, for example, extraction, concentration, neutralization, filtration, distillation, recrystallization, column chromatography, thin layer chromatography, preparative high performance liquid chromatography (preparative HPLC), moderate-pressure preparative liquid chromatography (moderate-pressure preparative LC), and the like.

Compound (I) may form a salt and can be produced according to methods known per se. For example, when compound (I) is a basic compound, it can be produced by adding an inorganic acid or organic acid, and when compound (I) is an acidic compound, it can be produced by adding an organic base or inorganic base.

When compound (I) is a solvate (e.g., hydrate), the solvate of the target compound can be isolated by various methods such as distillation, crystallization, and the like from the reaction mixture after reacting the starting compounds in

an appropriate solvent. A non-solvate can be produced by desolvating a solvate by heating, drying, or the like.

When compound (I) contains optical isomers, both these individual optical isomers and mixtures thereof are naturally encompassed in the scope of the present invention. When desired, these isomers can also be optically resolved or produced individually according to methods known per se.

When compound (I) exists as a configurational isomer, diastereomer, conformer, or the like, each can be isolated by the aforementioned separation and purification means, when desired. When compound (I) is a racemate, it can be separated into an S form and an R form by general optical resolution means.

When compound (I) contains a stereoisomer, the present invention also encompasses both the isomer alone or a mixture thereof.

[Use of compound (I) or a salt thereof]

Compound (I) or a salt thereof has a superior MC2R antagonist action, and therefore can be effective for the prophylaxis or treatment of diseases caused by ACTH signal abnormalities, for example, endocrine diseases (e.g., Cushing's disease, ectopic ACTH syndrome, ectopic CRH syndrome, congenital adrenal hyperplasia, polycystic ovary syndrome), metabolic diseases (e.g., obesity, type 2 diabetes, dyslipidemia, osteoporosis, sarcopenia), cardiovascular diseases (e.g., hypertension, arteriosclerosis), central nervous system diseases (e.g., depression, dementia, insomnia), eye diseases (e.g., cataract, glaucoma), and infectious diseases.

Particularly, based on the MC2R antagonist action, compound (I) or a salt thereof may be useful as a prophylactic or therapeutic agent for endocrine diseases (e.g., Cushing's disease, ectopic ACTH syndrome, ectopic CRH syndrome, congenital adrenal hyperplasia, polycystic ovary syndrome), metabolic diseases (e.g., obesity, type 2 diabetes, dyslipidemia, osteoporosis, sarcopenia), cardiovascular diseases (e.g., hypertension, arteriosclerosis), central nervous system diseases (e.g., depression, dementia, insomnia), eye diseases (e.g., cataract, glaucoma), infectious diseases, and the like.

In the present specification, the "prophylaxis" includes preventing the onset of a disease (all pathological conditions or one or more pathological conditions) and delaying the onset of the disease. A "prophylactically effective amount" refers to a dose of compound (I) sufficient to achieve such purposes.

In the present specification, the "treatment" includes curing a disease (all pathological conditions or one or more pathological conditions), improving the disease, and suppressing the progression of the severity of the disease. A "therapeutically effective amount" refers to a dose of compound (I) sufficient to achieve such purposes.

Compound (I) or a salt thereof (the present compound) can be used either alone or in the form of a pharmaceutical composition containing the present compound as an active ingredient together with a pharmaceutically acceptable carrier.

Examples of such pharmaceutical composition include tablets (including sugar-coated tablet, film-coated tablet, sublingual tablet, orally disintegrating tablet, buccal tablet, and the like), pill, powder, granule, capsules (including soft capsule, and microcapsule), syrup, liquid, emulsion, suspension, controlled-release preparations (e.g., immediate-release preparation, sustained-release preparation, sustained-release microcapsule), aerosols, films (e.g., orally disintegrable films, and mouth cavity mucosa patching film), injections (e.g., subcutaneous injection, intravenous injections (e.g., bolus), intramuscular injection, and intraperitoneal injection), drip transfusion, transdermal absorption type preparation, ointment, lotion, adhesive preparation, suppositories (e.g., rectal suppository, vaginal suppository), pellet, nasal preparation, pulmonary preparation (inhalant), eye drop, and the like.

In the present specification, as the "pharmaceutically acceptable carrier", various carriers conventionally used in the field of pharmaceutical technology can be used.

As the specific examples of the "pharmaceutically acceptable carrier", in solid preparations, excipients (e.g., lactose, sucrose, D-mannitol, starch, cornstarch, crystalline cellulose, light anhydrous silicic acid, etc.), lubricants (e.g., magnesium stearate, talc, colloid silica, etc.), binders (e.g., crystalline cellulose, sucrose, D-mannitol, dextrin, hydroxypropylcellulose, hydroxypropylmethylcellulose, polyvinylpyrrolidone, starch, sucrose, gelatin, methylcellulose, sodium carboxymethylcellulose, etc.), disintegrants (e.g., starch, carboxymethylcellulose, carboxymethylcellulose calcium, sodium carboxymethyl starch, L-hydroxypropylcellulose, etc.), and the like can be used.

In liquid preparations, solvents (e.g., water for injection, isotonic brine, alcohol, propylene glycol, macrogol, sesame oil, etc.), solubilizing agents (e.g., polyethylene glycol, propylene glycol, D-mannitol, benzoic acid benzyl, ethanol, triethanolamine, sodium carbonate, sodium citrate, etc.), suspending agents (e.g., surfactants such as stearyltriethanolamine, sodium lauryl sulfate, lauryl aminopropionic acid, lecithin, benzalkonium chloride, glycerol monostearate, and the like; for example, hydrophilic polymers such as poly(vinyl alcohol), polyvinylpyrrolidone, sodium carboxymethylcellulose, methylcellulose, hydroxymethylcellulose, hydroxypropylcellulose, and the like, and the like), isotonicity agents (e.g., glucose, D-sorbitol, sodium chloride, glycerol, D-mannitol, etc.), buffering agents (e.g., buffer of phosphate, citrate, or the like, etc.), and soothing agents (e.g., benzyl alcohol etc.), and the like can be used.

Where necessary, formulation additives such as antiseptics (e.g., paraoxybenzoic acid esters, chlorobutanol, benzyl alcohol, sorbic acid, etc.), antioxidants (e.g., sulfite, ascorbic acid, $\alpha$-tocopherol, etc.), colorants, sweeteners, and

the like may be further added.

The above-mentioned pharmaceutical composition generally containing the compound of the present invention in an amount of 0.01 to 99% (w/w), preferably 0.1 to 85% (w/w), of the total amount of the preparation, can be produced, though the amount varies depending on the dosage form, administration method, carrier, and the like. The pharmaceutical composition can be produced by methods conventionally used in the field of pharmaceutical technology. The pharmaceutical composition may be formulated into a sustained release preparation containing the active ingredient.

(Subject of administration)

The present compound is expected to have low toxicity and few side effects, and also has superior properties as a pharmaceutical product. Therefore, the compound of the present invention can be safely administered to mammals (e.g., human, dog, or cat, particularly human).

(Administration route)

The present compound may be administered orally or parenterally (e.g., intravenously, intramuscularly, subcutaneously, intraviscerally, intranasally, intradermally, ophthalmically, intracerebrally, rectally, intravaginally, intraperitoneally, intralesionally) alone or as a pharmaceutical composition.

(Dose)

The dose of the present compound varies depending on the subject of administration, administration route, and the age and symptoms of the subject, and is not particularly limited. For example, the dose of the present compound is 1 to 250 mg per oral administration, and 0.1 to 1000 mg per parenteral administration.

(Use as prodrug)

Compound (I) can also be used in the form of a prodrug, and the prodrug is encompassed in compound (I). A prodrug of the compound (I) means a compound which is converted to the compound (I) with a reaction due to an enzyme, a gastric acid, etc. under the physiological condition in the living body, that is, a compound which is converted to the compound (I) with oxidation, reduction, hydrolysis, etc. according to an enzyme; a compound which is converted to the compound (I) by hydrolysis etc. due to gastric acid, etc. A prodrug of compound (I) may be a compound obtained by subjecting an amino group in compound (I) to an acylation, alkylation or phosphorylation [e.g., a compound obtained by subjecting an amino group in compound (I) to an eicosanoylation, alanylation, pentylaminocarbonylation, (5-methyl-2-oxo-1,3-dioxolen-4-yl)methoxycarbonylation, tetrahydrofuranylation, pyrrolidylmethylation, pivaloyloxymethylation and tert-butylation, etc.]; a compound obtained by subjecting a hydroxy group in compound (I) to an acylation, alkylation, phosphorylation or boration [e.g., a compound obtained by subjecting an hydroxy group in compound (I) to an acetylation, palmitoylation, propanoylation, pivaloylation, succinylation, fumarylation, alanylation, dimethylaminomethylcarbonylation, etc.]; a compound obtained by subjecting a carboxyl group in compound (I) to an esterification or amidation [e.g., a compound obtained by subjecting a carboxyl group in compound (I) to an ethyl esterification, phenyl esterification, carboxymethyl esterification, dimethylaminomethyl esterification, pivaloyloxymethyl esterification, ethoxycarbonyloxyethyl esterification, phthalidyl esterification, (5-methyl-2-oxo-1,3-dioxolen-4-yl)methyl esterification, cyclohexyloxycarbonylethyl esterification and methylamidation, etc.] and the like. Any of these compounds can be produced from compound (I) by a method known per se.

A prodrug for compound (I) may also be one which is converted into compound (I) under a physiological condition, such as those described in IYAKUHIN no KAIHATSU (Development of Pharmaceuticals), Vol.7, Design of Molecules, p.163-198, Published by HIROKAWA SHOTEN (1990).

Compound (I) may be any of hydrate, non-hydrate, solvate, and non-solvate.

Compound (I) may be labeled or substituted with an isotope (e.g., $^{2}H$, $^{3}H$, $^{11}C$, $^{14}C$, $^{18}F$, $^{35}S$, $^{125}I$) and the like. A compound labeled or substituted with an isotope can be used, for example, as a tracer used for Positron Emission Tomography (PET) (PET tracer), and may be useful in the field of medical diagnosis and the like.

Compound (I) also encompasses a deuterium conversion form wherein $^{1}H$ is converted to $^{2}H$(D).

Compound (I) also encompasses a tautomer.

Compound (I) may be a pharmaceutically acceptable cocrystal or cocrystal salt. The cocrystal or cocrystal salt means a crystalline substance constituted with two or more special solids at room temperature, each having different physical properties (e.g., structure, melting point, melting heat, hygroscopicity, solubility and stability). The cocrystal or cocrystal salt can be produced by a cocrystallization method known per se.

(Combination use with other agents)

The present compound may be used in combination with other active ingredients (hereinafter to be abbreviated as concomitant drug).

As a concomitant drug, a compound or a salt thereof that can have prophylactic and/or therapeutic effects may be appropriately blended according to the disease to be prevented or treated. Examples thereof include adrenal steroid synthesis inhibitors such as osilodrostat, metyrapone and the like, glucocorticoid receptor antagonists such as mifepristone and the like, somatostatin analogs such as pasireotide and the like, CRF1 receptor antagonists such as crinecerfont, tildacerfont and the like, glucocorticoids such as hydrocortisone, predonisolone, dexamethasone, and the like, and mineralocorticoids such as fludrocortisone and the like.

By combining the present compound and a concomitant drug, a superior effect, for example,

(1) the dose can be reduced as compared to single administration of the present compound or a concomitant drug,
(2) the drug to be combined with the present compound can be selected according to the condition of patients (mild case, severe case and the like),
(3) a sustained treatment effect can be designed by selecting a concomitant drug having different action and mechanism from the present compound,
(4) a synergistic effect can be afforded by a combined use of the present compound and a concomitant drug, and the like, can be achieved.

In the following, the present compound and a concomitant drug used in combination are referred to as the "combination agent of the present compound".

When using the combination agent of the present compound, the administration time of the present compound and the concomitant drug is not restricted, and the present compound or a pharmaceutical composition thereof, or the concomitant drug or a pharmaceutical composition thereof can be administered to an administration subject simultaneously, or may be administered at different times. The dosage of the concomitant drug may be determined according to the dose clinically used, and can be appropriately selected depending on an administration subject, administration route, disease, combination and the like.

The administration mode of the combination agent of the present compound and the concomitant drug is not particularly limited, and the present compound and the concomitant drug only need to be combined on administration. Examples of such administration mode include the following:

(1) administration of a single preparation obtained by simultaneously processing the present compound and the concomitant drug, (2) simultaneous administration of two kinds of preparations of the present compound and the concomitant drug, which have been separately produced, by the same administration route, (3) administration of two kinds of preparations of the present compound and the concomitant drug, which have been separately produced, by the same administration route in a staggered manner, (4) simultaneous administration of two kinds of preparations of the present compound and the concomitant drug, which have been separately produced, by different administration routes, (5) administration of two kinds of preparations of the present compound and the concomitant drug, which have been separately produced, by different administration routes in a staggered manner (e.g., administration in the order of the present compound and the concomitant drug, or in the reverse order) and the like.

The mixing ratio of the present compound and a concomitant drug in the concomitant drug of the present compound can be appropriately determined depending on the administration subject, administration route, target disease, and the like.

For example, the content of the present compound in the combination agent of the present compound varies depending on the form of a preparation, and is generally from about 0.01 to about 100 wt%, preferably from about 0.1 to about 50 wt%, further preferably from about 0.5 to about 20 wt%, based on the whole preparation.

The content of the concomitant drug in the combination agent of the present compound varies depending on the form of a preparation, and is generally from about 0.01 to about 100 wt%, preferably from about 0.1 to about 50 wt%, further preferably from about 0.5 to about 20 wt%, based on the whole preparation.

The content of additives such as a carrier and the like in the combination agent of the present compound varies depending on the form of a preparation, and is generally from about 1 to about 99.99 wt%, preferably from about 10 to about 90 wt%, based on the whole preparation.

Similar contents may be employed even when the present compound and a concomitant drug are separately formulated into preparations.

[Example]

The present invention is further explained in detail by referring to the following Examples, Experimental Examples and Formulation Examples which do not limit the present invention and may be changed without departing from the scope of the present invention.

The "room temperature" in the following Examples indicates the range of generally from about 10°C to about 35°C. The ratio for a mixed solvent is, unless otherwise specified, a volume mixing ratio and % means wt% unless otherwise specified.

Unless particularly indicated, the elution in column chromatography in the Examples was performed under observation by TLC (Thin Layer Chromatography). For TLC observation, $60F_{254}$ manufactured by Merck was used as a TLC plate, and the solvent used as an elution solvent for column chromatography was used as developing solvent. For detection, moreover, a UV detector was adopted. In silica gel column chromatography, the indication of NH means use of aminopropylsilane-bound silica gel.

In the following Examples, the following abbreviations are used.

MS: mass spectrum
M: mol concentration
N: normality
$CDCl_3$: deuterochloroform
DMSO-$d_6$: deuterodimethyl sulfoxide
$^1$H NMR: proton nuclear magnetic resonance
LC/MS: liquid chromatograph mass spectrometer
ESI: electrospray ionization
APCI: atmospheric pressure chemical ionization
DMSO: dimethyl sulfoxide
HATU: 2-(7-azabenzotriazol-1-yl)-1,1,3,3-tetramethyluronium hexafluorophosphate
HPLC: high-speed liquid chromatogram
Boc: tert-butoxycarbonyl
t-Bu: tert-butyl
DPPA: diphenylphosphoryl azide
dppf: 1,1'-bis(diphenylphosphino)ferrocene
DtBPF: 1,1'-bis(di-tert-butylphosphino)ferrocene
TCFH: chloro-N,N,N',N'-tetramethylformamidinium hexafluorophosphate

$^1$H NMR was measured by Fourier transform NMR. For the analysis, ACD/SpecManager (trade name) and the like were used. Very mild peaks of protons of hydroxy group, amino group and the like are not described.

MS was measured by LC/MS. As ionization method, ESI method, or APCI method was used. The data indicates those found. Generally, molecular ion peaks ($[M+H]^+$, $[M-H]^-$ and the like) are observed. For example, in the case of a compound having a tert-butoxycarbonyl group, a peak in which the tert-butoxycarbonyl group or tert-butyl group has been removed is observed as a fragment ion, and in the case of a compound having a hydroxy group, a peak in which $H_2O$ has been removed is sometimes observed as a fragment ion. In the case of a salt, generally, a molecular ion peak or a fragment ion peak is observed.

Example 1

3-[2-[4-chloro-2-(trifluoromethyl)phenyl]-3-oxo-5,6,8,8a-tetrahydro-1H-imidazo[1,5-a]pyrazin-7-yl]-6-(2-ethoxypyridin-3-yl)pyridine-2-carbaldehyde

A) tert-butyl 2-(4-chloro-2-(trifluoromethyl)phenyl)-1,3-dioxohexahydroimidazo[1,5-a]pyrazine-7(1H)-carboxylate

To a mixture of tetrahydrofuran (50 mL) and 4-chloro-2-(trifluoromethyl)benzoic acid (3.0 g, 13.39 mmol) were added triethylamine (5.7 mL, 40.18 mmol) and diphenylphosphoryl azide (5.75 mL, 26.78 mmol) at 0°C, and the reaction mixture was stirred at the same temperature for 1 hr. Then, 1-(tert-butyl)3-methylpiperazine-1,3-dicarboxylate (3.92 g, 16.07 mmol.) was added at room temperature, and the reaction mixture was stirred at 80°C for 16 hr. After completion of the reaction, the reaction mixture was diluted with sodium hydrogen carbonate aqueous solution and extracted twice with ethyl acetate. The combined organic layer was dried over anhydrous sodium sulfate, filtered, and concentrated under reduced pressure. The residue was purified by silica gel column chromatography (ethyl acetate/petroleum ether) to give the title compound (3 g).

$^1$H NMR (400 MHz, $CDCl_3$) δ 1.50 (9H, s), 2.75-2.88 (2H, m), 3.01-3.19 (1H, m), 4.11-4.24 (3H, m), 4.54-4.65 (1H, m), 7.27-7.28 (1H, m), 7.67 (1H, d, J = 8.25 Hz), 7.79 (1H, s).
MS: 334.2.

B) tert-butyl 2-(4-chloro-2-(trifluoromethyl)phenyl)-3-oxohexahydroimidazo[1,5-a]pyrazine-7(1H)-carboxylate

To a mixture of tert-butyl 2-(4-chloro-2-(trifluoromethyl)phenyl)-1,3-dioxohexahydroimidazo[1,5-a]pyrazine-7(1H)-carboxylate (1.5 g, 3.46 mmol) in tetrahydrofuran (30 mL) was added at 0°C a borane-tetrahydrofuran complex (17.32 ml, 17.32 mmol, 1.0M tetrahydrofuran solution), and the mixture was stirred at 70°C for 16 hr. After completion of the reaction, to the reaction mixture were added methanol and saturated sodium hydroxide aqueous solution, and the mixture was stirred for 1 hr and extracted twice with ethyl acetate. The organic layer was washed with water and saturated brine, dried over anhydrous sodium sulfate, filtered, and concentrated under reduced pressure. The residue was purified by silica gel column chromatography (ethyl acetate/petroleum ether) to give the title compound (1.2 g).

$^1$H NMR (400 MHz, DMSO-$d_6$) δ 1.43 (9H, s), 2.67-2.79 (2H, m), 2.88 (2H, td, J = 12.65, 3.55 Hz), 3.63-3. 69 (1H, m), 3.70-3.78 (2H, m), 3.88-3.95 (1H, m), 4.00-4.12 (1H, m), 7.58 (1H, d, J = 8.0 Hz), 7.83-7.87 (2H, m).
MS: 420.38.

C) 2-(4-chloro-2-(trifluoromethyl)phenyl)hexahydroimidazo[1,5-a]pyrazin-3(2H)-one hydrochloride

To a mixture of tert-butyl 2-(4-chloro-2-(trifluoromethyl)phenyl)-3-oxohexahydroimidazo[1,5-a]pyrazine-7(1H)-carboxylate (1.2 g, 2.86 mmol) and 1,4-dioxane (20 mL) was added at 0°C 4N hydrogen chloride (5 mL, 1,4-dioxane solution), and the reaction mixture was stirred at room temperature for 16 hr. After completion of the reaction, the reaction mixture was concentrated under reduced pressure. The residue was washed with diethyl ether and n-pentane to give the title compound (1.1 g).

$^1$H NMR (400 MHz, DMSO-$d_6$) δ 2.78-2.92 (2H, m), 3.21-3.31 (2H, m), 3.39-3.45 (2H, m), 3.79-3.87 (2H, m), 4.07-4.18 (1H, m), 7.64 (1H, d, J = 9.29 Hz), 7.85-7.93 (2H, m), 9.46-9.54 (2H, m).
MS: 320.26.

D) 6-bromo-3-(2-(4-chloro-2-(trifluoromethyl)phenyl)-3-oxohexahydroimidazo[1,5-a]pyrazin-7(1H)-yl)picoline aldehyde

To a mixture of 2-(4-chloro-2-(trifluoromethyl)phenyl)hexahydroimidazo[1,5-a]pyrazine-3(2H)-one hydrochloride (0.938 g, 2.94 mmol) and toluene (10 mL) was added at 0°C potassium carbonate (1.01 g, 7.35 mmol), and the reaction mixture was stirred at room temperature for 30 min. Thereafter, 6-bromo-3-fluoropicoline aldehyde (0.5 g, 2.45 mmol) was added at room temperature, and the reaction mixture was stirred at 120°C for 16 hr. After completion of the reaction, the reaction mixture was diluted with water, and extracted twice with ethyl acetate. The organic layer was washed with water and saturated brine, dried over anhydrous sodium sulfate, filtered, and concentrated under reduced pressure. The residue was purified by silica gel column chromatography (ethyl acetate/petroleum ether) to give the title compound (0.35 g).

$^1$H NMR (400 MHz, DMSO-$d_6$) δ 2.83-2.93 (2H, m), 3.24 (1H, td, J = 12.47, 3.42 Hz), 3.34-3.37 (2H, m), 3.48 (1H, d, J = 10.27 Hz), 3.77 (1H, dd, J = 13.08, 2.08 Hz), 3.84 (1H, t, J = 8.6 Hz), 4.06-4.11 (1H, m), 7.61 (1H, d, J = 8.31 Hz), 7.67 (1H, d, J = 8.80 Hz), 7.79 (1H, d, J = 8.80 Hz), 7.84-7.91 (2H, m), 9.90 (1H, s).
MS: 502.9.

E) 5-(2-(4-chloro-2-(trifluoromethyl)phenyl)-3-oxohexahydroimidazo[1,5-a]pyrazin-7(1H)-yl)-2'-ethoxy-[2,3'-bipyridine]-6-carbaldehyde

A mixture of 6-bromo-3-(2-(4-chloro-2-(trifluoromethyl)phenyl)-3-oxohexahydroimidazo[1,5-a]pyrazine-7(1H)-yl)picoline aldehyde (0.350 g, 0.69 mmol), (2-ethoxypyridin-3-yl)boronic acid (0.174 g, 1.04 mmol), potassium phosphate (0.442 g, 2.09 mmol, 3.0 eq.) and [1,1'-bis(diphenylphosphino)ferrocene]dichloropalladium(II)-dichloromethane complex (0.056 g, 0.07 mmol) in 1,4-dioxane-water (10 mL, 4:1) was stirred at room temperature, deaerated, and stirred at 80°C for 16 hr. After completion of the reaction, the reaction mixture was filtered through celite and washed with ethyl acetate. The filtrate was washed with water and saturated brine, dried over anhydrous sodium sulfate, filtered, and concentrated under reduced pressure to give a residue. The residue was purified by silica gel column chromatography (ethyl acetate/petroleum ether) to give the title compound (0.18 g).

$^1$H NMR (400 MHz, $CDCl_3$) δ 1.45 (3H, t, J = 7.09 Hz), 2.91-3.07 (2H, m), 3.30-3.49 (4H, m), 3.84 (1H, t, J = 8.68 Hz),

4.10 (1H, dd, J = 13.20, 2.20 Hz), 4.30-4.39 (1H, m), 4.52 (2H, q, J = 7.09 Hz), 7.05 (1H, dd, J = 7.46, 5.01 Hz), 7.34 (1H, d, J = 8.56 Hz), 7.48 (1H, d, J = 8.80 Hz), 7.60 (1H, dd, J = 8.44, 2.32 Hz), 7.71 (1H, d, J = 2.20 Hz), 8.21 (1H, dd, J = 4.89, 1.96 Hz), 8.26 (1H, d, J = 8.80 Hz), 8.40 (1H, dd, J = 7.34, 1.96 Hz), 10.20 (1H, brs).
MS: 546.0.

Example 2

3-[2-[4-chloro-2-(trifluoromethyl)phenyl]-3-oxo-5,6,8,8a-tetrahydro-1H-imidazo[1,5-a]pyrazin-7-yl]-6-(2-ethoxypyridin-3-yl)pyridine-2-carboxamide

A) 5-(2-(4-chloro-2-(trifluoromethyl)phenyl)-3-oxohexahydroimidazo[1,5-a]pyrazine-7(1H)-yl)-2'-ethoxy-[2,3'-bipyridine]-6-carboxylic acid

To a mixture of 5-(2-(4-chloro-2-(trifluoromethyl)phenyl)-3-oxohexahydroimidazo[1,5-a]pyrazine-7(1H)-yl)-2'-ethoxy-[2,3'-bipyridine]-6-carbaldehyde (0.150 g, 0.27 mmol) and methanol (3 mL) was added a mixture of potassium hydroxide (0.046 g, 0.82 mmol) and methanol (2 mL) at 0°C, and the mixture stirred at the same temperature for 3 min. Then, iodine (0.0.27 mmol) was added, and the mixture was stirred at the same temperature for 16 hr. After completion of the reaction, the reaction mixture was concentrated under reduced pressure to give a residue. The residue was diluted with water and extracted with ethyl acetate. The aqueous layer was acidified with 1N HCl and extracted twice with ethyl acetate. The organic layer was washed with water and saturated brine, dried over anhydrous sodium sulfate, filtered, and concentrated under reduced pressure to give the title compound (0.1 g).

$^1$H NMR (500 MHz, DMSO-$d_6$) δ 1.37 (3H, t, J = 7.0 Hz), 1.80-1.88 (2H, m), 3.08-3.12 (2H, m), 3.36-3.38 (1H, m), 3.44-3.47 (1H, m), 3.77-3.86 (2H, m), 3.94-3.98 (1H, m), 4.44 (2H, q, J = 7.0 Hz), 7.11-7.14 (1H, m), 7.61 (1H, d, J = 8.0 Hz), 7.71 (1H, d, J = 9.0 Hz), 7.86-7.88 (2H, m), 8.12 (1H, d, J = 9.0 Hz), 8.20-8.26 (2H, m), 12.01 (1H, brs).
MS: 562.4.

B) 3-[2-[4-chloro-2-(trifluoromethyl)phenyl]-3-oxo-5,6,8,8a-tetrahydro-1H-imidazo[1,5-a]pyrazin-7-yl]-6-(2-ethoxypyridin-3-yl)pyridine-2-carboxamide

To a stirred mixture of 5-(2-(4-chloro-2-(trifluoromethyl)phenyl)-3-oxohexahydroimidazo[1,5-a]pyrazine-7(1H)-yl)-2'-ethoxy-[2,3'-bipyridine]-6-carboxylic acid (0.060 g, 0.11 mmol) and N,N-dimethylformamide/tetrahydrofuran (4 mL, 1:1) were added N,N-diisopropylethylamine (0.093 ml, 0.53 mmol) and HATU (0.081 g, 0.21 mmol) at 0°C, and the reaction mixture was stirred at room temperature for 1 hr, ammonium chloride (0.028 g, 0.53 mmol) was added at room temperature, and the reaction mixture was stirred at the same temperature for 16 hr. After completion of the reaction, the reaction mixture was diluted with water and extracted twice with ethyl acetate. The organic layer was washed with saturated brine, dried over anhydrous magnesium sulfate, filtered, and concentrated under reduced pressure to give a residue. The residue was purified by Preparative HPLC to give the title compound (35 mg). Preparative HPLC column: Inertsil ODS (250*20) mm, 5 μm, mobile phase: A-0.1% trifluoroacetic acid aqueous solution: B-acetonitrile.

$^1$H NMR (500 MHz, $CDCl_3$) δ 1.46 (3H, t, J = 7.02 Hz), 2.78-3.02 (2H, m), 3.33-3.48 (3H, m), 3.58 (1H, d, J = 10.38 Hz), 3.81 (1H, t, J = 8.54 Hz), 4.08 (1H, d, J = 11.60 Hz), 4.27-4.33 (1H, m), 4.54 (2H, q, J = 7.0 Hz), 5.47 (1H, brs), 7.00-7.08 (1H, m), 7.33 (1H, d, J = 8.54 Hz), 7.49-7.63 (2H, m), 7.70 (1H, d, J = 2.14 Hz), 7.98 (1H, brs), 8.17-8.21 (3H, m).
MS: 561.3.

Example 3

6-(2-ethoxypyridin-3-yl)-3-[3-oxo-2-[1-(trifluoromethyl)cyclopentyl]-5,6,8,8a-tetrahydro-1H-imidazo[1,5-a]pyrazin-7-yl] pyridine-2-carboxamide

The compound was synthesized according to the method shown in Example 1 and Example 2 or a method analogous thereto. MS: 519.0.

Example 4

3-[2-[4-chloro-2-(trifluoromethyl)phenyl]-3-oxo-5,6,8,8a-tetrahydro-1H-imidazo[1,5-a]pyrazin-7-yl]-N-[2-(dimethylamino)ethyl]-6-(2-ethoxypyridin-3-yl)pyridine-2-carboxamide

The compound was synthesized according to the method shown in Example 1 and Example 2 or a method analogous thereto. MS: 632.3.

Example 5

3-[(8aS)-2-[4-chloro-2-(trifluoromethyl)phenyl]-3-oxo-5,6,8,8a-tetrahydro-1H-imidazo[1,5-a]pyrazin-7-yl]-6-(2-ethoxypyridin-3-yl)pyridine-2-carbaldehyde

A) tert-butyl (3S)-4-[[4-chloro-2-(trifluoromethyl)phenyl]carbamoyl]-3-(hydroxymethyl)piperazine-1-carboxylate

To a mixture of toluene (100 mL), 4-chloro-2-(trifluoromethyl)benzoic acid (2.08 g, 9.25 mmol) and triethylamine (1.93 mL, 13.9 mmol) was added DPPA (2.39 mL, 11.1 mmol). The mixture was stirred at room temperature for 1 hr, tert-butyl (3S)-3-(hydroxymethyl)piperazine-1-carboxylate (2.0 g, 9.25 mmol) was added, and the mixture was stirred at 100°C for 5 hr. The solvent was concentrated, and the residue was purified by silica gel column chromatography (hexane/ethyl acetate) to give the title compound (1.6 g).
MS: 382.2.

B) tert-butyl (8aR)-2-[4-chloro-2-(trifluoromethyl)phenyl]-3-oxo-5,6,8,8a-tetrahydro-1H-imidazo[1,5-a]pyrazine-7-carboxylate

To a mixture of tetrahydrofuran (50 mL), tert-butyl (3S)-4-[[4-chloro-2-(trifluoromethyl)phenyl]carbamoyl]-3-(hydroxymethyl)piperazine-1-carboxylate (1.6g, 3.65 mmol) and triphenyl phosphine (1.441 g, 5.48 mmol) was added azodicarboxylic acid diisopropyl (1.18 mL, 5.48 mmol), and the mixture was stirred at room temperature overnight. The solvent was concentrated, and the residue was purified by silica gel column chromatography (hexane/ethyl acetate) to give the title compound (0.88 g,2.10 mmol).
MS: 364.2.

C) (8aS)-2-[4-chloro-2-(trifluoromethyl)phenyl]-1,5,6,7,8,8a-hexahydroimidazo[1,5-a]pyrazin-3-one

A mixture of tetrahydrofuran (10 mL), tert-butyl(8aR)-2-[4-chloro-2-(trifluoromethyl)phenyl]-3-oxo-5,6,8,8a-tetrahydro-1H-imidazo[1,5-a]pyrazine-7-carboxylate (0.88 g, 2.10 mmol) and 6M hydrochloric acid (5.0 mL) was stirred at room temperature overnight. The mixture was basified with sodium hydroxide aqueous solution and potassium carbonate aqueous solution and extracted with ethyl acetate. The organic layer was dried over magnesium sulfate and concentrated to give the title compound (0.64 g).
MS: 320.2.

D) 6-(2-ethoxy-3-pyridyl)-3-fluoropyridine-2-carbaldehyde

A mixture of 6-chloro-3-fluoro-pyridine-2-carbaldehyde (2.00 g, 12.5 mmol), (2-ethoxy-3-pyridyl)boronic acid (2.09 g), tetrakis(triphenyl phosphine) palladium (0) (0.724 g, 0.627 mmol), 2M sodium carbonate aqueous solution (2.00 mol/L, 31.3 mL, 63.7 mmol) and 1,2-dimethoxyethane (50 mL) was deaerated under nitrogen stream and heated to 80°C. After stirring at 80°C for 2 hr, water and ethyl acetate were added to the mixture. The mixture was extracted with ethyl acetate, washed with saturated brine, dried over magnesium sulfate and concentrated. The residue was purified by silica gel column chromatography (hexane/ethyl acetate) to give a colorless oil. The obtained oil was solidified with hexane. The formed solid was collected by filtration and washed with hexane to give the title compound (2.67 g).

$^1$H NMR (300 MHz, DMSO-$d_6$) δ 1.36 (3H, t, J = 7.0 Hz), 4.46 (2H, q, J = 7.0 Hz), 7.16-7.22 (1H, m), 8.03 (1H, dd, J = 10.1, 9.2 Hz), 8.28 (1H, s), 8.30 (1H, q, J = 2.0 Hz), 8.37 (1H, dd, J = 8.9, 3.9 Hz), 10.10 (1H, s).
MS: 247.1.

E) 3-[(8aS)-2-[4-chloro-2-(trifluoromethyl)phenyl]-3-oxo-5,6,8,8a-tetrahydro-1H-imidazo[1,5-a]pyrazin-7-yl]-6-(2-ethoxypyridin-3-yl)pyridine-2-carbaldehyde

(8aS)-2-[4-chloro-2-(trifluoromethyl)phenyl]-1,5,6,7,8,8a-hexahydroimidazo[1,5-a]pyrazin-3-one (130.0 mg, 0.407 mmol), 6-(2-ethoxy-3-pyridyl)-3-fluoropyridine-2-carbaldehyde (100 mg, 0.407 mmol) and potassium carbonate

(281 mg, 2.03 mmol) were stirred in toluene (5 mL) at 110°C overnight. After completion of the reaction, the mixture was purified by silica gel column chromatography (hexane/ethyl acetate) to give the title compound (100 mg).
MS: 546.4.

Example 6

3-[(8aS)-2-[4-chloro-2-(trifluoromethyl)phenyl]-3-oxo-5,6,8,8a-tetrahydro-1H-imidazo[1,5-a]pyrazin-7-yl]-N-[2-(dimethylamino)ethyl]-6-(2-ethoxypyridin-3-yl)pyridine-2-carboxamide

3-[(8aS)-2-[4-chloro-2-(trifluoromethyl)phenyl]-3-oxo-5,6,8,8a-tetrahydro-1H-imidazo[1,5-a]pyrazin-7-yl]-6-(2-ethoxypyridin-3-yl)pyridine-2-carbaldehyde (100 mg, 0.183 mmol), $NaH_2PO_4$ (44.0 mg, 0.366 mmol) and 2-methyl-2-butene (389 μL, 3.66 mmol) were stirred in tert-butanol (3 mL) and water (3 mL), and $NaClO_2$ (51.8 mg, 0.458 mmol) was added. The mixture was stirred at room temperature for 2 hr. To the mixture was added $Na_2S_2O_3$ aqueous solution, and the mixture was concentrated and extracted with ethyl acetate. The organic layer was dried over sodium sulfate and concentrated to give a residue. The residue was used in the next step without further purification.

To a solution of the obtained residue and N',N'-dimethylethane-1,2-diamine (16.1 mg, 0.183 mmol) in N,N-dimethylformamide (1 mL) were added N,N-diisopropylethylamine (63.8 μL, 0.366 mmol) and HATU (90.5 mg, 0.238 mmol), and the mixture was stirred at room temperature overnight. After completion of the reaction, the mixture was purified by silica gel column chromatography (NH, hexane/ethyl acetate) to give the title compound (61.0 mg).

$^1$H NMR (300 MHz, DMSO-$d_6$) δ1.37 (3H, t, J = 7.06 Hz), 2.19 (6H, s), 2.42 (2H, s), 2.67-2.89 (2H, m), 3.07-3.24 (1H, m), 3.34-3.43 (4H, m), 3.46-3.56 (1H, m), 3.70-3.79 (1H, m), 3.80-3.90 (1H, m), 3.93-4.03 (1H, m), 4.44 (2H, d, J = 6.97 Hz), 7.13 (1H, dd, J = 7.47, 4.91 Hz), 7.62 (2H, dd, J = 8.34, 4.49 Hz), 7.82-7.93(2H, m), 8.10 (1H, d, J = 8.62 Hz), 8.20 (1H, dd, J = 4.86, 1.93 Hz), 8.32 (1H,dd, J = 7.52, 1.93 Hz), 8.47-8.57 (1H, m).
MS: 632.5.

Example 7

3-[(8aR)-2-[4-chloro-2-(trifluoromethyl)phenyl]-3-oxo-5,6,8,8a-tetrahydro-1H-imidazo[1,5-a]pyrazin-7-yl]-6-(2-ethoxypyridin-3-yl)pyridine-2-carbaldehyde

The compound was synthesized according to the method shown in Example 5 or a method analogous thereto. MS: 546.3.

Example 8

3-[(8aR)-2-[4-chloro-2-(trifluoromethyl)phenyl]-3-oxo-5,6,8,8a-tetrahydro-1H-imidazo[1,5-a]pyrazin-7-yl]-N-[2-(dimethylamino)ethyl]-6-(2-ethoxypyridin-3-yl)pyridine-2-carboxamide

The compound was synthesized according to the method shown in Example 6 or a method analogous thereto. MS: 632.5.

Example 9

3-[2-[4-chloro-2-(trifluoromethyl)phenyl]-8a-methyl-3-oxo-1,5,6,8-tetrahydroimidazo[1,5-a]pyrazin-7-yl]-6-(2-ethoxypyridin-3-yl)pyridine-2-carbaldehyde

The compound was synthesized according to the method shown in Example 1 and Example 5 or a method analogous thereto. MS: 560.3.

Example 10

3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-(2-ethoxypyridin-3-yl)pyridine-2-carbaldehyde

A) methyl (2R)-2-[[(2R)-2-(benzyloxycarbonylamino)-3-hydroxy-propanoyl]amino]butanoate

HATU (66.8 g, 176 mmol) was added to a mixture of (2R)-2-(benzyloxycarbonylamino)-3-hydroxy-propanoic

acid (28.0 g, 117 mmol), methyl (2R)-2-aminobutanoate hydrochloride (21.6 g, 140 mmol) and N,N-diisopropylethylamine (45.4 g, 351 mmol) in acetonitrile (280 mL) at room temperature. The mixture was stirred at the same temperature over the weekend. Saturated aqueous sodium hydrogen carbonate solution was added to the mixture. The mixture was extracted with ethyl acetate (x3). The organic layer was washed with 1M hydrochloric acid, then water and saturated brine, dried over magnesium sulfate, and concentrated to give residue A. The aqueous layer of 1N hydrochloric acid was extracted with ethyl acetate, and the organic layer was washed with saturated brine, dried over magnesium sulfate, and concentrated to give residue B. The residues A and B were purified by silica gel column chromatography (ethyl acetate/hexane) to give a colorless solid. The solid was washed with toluene/ethyl acetate (2:1) to give colorless solid A (17.76 g). The filtrate was concentrated, and the residue was purified by silica gel column chromatography (NH, hexane/ethyl acetate) to give colorless solid B (16.31 g). The solids A and B were combined to give the title compound (34.0 g).

$^1$H NMR (300 MHz, DMSO-$d_6$) δ 0.86 (3H, t, J = 7.4 Hz), 1.54-1.80 (2H, m), 3.43-3.68 (5H, m), 4.07-4.28 (2H, m), 4.82 (1H, br s), 5.03 (2H, s), 7.22 (1H, br d, J= 8.3 Hz), 7.28-7.46 (5H, m), 8.17 (1H, br d, J = 7.5 Hz).
MS: 339.2.

B) (3R,6R)-3-ethyl-6-(hydroxymethyl)piperazine-2,5-dione

A mixture of methyl (2R)-2-[[(2R)-2-(benzyloxycarbonylamino)-3-hydroxy-propanoyl]amino]butanoate (34 g, 100 mmol) and 10% Pd/C (1.7 g, ca.55% in water) in methanol (340 mL) was stirred under a hydrogen atmosphere at room temperature for 16 hr. After completion of the reaction, the mixture was filtered through Celite and the filtrate was concentrated. The residue was used for the next reaction without further purification.

A mixture of the obtained residue and methanol (200 mL) was stirred at 80°C for 24 hr and concentrated. The residue was washed with ethyl acetate to give the title compound (12.1 g).

$^1$H NMR (300 MHz, DMSO-$d_6$) δ 0.87 (3H, t, J = 7.4 Hz), 1.61-1.89 (2H, m), 3.52 (1H, ddd, J = 10.7, 5.2, 3.0 Hz), 3.61-3.82 (3H, m), 5.01 (1H, t, J = 5.5 Hz), 7.88 (1H, br s), 8.15 (1H, br s).
MS: 173.2.

C) tert-butyl (2R,5S)-2-ethyl-5-(hydroxymethyl)piperazine-1-carboxylate

To a mixture of (3R,6R)-3-ethyl-6-(hydroxymethyl)piperazine-2,5-dione (2.85 g, 16.6 mmol) and tetrahydrofuran (20 mL) was added a borane-tetrahydrofuran complex (1M tetrahydrofuran solution, 122 mL, 122 mmol) at room temperature. The mixture was stirred at 70°C for 16 hr. After cooling to 0°C, methanol (40 mL) was added to the reaction mixture. Thereafter, 6M hydrochloric acid (20 mL) was added to the reaction mixture. The mixture was stirred at 70°C for 2 hr. The mixture was concentrated and toluene was added to the residue. The mixture was concentrated. Toluene was further added to the residue and the mixture was concentrated. The obtained residue was used for the next reaction without further purification.

To a mixture of the obtained residue in methanol (60 mL) and triethylamine (16.8 g, 166 mmol) was added di-tert-butyldicarbonate (14.5 g, 66.3 mmol) at 0°C. The mixture was stirred at 70°C for 16 hr. After completion of the reaction, the mixture was cooled to room temperature and concentrated.

An aqueous solution of water (30 mL) and sodium hydroxide (1.79 g, 44.8 mmol) was added to a mixture of ethanol (60 mL) and the residue at room temperature. The mixture was refluxed for 16 hr. After cooling to room temperature, an aqueous solution of water (15 mL) and sodium hydroxide (895 mg, 22.4 mmol) was added to the mixture.

The mixture was heated under reflux for 4 hr. After cooling to room temperature, the mixture was concentrated in vacuo to remove ethanol. The residue was adjusted to pH 8 to 9 by adding 1M HCl and diluted with ethyl acetate and tetrahydrofuran. Then, sodium chloride was added to the mixture. The mixture was extracted with ethyl acetate and tetrahydrofuran. The organic layer was separated, dried over magnesium sulfate, and concentrated. The residue was purified by silica gel column chromatography (ethyl acetate/methanol) to give the title compound (2.78 g).

$^1$H NMR (300 MHz, DMSO-$d_6$) δ 0.78 (3H, br t, J = 7.3 Hz), 1.39 (9H, s), 1.5-1.8 (2H, m), 2.1-2.5 (4H, m), 2.5-2.7 (1H, m), 2.79 (1H, br d, J = 11.6 Hz), 3.79 (2H, br d, J = 12.5 Hz), 4.62 (1H, br s).
MS: 245.3.

D) tert-butyl (2R,5S)-4-[[4-chloro-2-(trifluoromethyl)phenyl]carbamoyl]-2-ethyl-5-(hydroxymethyl)piperazine-1-carboxylate

To a mixture of tetrahydrofuran (100 mL), 4-chloro-2-(trifluoromethyl)aniline (2.61 g, 13.4 mmol) and triethylamine (3.38 g, 33.4 mmol) was added triphosgene (1.65 g, 5.57 mmol) at 0°C. After stirring at room temperature for 16 hr, a

mixture of tetrahydrofuran (20 mL), tert-butyl (2R,5S)-2-ethyl-5-(hydroxymethyl)piperazine-1-carboxylate (2.72 g, 11.1 mmol) and triethylamine (3.38 g, 33.4 mmol) was added to the reaction mixture. The mixture was stirred at room temperature for 2 hr, and water was added to the reaction mixture. The mixture was extracted with ethyl acetate. The organic layer was separated, washed with water and saturated brine, dried over magnesium sulfate, and concentrated. The residue was purified by silica gel column chromatography (hexane/ethyl acetate) to give the title compound (3.12 g).

$^1H$ NMR (300 MHz, DMSO-$d_6$) δ 0.85 (3H, t, J = 7.5Hz), 1.3-1.6 (11H, m), 2.7-2.9 (2H, m), 3.4-3.6 (2H, m), 3.8-4.1 (4H, m), 5.68 (1H, br s), 7.6-7.7 (3H, m), 8.70 (1H, br s).
MS: 488.3.

E) tert-butyl (6R,8aR)-2-[4-chloro-2-(trifluoromethyl)phenyl]-6-ethyl-3-oxo-5,6,8,8a-tetrahydro-1H-imidazo[1,5-a]pyrazine-7-carboxylate

Methanesulfonyl chloride (2.31 g, 20.2 mmol) was added dropwise to a mixture of tert-butyl (2R,5S)-4-[[4-chloro-2-(trifluoromethyl)phenyl]carbamoyl]-2-ethyl-5-(hydroxymethyl)piperazine-1-carboxylate (3.12 g, 6.70 mmol) and 1,8-diazabicyclo[5.4.0]-7-undecene (8.16 g, 53.6 mmol) in tetrahydrofuran (50 mL) at 0°C. The mixture was stirred at room temperature for 16 hr, and water was added to the reaction mixture. The mixture was extracted with ethyl acetate. The organic layer was separated, washed with water and saturated brine, dried over magnesium sulfate, and concentrated. The residue was purified by silica gel column chromatography (hexane/ethyl acetate) to give the title compound (2.34 g).

$^1H$ NMR (300 MHz, DMSO-$d_6$) δ 0.8-0.9 (3H, m), 1.4-1.7 (11H, m), 2.7-3.0 (1H, m), 3.03 (1H, td, J = 4.8, 13.6 Hz), 3.3-3.4 (1H, m), 3.58 (1H, br dd, J = 6.7, 13.3 Hz), 3.7-3.8 (2H, m), 3.9-4.1 (2H, m), 7.60 (1H, d, J = 8.1 Hz), 7.8-7.9 (2H, m).
MS: 448.3.

F) (6R,8aS)-2-[4-chloro-2-(trifluoromethyl)phenyl]-6-ethyl-1,5,6,7,8,8a-hexahydroimidazo[1,5-a]pyrazin-3-one

A mixture of tert-butyl (6R,8aR)-2-[4-chloro-2-(trifluoromethyl)phenyl]-6-ethyl-3-oxo-5,6,8,8a-tetrahydro-1H-imidazo[1,5-a]pyrazine-7-carboxylate (2.33 g, 5.20 mmol) and trifluoroacetic acid (20 mL) was stirred at room temperature for 2 hr, and the mixture was concentrated. To the residue was added toluene. The mixture was concentrated. The residue was diluted with ethyl acetate and saturated aqueous sodium hydrogen carbonate solution. The organic layer was separated, and sodium chloride was added to the aqueous layer. The aqueous layer was extracted with ethyl acetate. The combined organic layer was dried over magnesium sulfate and concentrated to give the title compound (1.81 g).

$^1H$ NMR (300 MHz, DMSO-$d_6$) δ 0.85 (3H, t, J = 7.4 Hz), 1.4-1.6 (2H, m), 2.6-2.8 (3H, m), 3.02 (1H, dd, J = 4.1, 12.9 Hz), 3.20 (1H, dd, J = 3.3, 7.9 Hz), 3.50 (1H, d, J = 12.9 Hz), 3.6-3.8 (2H, m), 7.55 (1H, d, J = 8.1 Hz), 7.8-7.9 (2H, m).
MS: 348.2.

G) 3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-bromo-pyridine-2-carbaldehyde

6-Bromo-3-fluoro-pyridine-2-carbaldehyde (733 mg, 3.59 mmol) and potassium carbonate (993 mg, 7.19 mmol) were added to a mixture of (6R,8aS)-2-[4-chloro-2-(trifluoromethyl)phenyl]-6-ethyl-1,5,6,7,8,8a-hexahydroimidazo[1,5-a]pyrazin-3-one (833 mg, 2.40 mmol) in toluene (3 mL) at room temperature. The mixture was stirred at 110°C for 48 hr. After cooling to room temperature, water was added to the reaction mixture. The mixture was extracted with ethyl acetate. The organic layer was separated, washed with water and saturated brine, dried over magnesium sulfate, and concentrated. The residue was purified by silica gel column chromatography (hexane/ethyl acetate) to give the title compound (661 mg).

$^1H$ NMR (300 MHz, DMSO-$d_6$) δ 0.72 (3H, t, J = 7.3 Hz), 1.4-1.7 (2H, m), 3.2-3.3 (1H, m), 3.3-3.5 (3H, m), 3.5-3.6 (1H, m), 3.70 (1H, d, J = 13.1 Hz), 3.8-4.0 (2H, m), 7.6-7.7 (2H, m), 7.7-7.7 (1H, m), 7.8-7.9 (2H, m), 9.81 (1H, s).
MS: 531.2.

H) 3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-(2-ethoxypyridin-3-yl)pyridine-2-carbaldehyde

$PdCl_2$(dppf)-$CH_2Cl_2$(99.9 mg, 0.122 mmol) was added to a mixture of 3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-bromo-pyridine-2-carbaldehyde (651 mg,

1.22 mmol), (2-ethoxy-3-pyridyl)boronic acid (225 mg, 1.35 mmol) and 2M sodium carbonate aqueous solution (1.84 mL, 3.67 mmol) in toluene (8 mL) at room temperature. The mixture was stirred at 90 °C for 16 hr. After cooling to room temperature, the mixture was diluted with ethyl acetate and water. The mixture was filtered through celite with ethyl acetate. The filtrate was extracted with ethyl acetate. The organic layer was separated, washed with water and saturated brine, dried over magnesium sulfate, and concentrated. The residue was purified by silica gel column chromatography (hexane/ethyl acetate) to give the title compound (390 mg).

$^1$H NMR (300 MHz, DMSO-$d_6$) δ 0.76 (3H, t, J = 7.3 Hz), 1.38 (3H, t, J = 7.1 Hz), 1.5-1.7 (2H, m), 3.2-3.3 (1H, m), 3.3-3.5 (3H, m), 3.5-3.7 (1H, m), 3.74 (1H, d, J = 13.1 Hz), 3.8-3.9 (1H, m), 3.9-4.0 (1H, m), 4.46 (2H, q, J = 7.1 Hz), 7.15 (1H, dd, J = 4.9, 7.5 Hz), 7.64 (1H, d, J = 8.1 Hz), 7.73 (1H, d, J = 8.9 Hz), 7.8-7.9 (2H, m), 8.2-8.3 (2H, m), 8.33 (1H, dd, J = 2.0, 7.5 Hz), 10.02 (1H, s).
MS: 574.3.

Example 11

3-[(8aS)-2-[4-chloro-2-(trifluoromethyl)phenyl]-3-oxo-5,6,8,8a-tetrahydro-1H-imidazo[1,5-a]pyrazin-7-yl]-6-(2-ethoxypyridin-3-yl)-N-(oxan-4-ylmethyl)pyridine-2-carboxamide

The compound was synthesized according to the method shown in Example 5 and Example 6 or a method analogous thereto. MS: 659.3.

Example 12

3-[(8aS)-2-[4-chloro-2-(trifluoromethyl)phenyl]-3-oxo-5,6,8,8a-tetrahydro-1H-imidazo[1,5-a]pyrazin-7-yl]-6-(2-ethoxypyridin-3-yl)-N-(2-methoxyethyl)pyridine-2-carboxamide

The compound was synthesized according to the method shown in Example 5 and Example 6 or a method analogous thereto. MS: 619.3.

Example 13

3-[(8aS)-2-[4-chloro-2-(trifluoromethyl)phenyl]-3-oxo-5,6,8,8a-tetrahydro-1H-imidazo[1,5-a]pyrazin-7-yl]-6-(2-ethoxypyridin-3-yl)-N-[2-(oxan-4-yl)ethyl]pyridine-2-carboxamide

The compound was synthesized according to the method shown in Example 5 and Example 6 or a method analogous thereto. MS: 673.3.

Example 14

3-[(8aS)-2-[4-chloro-2-(trifluoromethyl)phenyl]-3-oxo-5,6,8,8a-tetrahydro-1H-imidazo[1,5-a]pyrazin-7-yl]-6-(2-ethoxypyridin-3-yl)-N-(3-pyridin-2-ylpropyl)pyridine-2-carboxamide

The compound was synthesized according to the method shown in Example 5 and Example 6 or a method analogous thereto. MS: 680.3.

Example 15

3-[(8aS)-2-[4-chloro-2-(trifluoromethyl)phenyl]-3-oxo-5,6,8,8a-tetrahydro-1H-imidazo[1,5-a]pyrazin-7-yl]-6-(2-ethoxypyridin-3-yl)-N-[(3-hydroxyoxetan-3-yl)methyl]pyridine-2-carboxamide

The compound was synthesized according to the method shown in Example 5 and Example 6 or a method analogous thereto. MS:647.2.

Example 16

3-[(8aS)-2-[4-chloro-2-(trifluoromethyl)phenyl]-3-oxo-5,6,8,8a-tetrahydro-1H-imidazo[1,5-a]pyrazin-7-yl]-6-(2-ethoxypyridin-3-yl)-N-[(1-hydroxycyclopropyl)methyl]pyridine-2-carboxamide

The compound was synthesized according to the method shown in Example 5 and Example 6 or a method analogous thereto. MS: 631.3.

Example 17

tert-butyl 3-[[3-[(8aS)-2-[4-chloro-2-(trifluoromethyl)phenyl]-3-oxo-5,6,8,8a-tetrahydro-1H-imidazo[1,5-a]pyrazin-7-yl]-6-(2-ethoxypyridin-3-yl)pyridine-2-carbonyl]amino]pyrrolidine-1-carboxylate

The compound was synthesized according to the method shown in Example 5 and Example 6 or a method analogous thereto. MS: 730.4.

Example 18

3-[(8aS)-2-[4-chloro-2-(trifluoromethyl)phenyl]-3-oxo-5,6,8,8a-tetrahydro-1H-imidazo[1,5-a]pyrazin-7-yl]-6-(2-ethoxypyridin-3-yl)-N-(2-methylsulfonylethyl)pyridine-2-carboxamide

The compound was synthesized according to the method shown in Example 5 and Example 6 or a method analogous thereto. MS: 667.2.

Example 19

3-[(8aS)-2-[4-chloro-2-(trifluoromethyl)phenyl]-3-oxo-5,6,8,8a-tetrahydro-1H-imidazo[1,5-a]pyrazin-7-yl]-6-(2-ethoxypyridin-3-yl)-N-(2-pyridin-2-ylethyl)pyridine-2-carboxamide

The compound was synthesized according to the method shown in Example 5 and Example 6 or a method analogous thereto. MS: 666.3.

Example 20 3-[(8aS)-2-[4-chloro-2-(trifluoromethyl)phenyl]-3-oxo-5,6,8,8a-tetrahydro-1H-imidazo[1,5-a]pyrazin-7-yl]-6-(2-ethoxypyridin-3-yl)-N-(oxolan-3-yl)pyridine-2-carboxamide

The compound was synthesized according to the method shown in Example 5 and Example 6 or a method analogous thereto. MS:631.3.

Example 21

tert-butyl 4-[2-[[3-[(8aS)-2-[4-chloro-2-(trifluoromethyl)phenyl]-3-oxo-5,6,8,8a-tetrahydro-1H-imidazo[1,5-a]pyrazin-7-yl]-6-(2-ethoxypyridin-3-yl)pyridine-2-carbonyl]amino]ethyl]piperidine-1-carboxylate

The compound was synthesized according to the method shown in Example 5 and Example 6 or a method analogous thereto. MS: 772.4.

Example 22

3-[(8aS)-2-[4-chloro-2-(trifluoromethyl)phenyl]-3-oxo-5,6,8,8a-tetrahydro-1H-imidazo[1,5-a]pyrazin-7-yl]-6-(2-ethoxypyridin-3-yl)-N-(3-pyrazol-1-ylpropyl)pyridine-2-carboxamide

The compound was synthesized according to the method shown in Example 5 and Example 6 or a method analogous thereto. MS: 669.3.

Example 23

3-[(8aS)-2-[4-chloro-2-(trifluoromethyl)phenyl]-3-oxo-5,6,8,8a-tetrahydro-1H-imidazo[1,5-a]pyrazin-7-yl]-6-(2-ethoxypyridin-3-yl)-N-[(3R)-1-methylpyrrolidin-3-yl]pyridine-2-carboxamide

The compound was synthesized according to the method shown in Example 5 and Example 6 or a method

analogous thereto. MS: 644.3.

Example 24

3-[(8aS)-2-[4-chloro-2-(trifluoromethyl)phenyl]-3-oxo-5,6,8,8a-tetrahydro-1H-imidazo[1,5-a]pyrazin-7-yl]-6-(2-ethoxypyridin-3-yl)-N-pyrrolidin-3-ylpyridine-2-carboxamide 2hydrochloride

The compound was synthesized by treating tert-butyl 3-[[3-[(8aS)-2-[4-chloro-2-(trifluoromethyl)phenyl]-3-oxo-5,6,8,8a-tetrahydro-1H-imidazo[1,5-a]pyrazin-7-yl]-6-(2-ethoxypyridin-3-yl)pyridine-2-carbonyl]amino]pyrrolidine-1-carboxylate with hydrochloric acid, followed by concentration. MS: 630.3.

Example 25

3-[(8aS)-2-[4-chloro-2-(trifluoromethyl)phenyl]-3-oxo-5,6,8,8a-tetrahydro-1H-imidazo[1,5-a]pyrazin-7-yl]-6-(2-ethoxypyridin-3-yl)-N-(2-piperidin-4-ylethyl)pyridine-2-carboxamide 2 hydrochloride

The compound was synthesized by treating tert-butyl 4-[2-[[3-[(8aS)-2-[4-chloro-2-(trifluoromethyl)phenyl]-3-oxo-5,6,8,8a-tetrahydro-1H-imidazo[1,5-a]pyrazin-7-yl]-6-(2-ethoxypyridin-3-yl)pyridine-2-carbonyl]amino]ethyl]piperidine-1-carboxylate with hydrochloric acid, followed by concentration. MS: 672.3.

Example 26

3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-N-[2-(dimethylamino)ethyl]-6-(2-ethoxypyridin-3-yl)pyridine-2-carboxamide

The compound was synthesized according to the method shown in Example 10 and Example 38 or a method analogous thereto.

$^1$H NMR (300 MHz, CHLOROFORM-d) δ 0.81-0.93 (3H, m), 1.39-1.51 (4H, m), 1.73 (1H, br dd, J = 6.83, 3.26 Hz), 2.32 (6H, s), 2.57 (2H, t, J = 6.10 Hz), 3.14 (1H, br dd, J = 11.65, 4.31 Hz), 3.41-3.62 (6H, m), 3.79 (1H, t, J = 8.62 Hz), 3.98 (1H, d, J = 12.38 Hz), 4.12 (1H, br dd, J = 10.22, 4.45 Hz), 4.46-4.55 (2H, m), 7.01 (1H, dd, J = 7.52, 4.86 Hz), 7.36 (2H, t, J = 7.84 Hz), 7.59 (1H, dd, J = 8.48, 2.34 Hz), 7.71 (1H, d, J = 2.38 Hz),
8.15-8.17 (1H, m), 8.18 (1H, s), 8.30-8.40 (2H, m).

Example 27

3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-(2-ethoxypyridin-3-yl)pyridine-2-carboxamide

The compound was synthesized according to the method shown in Example 10 and Example 38 or a method analogous thereto.
MS: 589.2.

Example 28

3-[(8aS)-2-[4-chloro-2-(trifluoromethyl)phenyl]-3-oxo-5,6,8,8a-tetrahydro-1H-imidazo[1,5-a]pyrazin-7-yl]-6-(2-ethoxypyridin-3-yl)-N-pyrrolidin-3-ylpyridine-2-carboxamide

To a mixture of 3-[(8aS)-2-[4-chloro-2-(trifluoromethyl)phenyl]-3-oxo-5,6,8,8a-tetrahydro-1H-imidazo[1,5-a]pyrazin-7-yl]-6-(2-ethoxy-3-pyridyl)pyridine-2-carboxylic acid (150 mg, 0.267 mmol) and tert-butyl 3-aminopyrrolidine-1-carboxylate (59.7 mg, 0.320 mmol) in N,N-dimethylformamide (1 mL) were added N,N-diisopropylethylamine (93.0 μL, 0.534 mmol) and HATU (132 mg, 0.347 mmol). The mixture was stirred at room temperature overnight. Water was added to the mixture, and the mixture was extracted with ethyl acetate. The organic layer was separated, concentrated, and the residue was purified by silica gel column chromatography (ethyl acetate/methanol) to give a crude product. The crude product was used for the next step without further purification.

To the crude product and tetrahydrofuran (2.0 mL) was added 6M hydrochloric acid, and the mixture was stirred at room temperature 5 hr. The mixture was basified with sodium hydroxide aqueous solution and potassium carbonate aqueous solution and extracted with ethyl acetate. The organic layer was separated, dried over magnesium sulfate, and

concentrated. The residue was dissolved in acetonitrile and water and freeze-dried to give the title compound.
MS: 630.4.

Example 29

3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-(2-ethoxypyridin-3-yl)-N-(5-oxopyrrolidin-3-yl)pyridine-2-carboxamide

The compound was synthesized according to the method shown in Example 10 and Example 38 or a method analogous thereto.
MS: 672.3.

Example 30

3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-(2-ethoxypyridin-3-yl)-N-[(3-hydroxyoxetan-3-yl)methyl]pyridine-2-carboxamide

The compound was synthesized according to the method shown in Example 10 and Example 38 or a method analogous thereto.
MS: 675.3.

Example 31

3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-(2-ethoxypyridin-3-yl)-N-[(3R)-pyrrolidin-3-yl]pyridine-2-carboxamide

The compound was synthesized according to the method shown in Example 10, Example 28 and Example 38 or a method analogous thereto.
MS 658.3.

Example 32

3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-(2-ethoxypyridin-3-yl)-N-[(1-hydroxycyclopropyl)methyl]pyridine-2-carboxamide

The compound was synthesized according to the method shown in Example 10 and Example 38 or a method analogous thereto.
MS: 659.3.

Example 33

3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-(2-ethoxypyridin-3-yl)-N-[(2R)-2-hydroxypropyl]pyridine-2-carboxamide

The compound was synthesized according to the method shown in Example 10 and Example 38 or a method analogous thereto.
MS: 647.3.

Example 34

3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-(2-ethoxypyridin-3-yl)-N-[(2S)-2-hydroxypropyl]pyridine-2-carboxamide

The compound was synthesized according to the method shown in Example 10 and Example 38 or a method analogous thereto.
MS: 647.3.

Example 35

3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-(2-ethoxypyridin-3-yl)-N-(2-hydroxy-2-methylpropyl)pyridine-2-carboxamide

The compound was synthesized according to the method shown in Example 10 and Example 38 or a method analogous thereto.
MS: 661.3.

Example 36

N-(2-acetamidoethyl)-3-[(6R,8aS)-2-[4-chloro-2-(trifluoromethyl)phenyl]-6-ethyl-3-oxo-5,6,8,8a-tetrahydro-1H-imidazo [1,5-a]pyrazin-7-yl]-6-(2-ethoxypyridin-3-yl)pyridine-2-carboxamide

The compound was synthesized according to the method shown in Example 10 and Example 38 or a method analogous thereto.
MS: 674.3.

Example 37

3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-(2-ethoxypyridin-3-yl)-N-[2-(methylamino)-2-oxoethyl]pyridine-2-carboxamide

The compound was synthesized according to the method shown in Example 10 and Example 38 or a method analogous thereto.
MS: 660.3.

Example 38

3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-(2-ethoxypyridin-3-yl)-N-[(3R)-5-oxopyrrolidin-3-yl]pyridine-2-carboxamide

Example 38a

3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-(2-ethoxy-3-pyridyl)pyridine-2-carboxylic acid

A) 3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-(2-ethoxy-3-pyridyl)pyridine-2-carboxylic acid

$NaClO_2$ (507 mg, 5.60 mmol) was added to a mixture of 3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-(2-ethoxy-3-pyridyl)pyridine-2-carbaldehyde (1.61 g, 2.80 mmol), $NaH_2PO_4$ (1.34 g, 11.2 mmol) and 2-methyl-2-butene (1.38 g, 19.6 mmol) in tert-butanol (10 mL), tetrahydrofuran (5 mL) and water (5 mL) at room temperature. The reaction mixture was stirred at room temperature for 3 hr. To the reaction mixture were added saturated sodium thiosulfate aqueous solution and saturated ammonium chloride aqueous solution. The mixture was diluted with ethyl acetate, and organic layer was separated. NaCl was added to the aqueous layer. The aqueous layer was extracted with ethyl acetate. The combined organic layer was dried over magnesium sulfate and concentrated to give the title compound (1.65 g).

$^1H$ NMR (300 MHz, DMSO-$d_6$) δ 0.79 (3H, t, J = 7.3 Hz), 1.38 (3H, t, J = 7.1 Hz), 1.5-1.7 (2H, m), 3.2-3.3 (4H, m), 3.5-3.6 (1H, m), 3.73 (1H, d, J = 13.1 Hz), 3.8-4.0 (2H, m), 4.44 (2H, q, J = 7.1 Hz), 7.11 (1H, dd, J = 4.9, 7.5 Hz), 7.62 (2H, dd, J = 2.9, 8.5 Hz), 7.8-8.0 (2H, m), 8.10 (1H, d, J = 8.8 Hz), 8.19 (1H, dd, J = 1.9, 4.9 Hz), 8.27 (1H, dd, J = 1.9, 7.5 Hz), 12.5-13.8 (1H, m).
MS: 590.3.

B) 3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-(2-ethoxypyridin-3-yl)-N-[(3R)-5-oxopyrrolidin-3-yl]pyridine-2-carboxamide

To a mixture of ethyl acetate-ethanol (2 mL) in tert-butyl N-[(3R)-5-oxopyrrolidin-3-yl]carbamate (255 mg, 1.27 mmol) was added 4N hydrogen chloride/cyclopentylmethylether solution (12 mL) at room temperature. After stirring at

room temperature for 3 hr, the mixture was concentrated.

The obtained residue was dissolved in N,N-dimethylformamide (10 mL), and 3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-(2-ethoxy-3-pyridyl)pyridine-2-carboxylic acid (600 mg, 1.02 mmol) and N,N-diisopropyl ethylamine (0.522 mL, 3.05 mmol) were added. After stirring at room temperature for 10 min, HATU (464 mg, 1.22 mmol) was added at room temperature, and the mixture was stirred at room temperature for 18 hr. The mixture was diluted with water and extracted twice with ethyl acetate/diethyl ether (2: 1). The combined extract was washed with saturated brine, dried over magnesium sulfate, and concentrated. The residue was purified by silica gel column chromatography (NH silica gel, hexane/ethyl acetate and ethyl acetate/methanol) to give the title compound (580 mg).

$^1$H NMR (300 MHz, DMSO-$d_6$) δ 0.77 (3H, t, J = 7.2 Hz), 1.37 (4H, t, J = 7.0 Hz), 1.48-1.73 (1H, m), 2.28 (1H, dd, J = 16.8, 5.6 Hz), 2.53-2.63 (1H, m), 3.11-3.27 (4H, m), 3.34-3.38 (1H, m), 3.41-3.53 (1H, m), 3.60 (1H, dd, J = 9.5, 7.3 Hz), 3.72 (1H, d, J = 13.0 Hz), 3.80-3.98 (2H, m), 4.44 (2H, q, J = 7.0 Hz), 4.50-4.70 (1H, m), 7.13 (1H, dd, J = 7.4, 4.9 Hz), 7.55 (1H, d, J = 8.8 Hz), 7.61-7.71 (2H, m), 7.82-7.93 (2H, m), 8.07 (1H, d, J = 8.8 Hz), 8.18 (1H, dd, J = 4.9, 2.0Hz), 8.32 (1H, dd, J = 7.5, 2.0 Hz), 8.86 (1H, d, J = 6.4 Hz).
MS: 674.4.

Example 39

3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-(2-ethoxypyridin-3-yl)-N-[(3S)-5-oxopyrrolidin-3-yl]pyridine-2-carboxamide

To a mixture of ethyl acetate -ethanol (0.5 mL) in tert-butyl N-[(3S)-5-oxopyrrolidin-3-yl]carbamate (27 mg, 0.135 mmol) was added 4N hydrogen chloride/cyclopentylmethylether solution (0.500 mL, 2.00 mmol) at room temperature. After stirring at room temperature for 3 hr, the mixture was concentrated.

The obtained residue was dissolved in N,N-dimethylformamide (1 mL), and 3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-(2-ethoxy-3-pyridyl)pyridine-2-carboxylic acid (25.0 mg, 0.0424 mmol) and N,N-diisopropyl ethylamine (0.0580 mL, 0.339 mmol) were added. After stirring at room temperature for 10 min, HATU (0.0322 g, 0.0847 mmol) was added at room temperature, and the mixture was stirred at room temperature for 18 hr. The mixture was diluted with water and extracted twice with ethyl acetate/diethyl ether (2: 1). The combined extract was washed with saturated brine, dried over magnesium sulfate, and concentrated. The residue was purified by silica gel column chromatography (NH silica gel, hexane/ethyl acetate and ethyl acetate/methanol) to give the title compound (0. 0180 g).

$^1$H NMR (300 MHz, DMSO-$d_6$) δ 0.77 (3H, t, J = 7.3 Hz), 1.32-1.50 (4H, m), 1.52-1.70 (1H, m), 2.24 (1H, dd, J = 16.7, 5.6 Hz), 2.52-2.60 (1H, m), 3.11-3.41 (5H, m), 3.42-3.53 (1H, m), 3.61 (1H, dd, J = 9.9, 7.4 Hz), 3.71 (1H, d, J = 12.9 Hz), 3.80-3.98 (2H, m), 4.44 (2H, q, J = 7.0 Hz), 4.51-4.65 (1H, m), 7.12 (1H, dd, J = 7.5, 4.9 Hz), 7.55 (1H, d, J = 8.8 Hz), 7.60-7.71 (2H, m), 7.84-7.92 (2H, m), 8.07 (1H, d, J = 8.7 Hz), 8.18 (1H, dd, J = 4.9, 2.0 Hz), 8.31 (1H, dd, J = 7.5, 2.0 Hz), 8.84 (1H, d, J = 6.8 Hz).
MS: 672.3.

Example 40

3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-(2-ethoxypyridin-3-yl)-N-[(2R)-1-(methylamino)-1-oxopropan-2-yl]pyridine-2-carboxamide

The compound was synthesized according to the method shown in Example 10 and Example 38 or a method analogous thereto.
MS: 674.3.

Example 41

3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-(2-ethoxypyridin-3-yl)-N-[(2S)-1-(methylamino)-1-oxopropan-2-yl]pyridine-2-carboxamide

The compound was synthesized according to the method shown in Example 10 and Example 38 or a method analogous thereto.
MS: 674.3.

Example 42

3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-(2-ethoxypyridin-3-yl)-N-(1-methyl-5-oxopyrrolidin-3-yl)pyridine-2-carboxamide

To a solution of 3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-(2-ethoxy-3-pyridyl)pyridine-2-carboxylic acid (20.0 mg,0.0339 mmol), 4-amino-1-methylpyrrolidin-2-one hydrochloride (15.3 mg, 0.102 mmol) and N,N-diisopropylethylamine (0.0464 mL, 0.271 mmol) in N,N-dimethylformamide (0.5 mL) was added HATU (0.0258 g, 0.0678 mmol) at room temperature. The mixture was stirred at room temperature for 18 hr, diluted with water, and extracted twice with ethyl acetate-diethyl ether (2:1). The combined extract was washed with saturated brine, dried over magnesium sulfate, and concentrated. The residue was purified by silica gel column chromatography (NH silica gel, hexane/ethyl acetate and methanol/ethyl acetate) to give the title compound (0.0150 g). $^1H$ NMR (300 MHz, DMSO-$d_6$) $\delta$ 0.77 (3H, t, J = 7.3 Hz), 1.30-1.48 (4H, m), 1.52-1.69 (1H, m), 2.28-2.43 (1H, m), 2.73 (3H, d, J = 3.6 Hz), 3.10-3.40 (6H, m), 3.41-3.53 (1H, m), 3.66-3.79 (2H, m), 3.81-3.98 (2H, m), 4.37-4.60 (3H, m), 7.13 (1H, dd, J = 7.5, 4.9 Hz), 7.55 (1H, dd, J = 8.8, 1.5 Hz), 7.63 (1H, d, J = 8.1 Hz), 7.84-7.91 (2H, m), 8.07 (1H, d, J = 8.8 Hz), 8.18 (1H, dd, J = 4.9, 1.9 Hz), 8.31 (1H, dd, J = 7.5, 2.0 Hz), 8.86 (1H, dd, J = 6.6, 2.6 Hz).
MS: 686.3.

Example 43

tert-butyl (2R)-2-[[[3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-(2-ethoxypyridin-3-yl)pyridine-2-carbonyl]amino]methyl]azetidine -1-carboxylate

To a stirred solution of tetrahydrofuran-water (1.00 mL, 10:1) in tert-butyl (2R)-2-(azidomethyl)azetidine-1-carboxylate (32.4 mg, 0.153 mmol) was added triphenyl phosphine (44.0 mg, 0.168 mmol) at room temperature. After stirring at 60°C for 2 hr, the mixture was concentrated, and azeotropically distilled twice with toluene.

The obtained residue was dissolved in N,N-dimethylformamide (1 mL), and then 3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-(2-ethoxy-3-pyridyl)pyridine-2-carboxylic acid (30 mg, 0.0508 mmol) and N,N-diisopropylethylamine(0.0435 mL, 0.254 mmol) were added. To this mixture was added HATU (0.0387 g, 0.102 mmol) at room temperature. The mixture was stirred at room temperature for 18 hr, diluted with water, and extracted twice with ethyl acetate-diethyl ether (2:1). The combined extract was washed with saturated brine, dried over magnesium sulfate, and concentrated. The residue was purified by silica gel column chromatography (NH silica gel, hexane/ethyl acetate) to give the title compound (0.0250 g).

$^1H$ NMR (300 MHz, $CDCl_3$) $\delta$ 0.87 (3H, t, J = 7.3 Hz), 1.51 (13H, s), 1.65-1.82 (1H, m), 1.96-2.13 (1H, m), 2.20-2.36 (1H, m), 3.13 (1H, dd, J = 11.4, 4.3 Hz), 3.34-3.67 (5H, m), 3.71-4.05 (5H, m), 4.07-4.21 (1H, m), 4.39-4.57 (3H, m), 7.02 (1H, dd, J = 7.4, 4.9 Hz), 7.34 (1H, d, J = 8.6 Hz), 7.38 (1H, d, J = 8.8 Hz), 7.42-7.51 (1H, m), 7.56-7.62 (1H, m), 7.69-7.73 (1H, m), 8.10-8.25 (2H, m), 8.39-9.16 (1H, m).
MS: 758.4.

Example 44

3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-N-[[(2R)-azetidin-2-yl]methyl]-6-(2-ethoxypyridin-3-yl)pyridine-2-carboxamide

tert-Butyl (2R)-2-[[[3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-(2-ethoxypyridin-3-yl)pyridine-2-carbonyl]amino]methyl]azetidine-1-carboxylate (22 mg, 0.0290 mmol) and formic acid (2 mL) were stirred at 50°C for 15 min. The mixture was concentrated, and the obtained residue was purified by column chromatography (NH silica gel, ethyl acetate/methanol) to give the title compound (0.0150 g).

$^1H$ NMR (300 MHz, $CDCl_3$) $\delta$ 0.77 (3H, t, J = 7.3 Hz), 1.32-1.49 (4H, m), 1.51-1.70 (1H, m), 1.99-2.10 (1H, m), 2.13-2.25 (1H, m), 2.42-2.49 (1H, m), 3.09-3.57 (9H, m), 3.69 (1H, d, J = 12.7 Hz), 3.78-4.00 (3H, m), 4.44 (2H, q, J = 7.0 Hz), 7.12 (1H, dd, J = 7.5, 4.9 Hz), 7.54 (1H, d, J = 8.8 Hz), 7.63 (1H, d, J = 8.1 Hz), 7.81-7.92 (2H, m), 8.08 (1H, d, J = 8.7 Hz), 8.18 (1H, dd, J = 4.9, 1.9 Hz), 8.35 (1H, dd, J = 7.5, 1.9 Hz), 8.56 (1H, t, J = 5.9 Hz).
MS: 658.3.

Example 45

tert-butyl (2S)-2-[[[3-[(6R,8aS)-2-[4-chloro-2-(trifluoromethyl)phenyl]-6-ethyl-3-oxo-5,6,8,8a-tetrahydro-1H-imidazo [1,5-a]pyrazin-7-yl]-6-(2-ethoxypyridin-3-yl)pyridine-2-carbonyl]amino]methyl]azetidine-1-carboxylate

The compound was synthesized according to the method shown in Example 43 or a method analogous thereto. MS: 758.4.

Example 46

3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-N-[[(2S)-azetidin-2-yl]methyl]-6-(2-ethoxypyridin-3-yl)pyridine-2-carboxamide

The compound was synthesized according to the method shown in Example 44 or a method analogous thereto. MS:658.3.

Example 47

3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-(2-ethoxypyridin-3-yl)-N-[(3R)-2-oxopyrrolidin-3-yl]pyridine-2-carboxamide

The compound was synthesized according to the method shown in Example 10 and Example 38 or a method analogous thereto.
MS: 672.3.

Example 48

3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-(2-ethoxypyridin-3-yl)-N-[(3S)-2-oxopyrrolidin-3-yl]pyridine-2-carboxamide

The compound was synthesized according to the method shown in Example 10 and Example 38 or a method analogous thereto.
MS: 672.3.

Example 49

3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-(2-ethoxypyridin-3-yl)-N-[(3R)-1-methyl-5-oxopyrrolidin-3-yl]pyridine-2-carboxamide

A) 1-[(1R)-1-(4-methoxyphenyl)ethyl]-5-oxo-pyrrolidine-3-carboxylic acid

A mixture of (1R)-1-(4-methoxyphenyl)ethaneamine (18.0 g, 119 mmol) and itaconic acid (15.5 g, 119 mmol) in N-methyl-2-pyrrolidone (10 mL) was stirred at 130°C for 4 hr. Water (200 mL) was added to the reaction mixture. The mixture was stirred at room temperature for 16 hr. The precipitate was filtered with filter paper, washed with water and vacuum dried at 65°C to give the title compound (28.8 g).

$^1$H NMR (DMSO-$d_6$, 300 MHz) δ 1.4-1.5 (3H, m), 2.4-2.5 (1H, m), 2.5-2.6 (1H, m), 3.0-3.2 (2H, m), 3.4-3.6 (1H, m), 3.74 (3H, s), 5.19 (1H, q, J = 7.1 Hz), 6.8-7.0 (2H, m), 7.20 (2H, t, J = 8.1 Hz), 12.60 (1H, br s).
MS: 264.2.

B) benzyl (3R)-1-[(1R)-1-(4-methoxyphenyl)ethyl]-5-oxo-pyrrolidine-3-carboxylate

To a mixture of N-methyl-2-pyrrolidone (70 mL), 1-[(1R)-1-(4-methoxyphenyl)ethyl]-5-oxo-pyrrolidine-3-carboxylic acid (12.2 g, 46.2 mmol) and cesium carbonate (18.1 g, 55.5 mmol) was added benzyl bromide (9.49 g, 55.5 mmol) at room temperature. The mixture was stirred at the same temperature for 14 hr, and water was added to the reaction mixture. The mixture was diluted with ethyl acetate and extracted with ethyl acetate. The organic layer was separated, washed with water and saturated brine, dried over magnesium sulfate, and concentrated. The residue was purified by silica gel column chromatography (hexane/ethyl acetate) to give the title compound (7.06 g).

$^1$H NMR (CHLOROFORM-d, 300 MHz) δ 1.47 (3H, d, J = 7.2 Hz), 2.6-2.8 (2H, m), 3.0-3.2 (2H, m), 3.52 (1H, dd, J = 5.5,

9.1 Hz), 3.79 (3H, s), 5.14 (2H, s), 5.44 (1H, q, J = 7.2 Hz), 6.86 (2H, d, J = 8.7 Hz), 7.21 (2H, d, J=8.5 Hz), 7.3-7.4 (5H, m).
MS: 354.3.

C) (3R)-1-[(1R)-1-(4-methoxyphenyl)ethyl]-5-oxo-pyrrolidine-3-carboxylic acid

To a solution of benzyl (3R)-1-[(1R)-1-(4-methoxyphenyl)ethyl]-5-oxo-pyrrolidine-3-carboxylate (29.3 g, 83.0 mmol) in ethyl acetate (300 mL) and ethanol (180 mL) was added 10% Pd/C (4.00 g, ca.55% in water) at room temperature. The mixture was stirred under a hydrogen atmosphere at the same temperature for 6 hr. Insoluble materials were filtered off through celite, washed with ethyl acetate and ethanol, and the filtrate was concentrated. To the residue was added toluene, and the mixture was concentrated. To the residue was further added toluene and the mixture was concentrated to give the title compound (20.7 g).

$^1$H NMR (CHLOROFORM-d, 300 MHz) δ 1.51 (3H, d, J = 7.2 Hz), 2.7-2.9 (2H, m), 3.0-3.3 (2H, m), 3.58 (1H, dd, J = 5.7, 9.7 Hz), 3.80 (3H, s), 5.45 (1H, q, J = 7.1Hz), 6.87 (2H, d, J = 8.8 Hz), 7.22 (2H, d, J = 8.6 Hz).
MS: 264.2.

D) tert-butyl N-[(3R)-1-[(1R)-1-(4-methoxyphenyl)ethyl]-5-oxopyrrolidin-3-yl]carbamate

To a mixture of (3R)-1-[(1R)-1-(4-methoxyphenyl)ethyl]-5-oxo-pyrrolidine-3-carboxylic acid (20.7 g, 78.5 mmol) and triethylamine (15.9 g, 157 mmol) in tert-butanol (90 mL) and toluene (180 mL) was added DPPA (32.4 g, 118 mmol) at room temperature. The mixture was stirred at 80°C for 16 hr. To the mixture was added saturated aqueous sodium hydrogen carbonate solution at room temperature, and the mixture was extracted with ethyl acetate. The organic layer was separated, washed with water and saturated brine, dried over magnesium sulfate, and concentrated. The residue was crystallized from ethyl acetate/diisopropyl ether/hexane to give the title compound (18.7 g).

$^1$H NMR (DMSO-$d_6$, 300 MHz) δ 1.3-1.5 (12H, m), 2.23 (1H, dd, J = 6.1, 16.7 Hz), 2.5-2.6 (1H, m), 3.0-3.2 (2H, m), 3.74 (3H, s), 3.9-4.1 (1H, m), 5.20 (1H, q, J = 7.2 Hz), 6.9-7.0 (2H, m), 7.1-7.2 (2H, m), 7.2-7.3 (1H, m).
MS: 335.2.

E) tert-butyl N-[(3R)-5-oxopyrrolidin-3-yl]carbamate

A solution of ammonium cerium(IV) nitrate (50.6 g, 92.3 mmol) in acetonitrile (30 mL) and water (30 mL) was added to a mixture of acetonitrile (100 mL), water (100 mL) and tert-butyl N-[(3R)-1-[(1R)-1-(4-methoxyphenyl)ethyl]-5-oxo-pyrrolidin-3-yl]carbamate (12.9 g, 38.5 mmol) under ice-cooling. The mixture was stirred at room temperature for 2 hr. After completion of the reaction, saturated aqueous sodium hydrogen carbonate solution was added to the reaction mixture at 0°C to adjust to pH6. The mixture was diluted with ethyl acetate and tetrahydrofuran, and sodium chloride was added. The mixture was extracted with ethyl acetate/tetrahydrofuran. The organic layer was separated, dried over magnesium sulfate, and concentrated. The residue was washed with diisopropyl ether to give the title compound (6.9 g).

$^1$H NMR (300 MHz, DMSO-$d_6$) δ 1.38 (9H, s), 2.04 (1H, dd, J = 16.6, 6.1 Hz), 2.36 (1H, dd, J = 16.6, 8.3 Hz), 2.99 (1H, dd, J = 9.6, 4.9 Hz), 3.43 (1H, dd, J = 9.8, 7.2 Hz), 3.99-4.25 (1H, m), 7.14-7.38 (1H, m), 7.54 (1H, br s).
MS: 145.2.

F) tert-butyl N-[(3R)-1-methyl-5-oxo-pyrrolidin-3-yl]carbamate

To a mixture of tert-butyl N-[(3R)-5-oxopyrrolidin-3-yl]carbamate (131 mg, 0.655 mmol) and potassium tert-butoxide (80.9 mg, 0.721 mmol) in tetrahydrofuran (5 mL) was added dropwise iodomethane (102 mg, 0.721 mmol) at 0°C. The mixture was stirred at room temperature for 16 hr, and water was added to the reaction mixture. The mixture was diluted with ethyl acetate. Sodium chloride was added to the mixture. The mixture was extracted with ethyl acetate/tetrahydrofuran. The organic layer was separated, washed with water and saturated brine, dried over magnesium sulfate, and concentrated. The residue was purified by silica gel column chromatography (ethyl acetate/methanol) to give the title compound (134 mg).

$^1$H NMR (DMSO-$d_6$, 300 MHz) δ 1.38 (9H, s), 2.13 (1H, dd, J = 5.3, 16.8 Hz), 2.4-2.5 (1H, m), 2.68 (3H, s), 3.10 (1H, dd, J = 4.4, 10.0 Hz), 3.55 (1H, dd, J = 7.3, 10.0 Hz), 3.9-4.1 (1H, m), 7.26 (1H, br d, J = 5.4 Hz).
MS: 159.2.

G) (4R)-4-amino-1-methyl-pyrrolidin-2-one 4-methylbenzenesulfonate

p-Toluenesulfonic acid monohydrate (3.24 g, 17.0 mmol) was added to a mixture of acetonitrile (40 mL) and tert-butyl N-[(3R)-1-methyl-5-oxo-pyrrolidin-3-yl]carbamate (2.43 g, 11.4 mmol) at room temperature. The mixture was stirred at 60°C for 4 hr, cooled to room temperature, and concentrated. The residue was crystallized from ethanol/ethyl acetate/acetonitrile to give the title compound (2.71 g).

$^1$H NMR (DMSO-$d_6$, 300 MHz) δ 2.2-2.3 (4H, m), 2.6-2.8 (4H, m), 3.25 (1H, dd, J =2.9, 11.1 Hz), 3.67 (1H, dd, J = 7.2, 11.1 Hz), 3.8-4.0 (1H, m), 7.12 (2H, d, J= 7.8 Hz), 7.4-7.6 (2H, m), 8.04 (3H, br s).
MS: 115.3.

H) 3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-(2-ethoxypyridin-3-yl)-N-[(3R)-1-methyl-5-oxopyrrolidin-3-yl]pyridine-2-carboxamide

HATU (1.70 g, 4.48 mmol) was added to a mixture of 3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-(2-ethoxy-3-pyridyl)pyridine-2-carboxylic acid (1.65 g, 2.80 mmol), (4R)-4-amino-1-methyl-pyrrolidin-2-one 4-methylbenzenesulfonate (1.04 g, 3.64 mmol) and triethylamine (1.13 g, 11.2 mmol) in acetonitrile (15 mL) at room temperature. The reaction mixture was stirred at room temperature for 16 hr, and saturated aqueous sodium hydrogen carbonate solution was added. The mixture was extracted with ethyl acetate, and the organic layer was separated, washed with water and saturated brine, dried over magnesium sulfate, and concentrated. The residue was purified by silica gel column chromatography (NH silica gel, ethyl acetate/methanol) to give a solid. The solid was crystallized from ethanol-water to give the title compound (1.44 g).

$^1$H NMR (DMSO-$d_6$, 300 MHz) δ 0.77 (3H, t, J = 7.3 Hz), 1.3-1.5 (4H, m), 1.5-1.7 (1H, m), 2.37 (1H, dd, J = 5.3, 16.7 Hz), 2.6-2.8 (4H, m), 3.1-3.3 (4H, m), 3.37 (1H, s), 3.4-3.5 (1H, m), 3.7-3.8 (2H, m), 3.8-4.0 (2H, m), 4.44 (2H, q, J = 7.0 Hz), 4.5-4.6 (1H, m), 7.13 (1H, dd, J = 4.9, 7.5 Hz), 7.55 (1H, d, J = 8.8 Hz), 7.63 (1H, d, J = 8.2 Hz), 7.8-8.0 (2H, m), 8.07 (1H, d, J = 8.8 Hz), 8.18 (1H,dd, J = 2.0, 4.8 Hz), 8.32 (1H, dd, J = 1.9, 7.4 Hz), 8.86 (1H, d, J = 6.6 Hz).
MS: 686.4.

Example 50

3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-(2-ethoxypyridin-3-yl)-N-[(3S)-1-methyl-5-oxopyrrolidin-3-yl]pyridine-2-carboxamide

HATU (23 mg, 0.061 mmol) was added to a mixture of 3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-(2-ethoxy-3-pyridyl)pyridine-2-carboxylic acid (30 mg, 0.508 mmol), (4S)-4-amino-1-methyl-pyrrolidin-2-one (7.27 mg, 0.0637 mmol) and N,N-diisopropyl ethylamine (13.1 mg) in N,N-dimethylformamide (1 mL) at room temperature. The reaction mixture was stirred at room temperature for 18 hr and water was added. The mixture was extracted with ethyl acetate, and the organic layer was separated, washed with saturated brine, dried over magnesium sulfate, and concentrated. The residue was purified by silica gel column chromatography (NH silica gel, ethyl acetate/methanol) to give the title compound (19 mg).

$^1$H NMR (300 MHz, DMSO-$d_6$) δ 0.76 (3H, t, J = 7.2 Hz), 1.31-1.47 (4H, m), 1.52-1.74 (1H, m), 2.25-2.39 (1H, m), 2.66 (1H, dd, J = 16.4, 8.3 Hz), 2.74 (3H, s), 3.06-3.29 (3H, m), 3.40-3.54 (2H, m), 3.69-3.79 (2H, m), 3.80-3.95 (2H, m), 4.03 (1H, q, J = 7.1 Hz), 4.36-4.59 (3H, m), 7.13 (1H, dd, J = 7.5, 4.8 Hz), 7.50-7.71 (2H, m), 7.84-7.94 (2H, m), 8.07 (1H, d, J = 8.8 Hz), 8.18 (1H, dd, J = 4.9, 1.9 Hz), 8.31 (1H, dd, J = 7.5, 2.0 Hz), 8.86 (1H, d, J = 6.6 Hz).
MS: 686.4.

Example 51

3-[(6R,8aS)-6-ethyl-2-[4-fluoro-2-(trifluoromethyl)phenyl]-3-oxo-5,6,8,8a-tetrahydro-1H-imidazo[1,5-a]pyrazin-7-yl]-6-(2-ethoxypyridin-3-yl)-N-[(3R)-5-oxopyrrolidin-3-yl]pyridine-2-carboxamide

Example 51a

3-[(6R,8aS)-6-ethyl-2-[4-fluoro-2-(trifluoromethyl)phenyl]-3-oxo-5,6,8,8a-tetrahydro-1H-imidazo[1,5-a]pyrazin-7-yl]-6-(2-ethoxy-3-pyridyl)pyridine-2-carboxylic acid

A) tert-butyl (2R,5S)-2-ethyl-4-[[4-fluoro-2-(trifluoromethyl)phenyl]carbamoyl]-5-(hydroxymethyl)piperazine-1-carboxylate

To a mixture of dichloromethane (100 mL) and 4-fluoro-2-(trifluoromethyl)aniline (2.2 g, 12.29 mmol) was added triethylamine(4.17 mL, 30.72 mmol) under an argon atmosphere at 0°C, triphosgene (1.519 g, 5.12 mmol) was successively added, and the reaction mixture was stirred at 25°C for 20 hr. To the reaction mixture was added a solution of tert-butyl (2R,5S)-2-ethyl-5-(hydroxymethyl)piperazine-1-carboxylate (2.5 g, 10.24 mmol) and triethylamine (4.17 mL, 30.723 mmol) in dichloromethane (100 mL) at 0°C, and the reaction mixture was stirred at 25°C for 2 hr. After completion of the reaction, the reaction mixture was poured into ice-cold water (300 mL). The aqueous layer was extracted with dichloromethane (300 mL x 3), the organic layer was washed with saturated aqueous sodium hydrogen carbonate solution, water and saturated brine, dried over sodium sulfate, filtered, and the filtrate was concentrated under reduced pressure. The obtained residue was purified by silica gel column chromatography (ethyl acetate/hexane) to give the title compound (3 g).

> $^1$H NMR (300 MHz, DMSO-$d_6$) $\delta$0.75-0.97 (3H, m), 1.35-1.42 (9H, m), 1.40-1.53 (2H,m), 2.72-2.92 (2H, m), 3.41-3.59 (2H, m), 3.73-4.10 (4H, m), 5.46-5.70 (1H, m), 7.29-7.70 (3H, m), 8.43-8.70 (1H, m).
> MS: 450.2.

B) tert-butyl (6R,8aR)-6-ethyl-2-[4-fluoro-2-(trifluoromethyl)phenyl]-3-oxo-5,6,8,8a-tetrahydro-1H-imidazo[1,5-a]pyrazine-7-carboxylate

To a mixture of dichloromethane (30 mL) and tert-butyl (2R,5S)-2-ethyl-4-[[4-fluoro-2-(trifluoromethyl)phenyl] carbamoyl]-5-(hydroxymethyl)piperazine-1-carboxylate (2.3 g, 5.12 mmol) was added 1,8-diazabicyclo[5.4.0]-7-undecene (6.11 mL, 40.94 mmol) under an argon atmosphere at 25°C, and methanesulfonyl chloride (0.99 mL, 12.79 mmol) was further added at 0°C. The reaction mixture was stirred at 25°C for 16 hr. After completion of the reaction, to the reaction mixture was added ice-cold water. The aqueous layer was extracted with ethyl acetate. The combined organic layer was washed with water and saturated brine, dried over sodium sulfate, filtered, and the filtrate was concentrated under reduced pressure. The obtained residue was purified by combiflash silica gel column chromatography (ethyl acetate/hexane) to give the title compound (1.9 g).

> $^1$H NMR (400 MHz, DMSO-$d_6$) $\delta$0.80 (4H, q, J = 7.2 Hz), 1.10-1.35 (1H, m), 1.42 (9H, s), 1.46-1.74 (1H, m), 2.71-3.13 (2H, m), 3.47-4.15 (5H, m), 7.57-7.74 (3H, m).
> MS: 432.1.

C) (6R,8aS)-6-ethyl-2-[4-fluoro-2-(trifluoromethyl)phenyl]-1,5,6,7,8,8a-hexahydroimidazo[1,5-a]pyrazin-3-one

To a stirred mixture of dichloromethane (30 mL) and tert-butyl (6R,8aR)-6-ethyl-2-[4-fluoro-2-(trifluoromethyl) phenyl]-3-oxo-5,6,8,8a-tetrahydro-1H-imidazo[1,5-a]pyrazine-7-carboxylate (2.4 g, 5.563 mmol) was added trifluoroacetic acid (4.3 mL, 55.62 mmol) at 0°C, and the reaction mixture was stirred at 25°C for 4 hr. After completion of the reaction, the reaction mixture was cooled to 0°C and poured into sodium hydrogen carbonate aqueous solution under ice-cooling. The aqueous layer was extracted with dichloromethane, and the combined organic layer was washed with water and saturated brine, dried over sodium sulfate, and filtered. The filtrate was concentrated under reduced pressure to give the title compound (1.7 g).
The residue was used for the next step without further purification.

> $^1$H NMR (400 MHz, DMSO-$d_6$) $\delta$ 0.84 (3H, t, J = 7.3 Hz), 1.38-1.61 (2H, m), 2.58-2.71 (2H, m), 2.78 (1H, t, J = 10.3 Hz), 3.02 (1H, dd, J = 4.2, 13.0 Hz), 3.19 (1H, dd, J = 3.3, 8.1 Hz), 3.50 (1H, d, J = 12.9 Hz), 3.60-3.76 (2H, m), 7.53-7.73 (3H, m).
> MS: 331.9.

D) 3-[(6R,8aS)-6-ethyl-2-[4-fluoro-2-(trifluoromethyl)phenyl]-3-oxo-5,6,8,8a-tetrahydro-1H-imidazo[1,5-a]pyrazin-7-yl]-6-bromo-pyridine-2-carbaldehyde

To a mixture of (6R,8aS)-6-ethyl-2-[4-fluoro-2-(trifluoromethyl)phenyl]-1,5,6,7,8,8a-hexahydroimidazo[1,5-a] pyrazin-3-one (1.7g, 5.136 mmol) in toluene (50 mL) was added 6-bromo-3-fluoro-pyridine-2-carbaldehyde (1.56 g, 7.70 mmol), and then potassium carbonate (2.126 g, 15.4 mmol) was added at room temperature. The reaction mixture was heated under reflux for 3 days. The reaction mixture was cooled to 25°C, ice-cold water (200 mL) was added, and the aqueous layer was extracted with ethyl acetate (2x100 mL). The combined organic layer was washed with saturated brine,

dried over anhydrous sodium sulfate, filtered, and the filtrate was concentrated under reduced pressure. The obtained residue was purified by silica gel column chromatography (ethyl acetate/hexane) to give the title compound (2.4 g).

$^1$H NMR (400 MHz, DMSO-$d_6$) δ 0.72 (3H, t, J = 7.3 Hz), 1.46-1.66 (2H, m), 3.20 (1H, dd, J = 12.7, 4.3 Hz), 3.25-3.56 (4H, m), 3.70 (1H, d, J = 13.2 Hz), 3.80 (1H, t, J = 8.4 Hz), 3.85-3.96 (1H, m), 7.62-7.68 (3H, m), 7.68-7.75 (2H, m), 9.81 (1H, s).
MS: 515.3.

E) 3-[(6R,8aS)-6-ethyl-2-[4-fluoro-2-(trifluoromethyl)phenyl]-3-oxo-5,6,8,8a-tetrahydro-1H-imidazo[1,5-a]pyrazin-7-yl]-6-(2-ethoxy-3-pyridyl)pyridine-2-carbaldehyde

3-[(6R,8aS)-6-ethyl-2-[4-fluoro-2-(trifluoromethyl)phenyl]-3-oxo-5,6,8,8a-tetrahydro-1H-imidazo[1,5-a]pyrazin-7-yl]-6-bromo-pyridine-2-carbaldehyde (2.3 g, 4.46 mmol) was dissolved in a mixture of dioxane (27 mL) and water (3 mL), deaerated, and (2-ethoxypyridin-3-yl)boronic acid (1.118 g, 6.695 mmol) and potassium carbonate (1.848 g, 13.39 mmol) were added at 25°C. The reaction mixture was deaerated again for 10 min with an argon gas. To this mixture was added Pd(DtBPF)$Cl_2$ (145.0 mg, 0.223 mmol), and the reaction mixture was heated at 100°C for 2 hr. After completion of the reaction, the reaction mixture was cooled to 25°C, and water (100 mL) was added. The aqueous layer was extracted with ethyl acetate (2x100 mL). The combined organic layer was washed with saturated brine, dried over anhydrous sodium sulfate, filtered, and the filtrate was concentrated under reduced pressure. The obtained residue was purified by silica gel column chromatography (ethyl acetate/hexane) to give the title compound (2.3 g).

$^1$H NMR (400 MHz, DMSO-$d_6$) δ 0.76 (3H, t, J = 7.2 Hz), 1.38 (3H, t, J = 7.0 Hz), 1.45-1.73 (2H, m), 3.18-3.39 (2H, m), 3.37-3.63 (3H, m), 3.74 (1H, d, J = 13.0 Hz), 3.82 (1H, t, J = 8.4 Hz), 3.94 (1H, s), 4.46 (2H, q, J = 6.9 Hz), 7.15 (1H,dd, J = 4.8, 7.5 Hz), 7.64-7.68 (2H, m), 7.73 (2H, d, J = 9.0 Hz), 8.16-8.25 (2H, m), 8.33 (1H, dd, J = 2.0, 7.5 Hz), 10.01 (1H, s).
MS: 557.7.

F) 3-[(6R,8aS)-6-ethyl-2-[4-fluoro-2-(trifluoromethyl)phenyl]-3-oxo-5,6,8,8a-tetrahydro-1H-imidazo[1,5-a]pyrazin-7-yl]-6-(2-ethoxy-3-pyridyl)pyridine-2-carboxylic acid

To a mixture of 3-[(6R,8aS)-6-ethyl-2-[4-fluoro-2-(trifluoromethyl)phenyl]-3-oxo-5,6,8,8a-tetrahydro-1H-imidazo[1,5-a]pyrazin-7-yl]-6-(2-ethoxy-3-pyridyl)pyridine-2-carbaldehyde (2.0 g, 3.591 mmol) in tert-butanol (20 mL), tetrahydrofuran (20 mL), water (20 mL) and 2-methyl-2-butene (3.80 mL, 35.907 mmol) was added at 0°C $NaClO_2$ (812.0 mg, 8.977 mmol). An aqueous solution of $NaH_2PO_4$ (977 mg, 7.181 mmol) and water (20 mL) was added to this mixture at 0°C. The reaction mixture was stirred at 25°C for 2 hr. After completion of the reaction, to the reaction mixture were added saturated sodium thiosulfate aqueous solution and saturated ammonium chloride aqueous solution, and the aqueous layer was extracted with ethyl acetate. The combined organic layer was washed with 1N hydrochloric acid, water and saturated brine, dried over anhydrous sodium sulfate, filtered, and the filtrate was concentrated under reduced pressure. The residue was dissolved in dichloromethane (5 mL), a pentane-ether mixed solution (50 mL, 4:1) was added, and the mixture was stirred for 15 min. The precipitated solid was collected by filtration, washed twice with pentane to give the title compound (1.75 g).

$^1$H NMR (400 MHz, DMSO-$d_6$) δ 0.83 (3H, t, J = 7.4 Hz), 1.40 (3H, t, J = 7.1 Hz), 1.49-1.82 (2H, m), 3.23-3.34 (2H, m), 3.36-3.48 (2H, m), 3.52-3.63 (1H, m), 3.76 (1H, d, J = 13.1 Hz), 3.85 (1H, t, J = 8.8 Hz), 3.90-4.01 (1H, m), 4.45-4.54 (2H, m), 7.09 (1H, t, J = 6.2 Hz), 7.61 (4H, t, J = 8.9 Hz), 8.10 (1H, d, J = 8.8 Hz), 8.18 (1H, s), 8.28 (1H, d, J = 6.8 Hz), 12.66 (1H, brs).
MS: 574.38.

G) 3-[(6R,8aS)-6-ethyl-2-[4-fluoro-2-(trifluoromethyl)phenyl]-3-oxo-5,6,8,8a-tetrahydro-1H-imidazo[1,5-a]pyrazin-7-yl]-6-(2-ethoxypyridin-3-yl)-N-[(3R)-5-oxopyrrolidin-3-yl]pyridine-2-carboxamide

To a stirred solution of 3-[(6R,8aS)-6-ethyl-2-[4-fluoro-2-(trifluoromethyl)phenyl]-3-oxo-5,6,8,8a-tetrahydro-1H-imidazo[1,5-a]pyrazin-7-yl]-6-(2-ethoxy-3-pyridyl)pyridine-2-carboxylic acid (120 mg) in acetonitrile (5 mL) was added TCFH (180 mg, 0.628 mmol), then N-methylimidazole (0.085 mL, 1.046 mmol) was added under an argon atmosphere at 25°C, and the reaction mixture was stirred at 25°C for 10 min. To the mixture was added (4R)-4-aminopyrrolidin-2-one (32 mg, 0.314 mmol) at 25°C, and the reaction mixture was stirred at 25°C for 16 hr. After completion of the reaction, ice water was added to the reaction mixture. The mixture was extracted with ethyl acetate, and the combined organic layer was washed with saturated brine, dried over anhydrous sodium sulfate, and concentrated under reduced pressure. The obtained residue was purified by combiflash column chromatography (ethyl acetate/hexane), and then reversed-phase

Preparative HPLC to give the title compound (20 mg).

<Preparative HPLC conditions>

Preparative HPLC was performed using an automatic purification device manufactured by Waters. column name: YMC-Actus C18(250×20 mm, 5 μm), periphery temperature: room temperature, flow rate: 16 mL/min, mobile phase: A=20 mM ammonium bicarbonate aqueous solution, B=acetonitrile.

$^1$H NMR (400 MHz, DMSO-$d_6$) δ 0.78-0.86 (3H, m), 1.39 (3H, t, J = 6.8 Hz), 1.43 - 1.76 (2H, m), 2.31 (1H, dd, J = 5.5, 16.6 Hz), 2.52-2.64 (1H, m), 3.16-3.45 (5H, m), 3.49-3.60 (1H, m), 3.63 (1H, t, J = 9.2 Hz), 3.74 (1H, d, J = 13.2 Hz), 3.85 (1H, t, J = 8.0 Hz), 3.91-3.96 (1H, m), 4.49 (2H, d, J = 6.8 Hz), 4.55-4.71 (1H, m), 7.10 (1H, t, J = 6.4 Hz), 7.26-7.47 (1H, m), 7.55 (1H, d, J = 8.8 Hz), 7.60 (3H, d, J = 6.4 Hz), 8.05 (1H, d, J = 8.4 Hz), 8.12-8.22 (1H, m), 8.31 (1H, d, J = 6.8 Hz), 8.47-8.71 (1H, m).
MS: 656.4.

Example 52

3-[(6R,8aS)-6-ethyl-2-[4-fluoro-2-(trifluoromethyl)phenyl]-3-oxo-5,6,8,8a-tetrahydro-1H-imidazo[1,5-a]pyrazin-7-yl]-6-(2-ethoxypyridin-3-yl)-N-[(3R)-1-methyl-5-oxopyrrolidin-3-yl]pyridine-2-carboxamide

3-[(6R,8aS)-6-ethyl-2-[4-fluoro-2-(trifluoromethyl)phenyl]-3-oxo-5,6,8,8a-tetrahydro-1H-imidazo[1,5-a]pyrazin-7-yl]-6-(2-ethoxy-3-pyridyl)pyridine-2-carboxylic acid (1.26 g) was dissolved in N,N-dimethylformamide (20 mL), and HATU (1.00 g), N,N-diisopropylethylamine (0.752 mL) and (4R)-4-amino-1-methyl-pyrrolidin-2-one (314 mg) were added to the mixture. The mixture was stirred at room temperature for 18 hr, diluted with saturated aqueous sodium hydrogen carbonate solution, and the aqueous layer was extracted 3 times with ethyl acetate. The extract was washed with water and saturated brine, dried over magnesium sulfate, and concentrated. The residue was purified by silica gel column chromatography (ethyl acetate/methanol) to give a solid. The solid was recrystallized from diisopropyl ether/ethyl acetate/heptane. The mixture was cooled to room temperature over the weekend. The mixture was stirred at 0°C for 1 hr. The crystal was collected by filtration to give the title compound (1.28 g).

$^1$H NMR (400 MHz, DMSO-$d_6$) δ 0.77 (3H, t, J = 7.3 Hz), 1.32-1.44 (4H, m), 1.52-1.73 (1H, m), 2.37 (1H, dd, J = 16.7, 5.1 Hz), 2.62-2.75 (4H, m), 3.05-3.30, (4H,m), 3.34-3.39 (1H, m), 3.41-3.52 (1H, m), 3.65-4.03 (4H, m), 4.35-4.62 (3H, m), 7.13 (1H, dd, J = 7.5, 4.9 Hz), 7.51-7.80 (4H, m), 8.07 (1H, d, J = 8.7Hz), 8.18 (1H, dd, J = 4.9, 1.9 Hz), 8.31 (1H, dd, J = 7.5, 2.0 Hz), 8.86 (1H, d, J = 6.6 Hz).
MS: 670.2.

Example 53

3-[(6R,8aS)-2-(4-chloro-2-cyanophenyl)-6-ethyl-3-oxo-5,6,8,8a-tetrahydro-1H-imidazo[1,5-a]pyrazin-7-yl]-6-(2-ethoxypyridin-3-yl)-N-[(3R)-5-oxopyrrolidin-3-yl]pyridine-2-carboxamide

Example 53a

3-[(6R,8aS)-2-(4-chloro-2-cyano-phenyl)-6-ethyl-3-oxo-5,6,8,8a-tetrahydro-1H-imidazo[1,5-a]pyrazin-7-yl]-6-(2-ethoxy-3-pyridyl)pyridine-2-carboxylic acid

A) tert-butyl (2R,5S)-4-[(4-chloro-2-cyano-phenyl)carbamoyl]-2-ethyl-5-(hydroxymethyl)piperazine-1-carboxylate

To a mixture of dichloromethane (50 mL) and 2-amino-5-chloro-benzonitrile (2.3 g, 15.35 mmol) was added triethylamine(4.3 mL, 30.70 mmol) under an argon atmosphere at 0°C, and then triphosgene (1.5 g, 5.12 mmol) was added, and the reaction mixture was stirred at 25°C for 20 hr. To the reaction mixture was added a solution of tert-butyl (2R,5S)-2-ethyl-5-(hydroxymethyl)piperazine-1-carboxylate (2.5 g, 10.23 mmol) and triethylamine (4.3 mL, 30.7 mmol) in dichloromethane (50 mL) at 0°C, and the reaction mixture was stirred at 25°C for 4 hr. After completion of the reaction, it was poured into ice-cold water. The aqueous layer was extracted with ethyl acetate, and the organic layer was washed with water and saturated brine, dried over sodium sulfate, filtered, and the filtrate was concentrated under reduced pressure. The obtained residue was purified by silica gel column chromatography (ethyl acetate/hexane) to give the title compound (2.65 g).

$^1$H NMR (400 MHz, DMSO-$d_6$) δ 0.86 (3H, t, J = 7.4 Hz), 1.37 (9H, s), 1.42-1.58 (2H, m), 2.77-2.98 (2H, m), 3.42-3.63 (2H, m), 3.76-4.22 (4H, m), 5.39-5.72 (1H, m), 7.52-7.75 (2H, m), 7.89 (1H, d, J = 2.4 Hz), 9.16 (1H, d, J = 6.3 Hz).
MS: 423.1.

B) tert-butyl (6R,8aR)-2-(4-chloro-2-cyano-phenyl)-6-ethyl-3-oxo-5,6,8,8a-tetrahydro-1H-imidazo[1,5-a]pyrazine-7-carboxylate

To a mixture of dichloromethane (40 mL) and tert-butyl (2R,5S)-4-[(4-chloro-2-cyano-phenyl)carbamoyl]-2-ethyl-5-(hydroxymethyl)piperazine-1-carboxylate (2.65 g, 6.28 mmol) was added 1,8-diazabicyclo[5.4.0]-7-undecene (7.5 mL, 50.24 mmol) under an argon atmosphere at 25°C, and methanesulfonyl chloride (1.2 mL, 15.70 mmol) was further added at 0°C. The reaction mixture was stirred at 25°C for 16 hr. After completion of the reaction, ice-cold water was added to the reaction mixture, and the aqueous layer was extracted with ethyl acetate. The combined organic layer was washed with water and saturated brine, dried over sodium sulfate, filtered, and the filtrate was concentrated under reduced pressure. The obtained residue was purified by combiflash silica gel column chromatography (ethyl acetate/hexane) to give the title compound (2.5 g).

$^1$H NMR (400 MHz, DMSO-$d_6$) δ 0.74-0.86 (3H, m), 1.42 (9H, s), 1.46-1.75 (2H, m), 2.75-2.89 (1H, m), 2.98-3.14 (1H, m), 3.57-3.65 (2H, m), 3.69-3.81 (1H, m), 3.85-4.25 (3H, m), 7.58 (1H, d, J = 8.8 Hz), 7.80 (1H, dd, J = 2.6, 8.8 Hz), 8.02 (1H, d, J = 2.5 Hz).
MS: 405.1.

C) 2-[(6R,8aS)-6-ethyl-3-oxo-1,5,6,7,8,8a-hexahydroimidazo[1,5-a]pyrazin-2-yl]-5-chloro-benzonitrile

To a stirred mixture of acetonitrile (25 mL) and tert-butyl (6R,8aR)-2-(4-chloro-2-cyano-phenyl)-6-ethyl-3-oxo-5,6,8,8a-tetrahydro-1H-imidazo[1,5-a]pyrazine-7-carboxylate (2.5 g, 6.17 mmol) was added trifluoroacetic acid (2.4 mL, 30.87 mmol) at 0°C, and the reaction mixture was stirred at 25°C for 4 hr. After completion of the reaction, the reaction mixture was cooled to 0°C, and poured into sodium hydrogen carbonate aqueous solution under ice-cooling. The aqueous layer was extracted with ethyl acetate. The combined organic layer was washed with water and saturated brine, dried over sodium sulfate, filtered, and the filtrate was concentrated under reduced pressure to give the title compound (1.5 g). The residue was used for the next step without further purification. MS: 304.9.

D) 2-[(6R,8aS)-7-(6-bromo-2-formyl-3-pyridyl)-6-ethyl-3-oxo-5,6,8,8a-tetrahydro-1H-imidazo[1,5-a]pyrazin-2-yl]-5-chloro-benzonitrile

To a mixture of 2-[(6R,8aS)-6-ethyl-3-oxo-1,5,6,7,8,8a-hexahydroimidazo[1,5-a]pyrazin-2-yl]-5-chloro-benzonitrile (1.5 g, 4.92 mmol) in toluene (20 mL) was added 6-bromo-3-fluoro-pyridine-2-carbaldehyde (1.5 g, 7.38 mmol), and then potassium carbonate (2.1 g, 14.8 mmol) was added at room temperature. The reaction mixture was heated under reflux for 3 days. The reaction mixture was cooled to 25°C, ice-cold water (100 mL) was added, and the aqueous layer was extracted with ethyl acetate (2x100 mL). The combined organic layer was washed with saturated brine, dried over anhydrous sodium sulfate, filtered, and the filtrate was concentrated under reduced pressure. The obtained residue was purified by silica gel column chromatography (ethyl acetate/hexane) to give the title compound (1.3 g).

$^1$H NMR (400 MHz, DMSO-$d_6$) δ 0.73 (3H, t, J = 7.3 Hz), 1.43-1.83 (2H, m), 3.19-3.32 (1H, m), 3.35-3.58 (3H, m), 3.57-3.66 (1H, m), 3.74 (1H, d, J = 13.4 Hz), 3.86-4.00 (1H, m), 4.10 (1H, t, J = 8.7 Hz), 7.58-7.69 (2H, m), 7.72 (1H, d, J = 8.8 Hz), 7.81 (1H, dd, J = 2.6, 8.8 Hz), 8.03 (1H, d, J = 2.4 Hz), 9.81 (1H, s).
MS: 490.2.

E) 2-[(6R,8aS)-7-[6-(2-ethoxy-3-pyridyl)-2-formyl-3-pyridyl]-6-ethyl-3-oxo-5,6,8,8a-tetrahydro-1H-imidazo[1,5-a]pyrazin-2-yl]-5-chloro-benzonitrile

A mixture of 2-[(6R,8aS)-7-(6-bromo-2-formyl-3-pyridyl)-6-ethyl-3-oxo-5,6,8,8a-tetrahydro-1H-imidazo[1,5-a]pyrazin-2-yl]-5-chloro-benzonitrile (1 g, 2.05 mmol), dioxane (90 mL) and water (10 mL) was subjected to a deaeration operation, (2-ethoxypyridin-3-yl)boronic acid (380 mg, 2.25 mmol) and potassium carbonate (850 mg, 6.14 mmol) were added at 25°C, and the reaction mixture was deaerated again for 10 min with an argon gas. To the mixture was added Pd(DtBPF)$Cl_2$ (70 mg, 0.102 mmol), and the reaction mixture was heated at 60°C for 0.5 hr. After completion of the reaction, the reaction mixture was cooled to 25°C, and water (100 mL) was added. The aqueous layer was extracted with ethyl acetate (2x100 mL). The combined organic layer was washed with saturated brine, dried over anhydrous sodium sulfate, filtered, and the filtrate was concentrated under reduced pressure. The obtained residue was purified by silica gel

column chromatography (ethyl acetate/hexane) to give the title compound (800 mg).

$^1$H NMR (400 MHz, DMSO) δ 0.77 (3H, t, J = 7.4 Hz), 1.38 (3H, t, J = 7.0 Hz), 1.50-1.61 (1H, m), 1.64-1.76 (1H, m), 3.39-3.54 (2H, m), 3.59-3.68 (2H, m), 3.77 (1H, d, J = 13.0 Hz), 3.90-4.08 (2H, m), 4.12 (1H, t, J = 8.7 Hz), 4.47 (2H, q, J= 7.0 Hz), 7.15 (1H, dd, J = 7.5, 4.9 Hz), 7.63 (1H, d, J = 8.8 Hz), 7.72 (1H, d, J = 8.9 Hz), 7.82 (1H, dd, J = 8.8, 2.6 Hz), 8.04 (1H, d, J = 2.4 Hz), 8.16-8.25 (2H, m), 8.33 (1H, dd, J = 7.5, 2.0 Hz), 10.02 (1H, s).
MS: 530.1.

F) 3-[(6R,8aS)-2-(4-chloro-2-cyano-phenyl)-6-ethyl-3-oxo-5,6,8,8a-tetrahydro-1H-imidazo[1,5-a]pyrazin-7-yl]-6-(2-ethoxy-3-pyridyl)pyridine-2-carboxylic acid

To a mixture of 2-[(6R,8aS)-7-[6-(2-ethoxy-3-pyridyl)-2-formyl-3-pyridyl]-6-ethyl-3-oxo-5,6,8,8a-tetrahydro-1H-imidazo[1,5-a]pyrazin-2-yl]-5-chloro-benzonitrile (800 mg, 1.51 mmol) in tert-butanol (5 mL), tetrahydrofuran (5 mL), water (5 mL) and 2-methyl-2-butene (1.6 mL, 15.1 mmol) was added $NaClO_2$ (341 mg, 3.766 mmol) at 0°C. To the mixture was added an aqueous solution of $NaH_2PO_4$ (410 mg, 3.03 mmol) and water (2 mL). The reaction mixture was stirred at 25°C for 2 hr. After completion of the reaction, to the reaction mixture were added saturated sodium thiosulfate aqueous solution and saturated ammonium chloride aqueous solution, and the aqueous layer was extracted with ethyl acetate. The combined organic layer was washed with 1N hydrochloric acid, water and saturated brine, dried over anhydrous sodium sulfate, filtered, and the filtrate was concentrated under reduced pressure. The residue was dissolved in dichloromethane (2 mL), pentane-ether mixed solution (20 mL, 4:1) was added, and the mixture was stirred for 15 min. The precipitated solid was collected by filtration to give the title compound (640 mg).

$^1$H NMR (400 MHz, DMSO) δ 0.84 (3H, t, J = 7.4 Hz), 1.39 (3H, t, J = 7.0 Hz), 1.53-1.65 (1H, m), 1.66-1.82 (1H, m), 3.27-3.37 (2H, m), 3.38-3.54 (1H, m), 3.54-3.70 (2H, m), 3.80 (1H, d, J = 12.8 Hz), 3.92-4.04 (1H, m), 4.11 (1H, t, J = 8.8 Hz), 4.50 (2H, q, J = 6.8 Hz), 7.09 (1H, dd, J = 4.8, 7.5 Hz), 7.62 (2H, dd, J = 5.0, 8.8 Hz), 7.76 (1H, dd, J = 2.5, 8.8 Hz), 7.92 (1H, d, J = 2.4 Hz), 8.09 (1H, d, J = 8.8 Hz), 8.18 (1H, dd, J = 1.9, 4.9 Hz), 8.28 (1H, dd, J = 2.0, 7.5 Hz), 12.74 (1H, brs).
MS: 547.28.

G) 3-[(6R,8aS)-2-(4-chloro-2-cyanophenyl)-6-ethyl-3-oxo-5,6,8,8a-tetrahydro-1H-imidazo[1,5-a]pyrazin-7-yl]-6-(2-ethoxypyridin-3-yl)-N-[(3R)-5-oxopyrrolidin-3-yl]pyridine-2-carboxamide

To a stirred solution of 3-[(6R,8aS)-2-(4-chloro-2-cyanophenyl)-6-ethyl-3-oxo-5,6,8,8a-tetrahydro-1H-imidazo [1,5-a]pyrazin-7-yl]-6-(2-ethoxy-3-pyridyl)pyridine-2-carboxylic acid (100 mg, 0.628mmol) in acetonitrile (5 mL) were added TCFH (180 mg, 0.628 mmol) and N-methylimidazole (0.085 mL, 1.046 mmol) under an argon gas atmosphere at 25°C. The reaction mixture was stirred at the same temperature for 10 min. To the reaction mixture was added (4R)-4-aminopyrrolidin-2-one (30 mg, 0.274 mmol) at 25°C, and the mixture was stirred at 25°C for 16 hr. After completion of the reaction, the reaction mixture was added into ice water, and the aqueous layer was extracted with ethyl acetate. The organic layer was washed with saturated brine, dried over anhydrous sodium sulfate, and concentrated under reduced pressure. The obtained residue was purified by combiflash column chromatography (ethyl acetate/hexane), and then purified by reversed-phase Preparative HPLC to give the title compound (35 mg).

<Preparative HPLC conditions>

Preparative HPLC was performed using an automatic purification device manufactured by Waters. column name: YMC-Actus C18(250×20 mm, 5 μm), periphery temperature: room temperature, flow rate: 16 mL/min, mobile phase: A=20 mM ammonium bicarbonate aqueous solution, B=acetonitrile.

$^1$H NMR (400 MHz, DMSO-$d_6$) δ 0.83 (3H, t, J = 7.3 Hz), 1.39 (3H, t, J = 7.0 Hz), 1.46-1.60 (1H, m), 1.64-1.81 (1H, m), 2.30-2.37 (1H, m), 2.54-2.62 (1H, m), 3.20-3.34 (3H, m), 3.40 (1H, t, J = 11.2 Hz), 3.55-3.69 (3H, m), 3.78 (1H, d, J = 12.8 Hz), 3.91-4.03 (1H, m), 4.12 (1H, t, J = 8.4 Hz), 4.50 (2H, q, J = 7.2 Hz), 4.56-4.69 (1H, m), 7.10 (1H, t, J = 5.6 Hz), 7.23-7.43 (1H, m), 7.54 (1H, d, J =8.8 Hz), 7.63 (1H, d, J = 8.8 Hz), 7.77 (1H, d, J = 8.8 Hz), 7.92 (1H, s), 8.05 (1H, d, J = 8.8 Hz), 8.14-8.20 (1H, m), 8.31 (1H, d, J = 7.5 Hz), 8.53-8.59 (1H, m).
MS: 629.39.

Example 54

3-[(6R,8aS)-2-(4-chloro-2-cyanophenyl)-6-ethyl-3-oxo-5,6,8,8a-tetrahydro-1H-imidazo[1,5-a]pyrazin-7-yl]-6-(2-ethoxypyridin-3-yl)-N-[(3R)-1-methyl-5-oxopyrrolidin-3-yl]pyridine-2-carboxamide

3-[(6R,8aS)-2-(4-chloro-2-cyano-phenyl)-6-ethyl-3-oxo-5,6,8,8a-tetrahydro-1H-imidazo[1,5-a]pyrazin-7-yl]-6-(2-ethoxy-3-pyridyl)pyridine-2-carboxylic acid (202 mg, 0.369 mmol) was dissolved in N,N-dimethylformamide (3 mL), and HATU (168 mg, 0.443 mmol), N,N-diisopropyl ethylamine (0.126 mL, 0.739 mmol) and (4R)-4-amino-1-methyl-pyrrolidin-2-one (52.8 mg, 0.462 mmol) were added to the mixture. The mixture was stirred at room temperature for 18 hr, diluted with saturated aqueous sodium hydrogen carbonate solution, and extracted 3 times with ethyl acetate. The extract was washed with water and saturated brine, dried over magnesium sulfate, and concentrated. The residue was purified by silica gel column chromatography (ethyl acetate/methanol) to give a solid. The solid was recrystallized from diisopropyl ether/ethyl acetate at 75°C. The mixture, was cooled to room temperature over the weekend. The mixture was stirred at 0°C for 1 hr. The crystal was collected by filtration to give the title compound (190 mg).

$^1H$ NMR (300 MHz, DMSO-$d_6$) δ 0.78 (3H, t, J = 7.2 Hz), 1.30-1.49 (4H, m), 1.57-1.80 (1H, m), 2.37 (1H, dd, J = 16.5, 5.3 Hz), 2.60-2.79 (4H, m), 3.14-3.29 (3H, m), 3.35-3.39 (1H, m), 3.42-3.52 (1H, m), 3.61-3.78 (3H, m), 3.84-4.02 (1H, m), 4.08-4.23 (1H, m), 4.35-4.67 (3H, m), 7.13 (1H, dd, J = 7.5, 4.9 Hz), 7.54 (1H, d, J = 8.9 Hz), 7.63 (1H, d, J = 8.9 Hz), 7.82 (1H, dd, J = 8.8, 2.5 Hz), 8.00-8.10 (2H, m), 8.18 (1H, dd, J = 4.9, 1.9 Hz), 8.31 (1H, dd, 7.5, 2.0 Hz), 8.87 (1H, d, J = 6.6 Hz).
MS: 643.1.

Example 55

3-[(6R,8aS)-2-(2-cyano-4-fluorophenyl)-6-ethyl-3-oxo-5,6,8,8a-tetrahydro-1H-imidazo[1,5-a]pyrazin-7-yl]-6-(2-ethoxypyridin-3-yl)-N-[(3R)-5-oxopyrrolidin-3-yl]pyridine-2-carboxamide

The compound was synthesized according to the method shown in Example 10 and Example 38 or a method analogous thereto.
MS: 613.39.

Example 56

3-[(6R,8aS)-2-[2-cyano-4-(trifluoromethyl)phenyl]-6-ethyl-3-oxo-5,6,8,8a-tetrahydro-1H-imidazo[1,5-a]pyrazin-7-yl]-6-(2-ethoxypyridin-3-yl)-N-[(3R)-5-oxopyrrolidin-3-yl]pyridine-2-carboxamide

The compound was synthesized according to the method shown in Example 10 and Example 38 or a method analogous thereto.
MS: 663.25.

Example 57

3-[(6R,8aS)-2-[2-cyano-4-(trifluoromethyl)phenyl]-6-ethyl-3-oxo-5,6,8,8a-tetrahydro-1H-imidazo[1,5-a]pyrazin-7-yl]-6-(2-ethoxypyridin-3-yl)-N-[(3R)-1-methyl-5-oxopyrrolidin-3-yl]pyridine-2-carboxamide

The compound was synthesized according to the method shown in Example 10 and Example 38 or a method analogous thereto.
MS: 677.41.

Example 58

3-[(6R,8aS)-2-(2-cyano-4-fluorophenyl)-6-ethyl-3-oxo-5,6,8,8a-tetrahydro-1H-imidazo[1,5-a]pyrazin-7-yl]-6-(2-ethoxypyridin-3-yl)-N-[(3R)-1-methyl-5-oxopyrrolidin-3-yl]pyridine-2-carboxamide

The compound was synthesized according to the method shown in Example 10 and Example 38 or a method analogous thereto.
MS: 627.42.
Example compounds described above are shown in the following Tables 1-1 to Table 1-13. MS in the Tables indicates actual values.

[Table 1-1]

| Example No. | Structural formula | Compound name | MS |
|---|---|---|---|
| Example 1 | 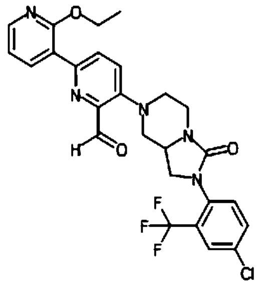 | 3-[2-[4-chloro-2-(trifluoromethyl)phenyl]-3-oxo-5, 6, 8, 8a-tetrahydro-1H-imidazo[1,5-a]pyrazin-7-yl]-6-(2-ethoxypyridin-3-yl)pyridine-2-carbaldehyde | 546.0 |
| Example 2 | 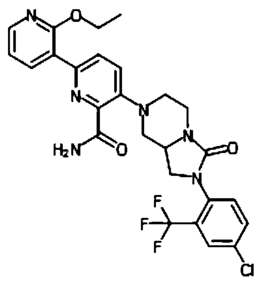 | 3-[2-[4-chloro-2-(trifluoromethyl)phenyl]-3-oxo-5, 6, 8, 8a-tetrahydro-1H-imidazo[1,5-a]pyrazin-7-yl]-6-(2-ethoxypyridin-3-yl)pyridine-2-carboxamide | 561.3 |
| Example 3 | 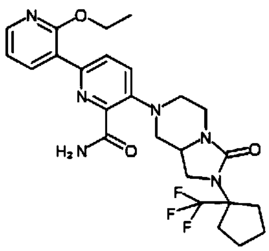 | 6-(2-ethoxypyridin-3-yl)-3-[3-oxo-2-[1-(trifluoromethyl)cyclopentyl]-5, 6, 8, 8a-tetrahydro-1H-imidazo[1,5-a]pyrazin-7-yl] pyridine-2-carboxamide | 519.0 |
| Example 4 | 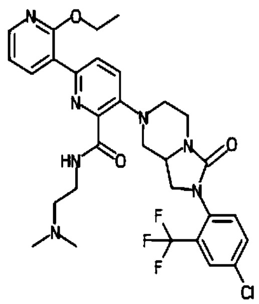 | 3-[2-[4-chloro-2-(trifluoromethyl)phenyl]-3-oxo-5, 6, 8, 8a-tetrahydro-1H-imidazo[1,5-a]pyrazin-7-yl]-N-[2-(dimethylamino) ethyl]-6-(2-ethoxypyridin-3-yl)pyridine-2-carboxamide | 632.3 |
| Example 5 | 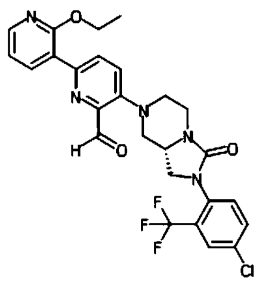 | 3-[(8aS)-2-[4-chloro-2-(trifluoromethyl)phenyl]-3-oxo-5,6,8,8a-tetrahydro-1H-imidazo[1,5-a]pyrazin-7-yl]-6-(2-ethoxypyridin-3-yl)pyridine-2-carbaldehyde | 546.4 |

[Table 1-2]

| | | | |
|---|---|---|---|
| Example 6 | 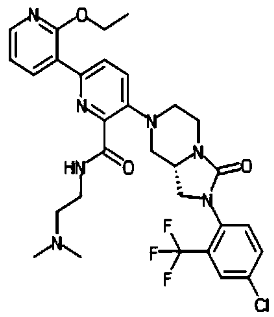 | 3-[(8aS)-2-[4-chloro-2-(trifluoromethyl)phenyl]-3-oxo-5, 6, 8, 8a-tetrahydro-1H-imidazo[1,5-a]pyrazin-7-yl]-N-[2-(dimethylamino)ethyl]-6-(2-ethoxypyridin-3-yl)pyridine-2-carboxamide | 632.5 |
| Example 7 | 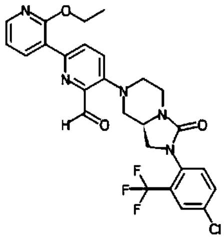 | 3-[(8aR)-2-[4-chloro-2-(trifluoromethyl)phenyl]-3-oxo-5, 6, 8, 8a-tetrahydro-1H-imidazo[1,5-a]pyrazin-7-yl]-6-(2-ethoxypyridin-3-yl)pyridine-2-carbaldehyde | 546.3 |
| Example 8 | 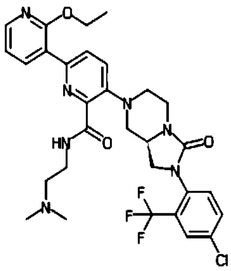 | 3-[(8aR)-2-[4-chloro-2-(trifluoromethyl)phenyl]-3-oxo-5, 6, 8, 8a-tetrahydro-1H-imidazo[1,5-a]pyrazin-7-yl]-N-[2-(dimethylamino)ethyl]-6-(2-ethoxypyridin-3-yl)pyridine-2-carboxamide | 632.5 |
| Example 9 | 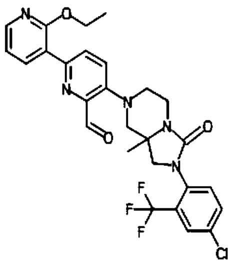 | 3-[2-[4-chloro-2-(trifluoromethyl)phenyl]-8a-methyl-3-oxo-1, 5,6, 8-tetrahydroimidazo[1,5-a]pyrazin-7-yl]-6-(2-ethoxypyridin-3-yl)pyridine-2-carbaldehyde | 560.3 |
| Example 10 | 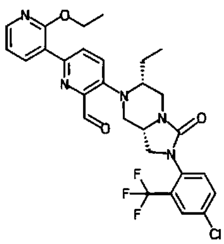 | 3-[(6R, 8aS)-2-[4-chloro-2-(trifluoromethyl)phenyl]-6-ethyl-3-oxo-5,6,8,8a-tetrahydro-1H-imidazo[1,5-a]pyrazin-7-yl]-6-(2-ethoxypyridin-3-yl)pyridine-2-carbaldehyde | 574.3 |

[Table 1-3]

| | | | |
|---|---|---|---|
| Example 11 | 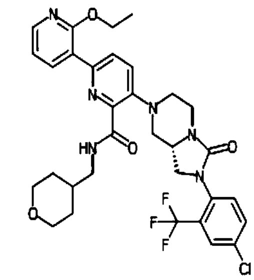 | 3-[(8aS)-2-[4-chloro-2-(trifluoromethyl)phenyl]-3-oxo-5, 6, 8, 8a-tetrahydro-1H-imidazo[1,5-a]pyrazin-7-yl]-6-(2-ethoxypyridin-3-yl)-N-(oxan-4-ylmethyl)pyridine-2-carboxamide | 659.3 |
| Example 12 | 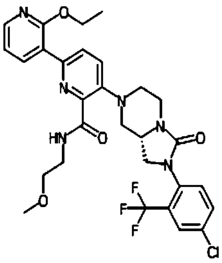 | 3-[(8aS)-2-[4-chloro-2-(trifluoromethyl)phenyl]-3-oxo-5, 6, 8, 8a-tetrahydro-1H-imidazo[1,5-a]pyrazin-7-yl]-6-(2-ethoxypyridin-3-yl)-N-(2-methoxyethyl)pyridine-2-carboxamide | 619.3 |
| Example 13 | 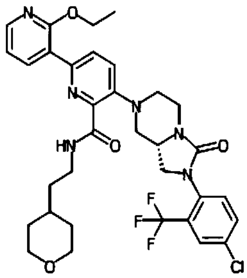 | 3-[(8aS)-2-[4-chloro-2-(trifluoromethyl)phenyl]-3-oxo-5, 6, 8, 8a-tetrahydro-1H-imidazo[1,5-a]pyrazin-7-yl]-6-(2-ethoxypyridin-3-yl)-N-[2-(oxan-4-yl)ethyl]pyridine-2-carboxamide | 673.3 |
| Example 14 | 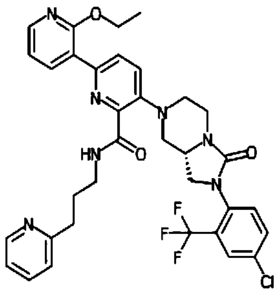 | 3-[(8aS)-2-[4-chloro-2-(trifluoromethyl)phenyl]-3-oxo-5, 6, 8, 8a-tetrahydro-1H-imidazo[1,5-a]pyrazin-7-yl]-6-(2-ethoxypyridin-3-yl)-N-(3-pyridin-2-ylpropyl)pyridine-2-carboxamide | 680.3 |
| Example 15 | 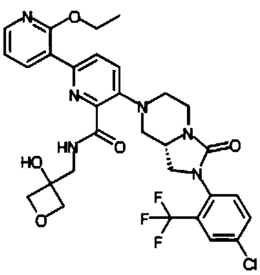 | 3-[(8aS)-2-[4-chloro-2-(trifluoromethyl)phenyl]-3-oxo-5, 6, 8, 8a-tetrahydro-1H-imidazo[1,5-a]pyrazin-7-yl]-6-(2-ethoxypyridin-3-yl)-N-[(3-hydroxyoxetan-3-yl)methyl]pyridine-2-carboxamide | 647.2 |

[Table 1-4]

| | | | |
|---|---|---|---|
| Example 16 | 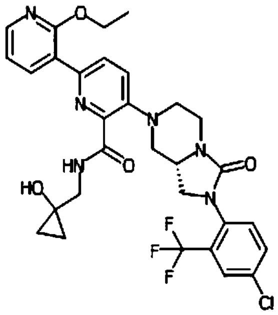 | 3-[(8aS)-2-[4-chloro-2-(trifluoromethyl)phenyl]-3-oxo-5, 6, 8, 8a-tetrahydro-1H-imidazo[1, 5-a]pyrazin-7-yl]-6-(2-ethoxypyridin-3-yl)-N-[(1-hydroxycyclopropyl)methyl]pyridine-2-carboxamide | 631.3 |
| Example 17 | 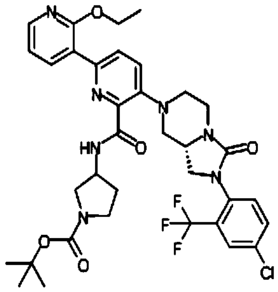 | tert-butyl 3-[[3-[(8aS)-2-[4-chloro-2-(trifluoromethyl)phenyl]-3-oxo-5, 6, 8, 8a-tetrahydro-1H-imidazo[1,5-a]pyrazin-7-yl]-6-(2-ethoxypyridin-3-yl)pyridine-2-carbonyl]amino]pyrrolidine-1-carboxylate | 730.4 |
| Example 18 | 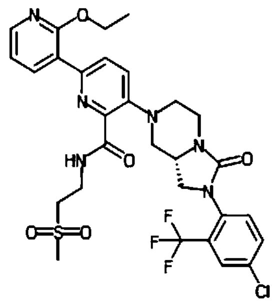 | 3-[(8aS)-2-[4-chloro-2-(trifluoromethyl)phenyl]-3-oxo-5, 6, 8, 8a-tetrahydro-1H-imidazo[1, 5-a]pyrazin-7-yl]-6-(2-ethoxypyridin-3-yl)-N-(2-methylsulfonylethyl)pyridine-2-carboxamide | 667.2 |
| Example 19 | 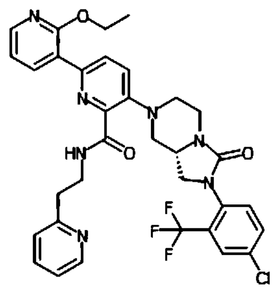 | 3-[(8aS)-2-[4-chloro-2-(trifluoromethyl)phenyl]-3-oxo-5,6,8,8a-tetrahydro-1H-imidazo[1,5-a]pyrazin-7-yl]-6-(2-ethoxypyridin-3-yl)-N-(2-pyridin-2-ylethyl)pyridine-2-carboxamide | 666.3 |
| Example 20 | 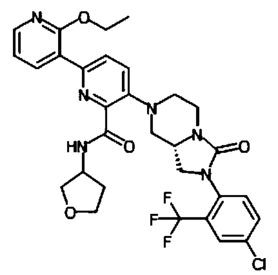 | 3-[(8aS)-2-[4-chloro-2-(trifluoromethyl)phenyl]-3-oxo-5, 6, 8, 8a-tetrahydro-1H-imidazo[1, 5-a]pyrazin-7-yl]-6-(2-ethoxypyridin-3-yl)-N-(oxolan-3-yl)pyridine-2-carboxamide | 631.3 |

[Table 1-5]

| | | | |
|---|---|---|---|
| Example 21 | 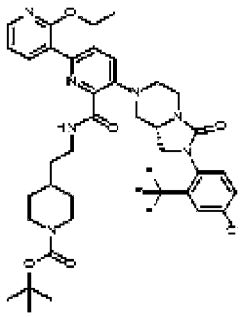 | tert-butyl 4-[2-[[3-[(8aS)-2-[4-chloro-2-(trifluoromethyl)phenyl]-3-oxo-5, 6, 8, 8a-tetrahydro-1H-imidazo[1,5-a]pyrazin-7-yl]-6-(2-ethoxypyridin-3-yl)pyridine-2-carbonyl]amino] ethyl]piperidine-1-carboxylate | 772.4 |
| Example 22 | 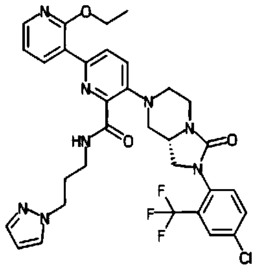 | 3-[(8aS)-2-[4-chloro-2-(trifluoromethyl)phenyl]-3-oxo-5, 6, 8, 8a-tetrahydro-1H-imidazo[1,5-a]pyrazin-7-yl]-6-(2-ethoxypyridin-3-yl)-N-(3-pyrazol-1-ylpropyl)pyridine-2-carboxamide | 669.3 |
| Example 23 | 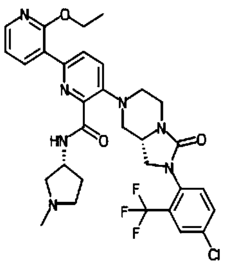 | 3-[(8aS)-2-[4-chloro-2-(trifluoromethyl)phenyl]-3-oxo-5, 6, 8, 8a-tetrahydro-1H-imidazo[1,5-a]pyrazin-7-yl]-6-(2-ethoxypyridin-3-yl)-N-[(3R)-1-methylpyrrolidin-3-yl]pyridine-2-carboxamide | 644.3 |
| Example 24 | 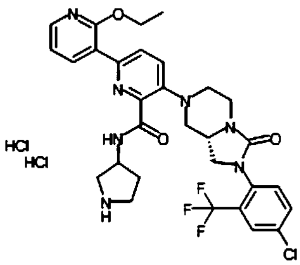 | 3-[(8aS)-2-[4-chloro-2-(trifluoromethyl)phenyl]-3-oxo-5, 6, 8, 8a-tetrahydro-1H-imidazo[1,5-a]pyrazin-7-yl]-6-(2-ethoxypyridin-3-yl)-N-pyrrolidin-3-ylpyridine-2-carboxamide dihydrochloride salt | 630.3 |
| Example 25 | 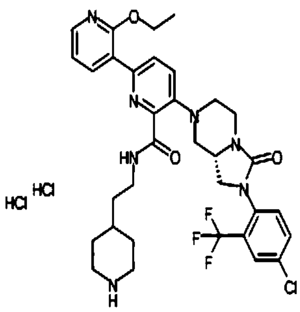 | 3-[(8aS)-2-[4-chloro-2-(trifluoromethyl)phenyl]-3-oxo-5, 6, 8, 8a-tetrahydro-1H-imidazo[1,5-a]pyrazin-7-yl]-6-(2-ethoxypyridin-3-yl)-N-(2-piperidin-4-ylethyl)pyridine-2-carboxamide dihydrochloride salt | 672.3 |

[Table 1-6]

| | | | |
|---|---|---|---|
| Example 26 | 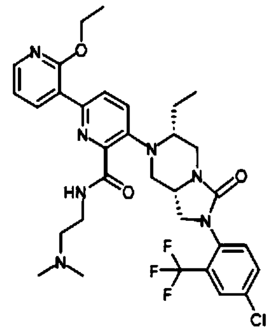 | 3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-N-[2-(dimethylamino)ethyl]-6-(2-ethoxypyridin-3-yl)pyridine-2-carboxamide | NMR alone |
| Example 27 | 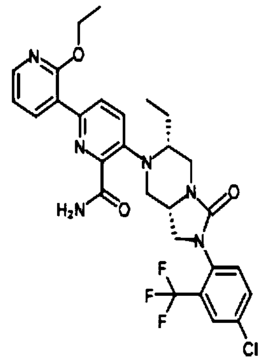 | 3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-(2-ethoxypyridin-3-yl)pyridine-2-carboxamide | 589.2 |
| Example 28 | 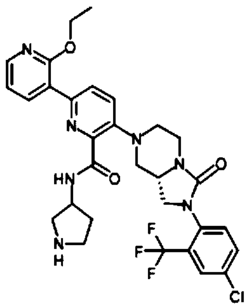 | 3-[(8aS)-2-[4-chloro-2-(trifluoromethyl)phenyl]-3-oxo-5, 6, 8, 8a-tetrahydro-1H-imidazo[1,5-a]pyrazin-7-yl]-6-(2-ethoxypyridin-3-yl)-N-pyrrolidin-3-ylpyridine-2-carboxamide | 630.4 |
| Example 29 | 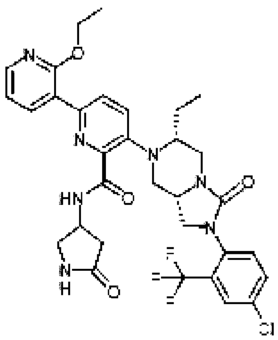 | 3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-(2-ethoxypyridin-3-yl)-N-(5-oxopyrrolidin-3-yl)pyridine-2-carboxamide | 672.3 |

[Table 1-7]

| | | | |
|---|---|---|---|
| Example 30 | 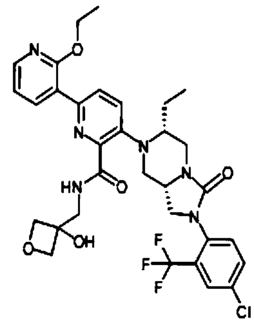 | 3-[(6R, 8aS)-2-[4-chloro-2-(trifluoromethyl)phenyl]-6-ethyl-3-oxo-5,6,8,8a-tetrahydro-1H-imidazo[1,5-a]pyrazin-7-yl]-6-(2-ethoxypyridin-3-yl)-N-[(3-hydroxyoxetan-3-yl)methyl]pyridine-2-carboxamide | 675.3 |
| Example 31 | 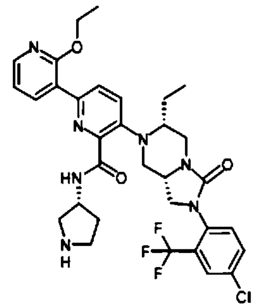 | 3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-(2-ethoxypyridin-3-yl)-N-[(3R)-pyrrolidin-3-yl]pyridine-2-carboxamide | 658.3 |
| Example 32 | 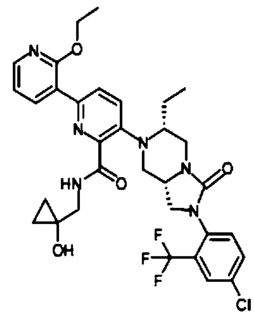 | 3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-(2-ethoxypyridin-3-yl)-N-[(1-hydroxycyclopropyl)methyl]pyridine-2-carboxamide | 659.3 |
| Example 33 | 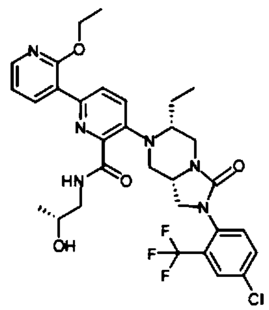 | 3-[(6R,8aS)-2-[4-chloro-2-(trifluoromethyl)phenyl]-6-ethyl-3-oxo-5, 6, 8, 8a-tetrahydro-1H-imidazo[1,5-a]pyrazin-7-yl]-6-(2-ethoxypyridin-3-yl)-N-[(2R)-2-hydroxypropyl]pyridine-2-carboxamide | 647.3 |
| Example 34 | 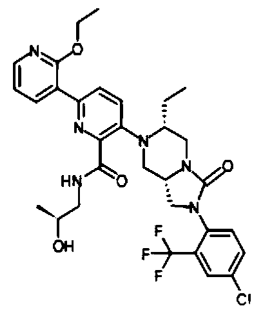 | 3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-(2-ethoxypyridin-3-yl)-N-[(2S)-2-hydroxypropyl]pyridine-2-carboxamide | 647.3 |

[Table 1-8]

| | | | |
|---|---|---|---|
| Example 35 | 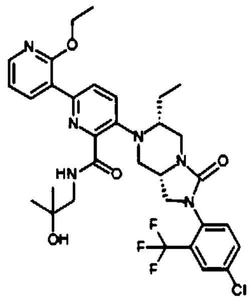 | 3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-(2-ethoxypyridin-3-yl)-N-(2-hydroxy-2-methylpropyl)pyridine-2-carboxamide | 661.3 |
| Example 36 | 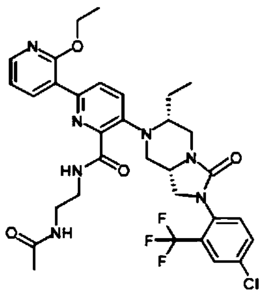 | N-(2-acetamidoethyl)-3-[(6R, 8aS)-2-[4-chloro-2-(trifluoromethyl)phenyl]-6-ethyl-3-oxo-5, 6, 8, 8a-tetrahydro-1H-imidazo [1,5-a]pyrazin-7-yl]-6-(2-ethoxypyridin-3-yl)pyridine-2-carboxamide | 674.3 |
| Example 37 | 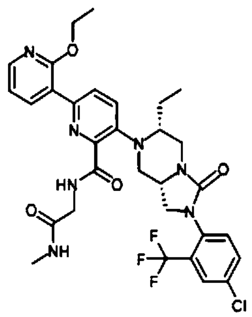 | 3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-(2-ethoxypyridin-3-yl)-N-[2-(methylamino)-2-oxoethyl]pyridine-2-carboxamide | 660.3 |
| Example 38 | 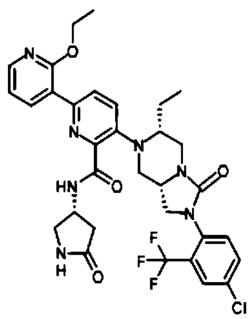 | 3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-(2-ethoxypyridin-3-yl)-N-[(3R)-5-oxopyrrolidin-3-yl]pyridine-2-carboxamide | 672.4 |
| Example 38a | 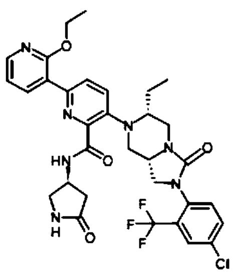 | 3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-(2-ethoxypyridyl)pyridine-2-carboxylic acid | 590.3 |

[Table 1-9]

| | | | |
|---|---|---|---|
| Example 39 | 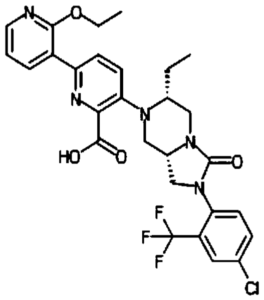 | 3-[(6R,8aS)-2-[4-chloro-2-(trifluoromethyl)phenyl]-6-ethyl-3-oxo-5, 6, 8, 8a-tetrahydro-1H-imidazo[1, 5-a]pyrazin-7-yl]-6-(2-ethoxypyridin-3-yl)-N-[(3S)-5-oxopyrrol idin-3-yl]pyridine-2-carboxamide | 672.3 |
| Example 40 | 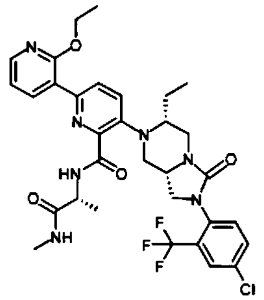 | 3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-(2-ethoxypyridin-3-yl)-N-[(2R)-1-(methylamino)-1-oxopropan-2-yl] pyridine-2-carboxamide | 674.3 |
| Example 41 | 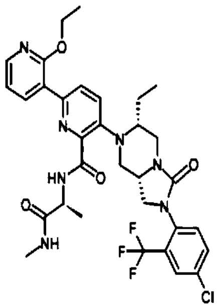 | 3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-(2-ethoxypyridin-3-yl)-N-[(2S)-1-(methylamino)-1-oxopropan-2-yl] pyridine-2-carboxamide | 674.3 |
| Example 42 | 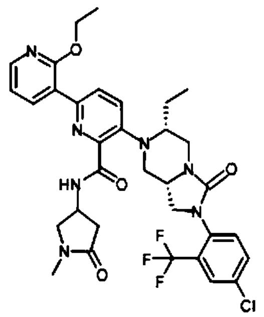 | 3-[(6R,8aS)-2-[4-chloro-2-(trifluoromethyl)phenyl]-6-ethyl-3-oxo-5, 6, 8, 8a-tetrahydro-1H-imidazo[1,5-a]pyrazin-7-yl]-6-(2-ethoxypyridin-3-yl)-N-(1-methyl-5-oxopyrrolidin-3-yl)pyridine-2-carboxamide | 686.3 |
| Example 43 | 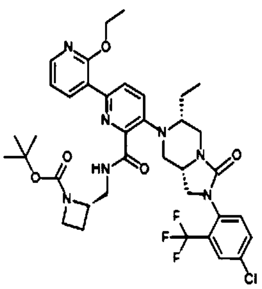 | tert-butyl (2R)-2-[[[3-[(6R,8aS)-2-[4-chloro-2-(trifluoromethyl) phenyl]-6-ethyl-3-oxo-5,6,8,8a-tetrahydro-1H-imidazo[1, 5-a] pyrazin-7-yl]-6-(2-ethoxypyridin-3-yl)pyridine-2-carbonyl]amino]methyl]azetidine-1-carboxylate | 758.4 |

[Table 1-10]

| | | | |
|---|---|---|---|
| Example 44 | 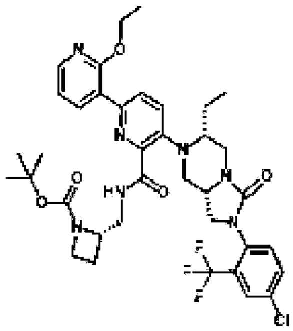 | 3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-N-[[(2R)-azetidin-2-yl]methyl]-6-(2-ethoxypyridin-3-yl)pyridine-2-carboxamide | 658.3 |
| Example 45 | 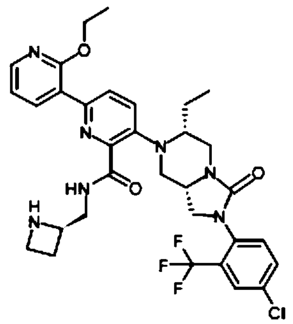 | tert-butyl (2S)-2-[[[3-[(6R,8aS)-2-[4-chloro-2-(trifluoromethyl) phenyl]-6-ethyl-3-oxo-5,6,8,8a-tetrahydro-1H-imidazo[1,5-a] pyrazin-7-yl]-6-(2-ethoxypyridin-3-yl)pyridine-2-carbonyl] amino]methyl]azetidine-1-carboxylate | 758.4 |
| Example 46 | 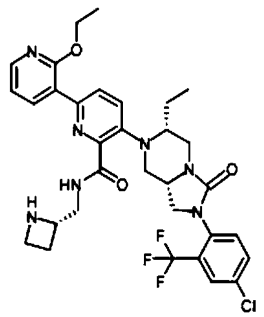 | 3-[(6R,8aS)-2-[4-chloro-2-(trifluoromethyl)phenyl]-6-ethyl-3-oxo-5, 6, 8, 8a-tetrahydro-1H-imidazo[1,5-a]pyrazin-7-yl]-N-[[(2S)-azetidin-2-yl]methyl]-6-(2-ethoxypyridin-3-yl)pyridine-2-carboxamide | 658.3 |
| Example 47 | 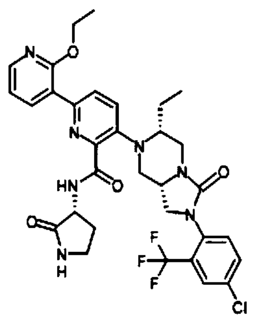 | 3-[(6R,8aS)-2-[4-chloro-2-(trifluoromethyl)phenyl]-6-ethyl-3-oxo-5,6,8,8a-tetrahydro-1H-imidazo[1, 5-a]pyrazin-7-yl]-6-(2-ethoxypyridin-3-yl)-N-[(3R)-2-oxopyrrol idin-3-yl]pyridine-2-carboxamide | 672.3 |

[Table 1-11]

| | | | |
|---|---|---|---|
| Example 48 | 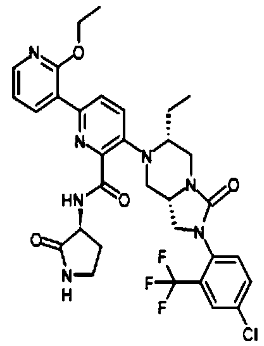 | 3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-(2-ethoxypyridin-3-yl)-N-[(3S)-2-oxopyrrolidin-3-yl]pyridine-2-carboxamide | 672.3 |
| Example 49 | 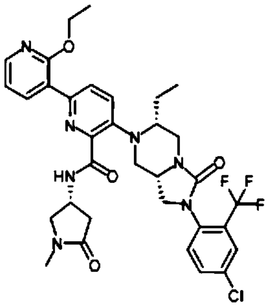 | 3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-(2-ethoxypyridin-3-yl)-N-[(3R)-1-methyl-5-oxopyrrolidin-3-yl]pyridine-2-carboxamide | 686.4 |
| Example 50 | 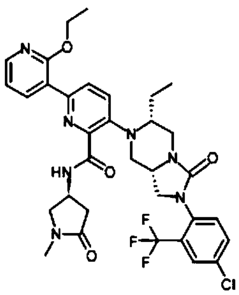 | 3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-(2-ethoxypyridin-3-yl)-N-[(3S)-1-methyl-5-oxopyrrolidin-3-yl]pyridine-2-carboxamide | 686.4 |
| Example 51 | 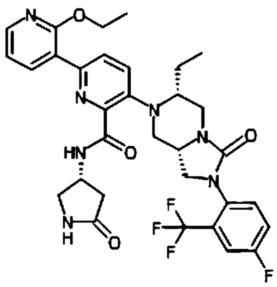 | 3-[(6R, 8aS)-6-ethyl-2-[4-fluoro-2-(trifluoromethyl)phenyl]-3-oxo-5, 6, 8, 8a-tetrahydro-1H-imidazo[1,5-a]pyrazin-7-yl]-6-(2-ethoxypyridin-3-yl)-N-[(3R)-5-oxopyrrolidin-3-yl]pyridine-2-carboxamide | 656.4 |
| Example 51a | 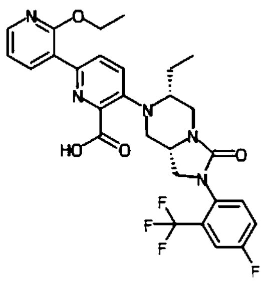 | 3-[(6R,8aS)-6-ethyl-2-[4-fluoro-2-(trifluoromethyl)phenyl]-3-oxo-5, 6, 8, 8a-tetrahydro-1H-imidazo[1, 5-a]pyrazin-7-yl]-6-(2-ethoxy-3-pyridyl)pyridine-2-carboxylic acid | 574.4 |

[Table 1-12]

| | | | |
|---|---|---|---|
| Example 52 | 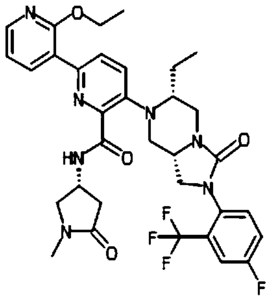 | 5, 6, 3-[(6R,8aS)-6-ethyl-2-[4-fluoro-2-(trifluoromethyl)phenyl]-3-oxo-5, 6, 8, 8a-tetrahydro-1H-imidazo[1,5-a]pyrazin-7-yl]-6-(2-ethoxypyridin-3-yl)-N-[(3R)-1-methyl-5-oxopyrrolidin-3-yl]pyridine-2-carboxamide | 670.2 |
| Example 53 | 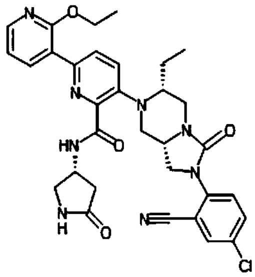 | 3-[(6R,8aS)-2-(4-chloro-2-cyanophenyl)-6-ethyl-3-oxo-5, 6, 8, 8a-tetrahydro-1H-imidazo[1,5-a]pyrazin-7-yl]-6-(2-ethoxypyridin-3-yl)-N-[(3R)-5-oxopyrrolidin-3-yl]pyridine-2-carboxamide | 629.4 |
| Example 53a | 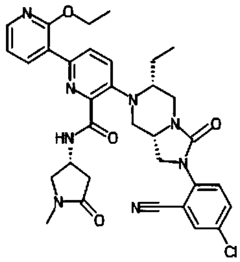 | 3-[(6R,8aS)-2-(4-chloro-2-cyanophenyl)-6-ethyl-3-oxo-5, 6, 8, 8a-tetrahydro-1H-imidazo[1, 5-a]pyrazin-7-yl]-6-(2-ethoxy-3-pyridyl)pyridine-2-carboxylic acid | 547.3 |
| Example 54 | 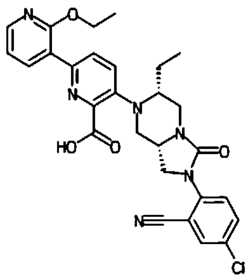 | 3-[(6R,8aS)-2-(4-chloro-2-cyanophenyl)-6-ethyl-3-oxo-5, 6, 8, 8a-tetrahydro-1H-imidazo[1,5-a]pyrazin-7-yl]-6-(2-ethoxypyridin-3-yl)-N-[(3R)-1-methyl-5-oxopyrrolidin-3-yl]pyridine-2-carboxamide | 643.1 |
| Example 55 | 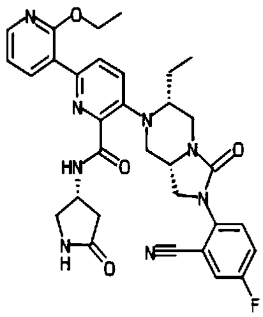 | 3-[(6R,8aS)-2-(2-cyano-4-fluorophenyl)-6-ethyl-3-oxo-5, 6, 8, 8a-tetrahydro-1H-imidazo[1,5-a]pyrazin-7-yl]-6-(2-ethoxypyridin-3-yl)-N-[(3R)-5-oxopyrrol idin-3-yl]pyridine-2-carboxamide | 613.4 |

[Table 1-13]

| | | | |
|---|---|---|---|
| Example 56 | 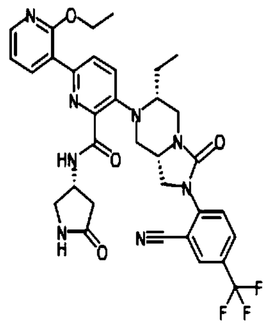 | 3-[(6R,8aS)-2-[2-cyano-4-(trifluoromethyl)phenyl]-6-ethyl-3-oxo-5,6,8,8a-tetrahydro-1H-imidazo[1,5-a]pyrazin-7-yl]-6-(2-ethoxypyridin-3-yl)-N-[(3R)-5-oxopyrrolidin-3-yl]pyridine-2-carboxamide | 663.3 |
| Example 57 | 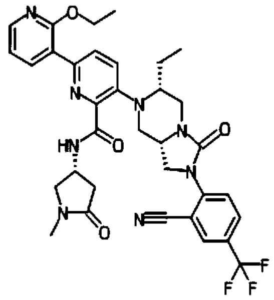 | 3-[(6R,8aS)-2-[2-cyano-4-(trifluoromethyl)phenyl]-6-ethyl-3-oxo-5,6,8,8a-tetrahydro-1H-imidazo[1,5-a]pyrazin-7-yl]-6-(2-ethoxypyridin-3-yl)-N-[(3R)-1-methyl-5-oxopyrrolidin-3-yl]pyridine-2-carboxamide | 677.4 |
| Example 58 | 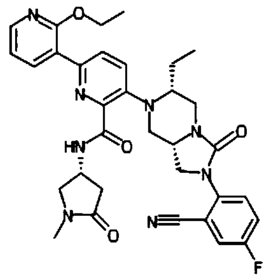 | 3-[(6R,8aS)-2-(2-cyano-4-fluorophenyl)-6-ethyl-3-oxo-5,6,8,8a-tetrahydro-1H-imidazo[1,5-a]pyrazin-7-yl]-6-(2-ethoxypyridin-3-yl)-N-[(3R)-1-methyl-5-oxopyrrolidin-3-yl]pyridine-2-carboxamide | 627.4 |

Example 59

3-[(6R,8aS)-6-ethyl-3-oxo-2-[1-(trifluoromethyl)cyclopropyl]-5,6,8,8a-tetrahydro-1H-imidazo[1,5-a]pyrazin-7-yl]-6-(2-ethoxy-3-pyridinyl)-N-[(3R)-1-methyl-5-oxopyrrolidin-3-yl]pyridine-2-carboxamide

The compound was synthesized according to the method shown in Example 10, Example 38 and Example 60 or a method analogous thereto.
MS: 616.4.

Example 60

3-[(6R,8aS)-2-[4-chloro-2-(trifluoromethyl)phenyl]-6-methyl-3-oxo-5,6,8,8a-tetrahydro-1H-imidazo[1,5-a]pyrazin-7-yl]-6-(2-ethoxy-3-pyridinyl)-N-[(3R)-1-methyl-5-oxopyrrolidin-3-yl]pyridine-2-carboxamide

Example 60a

3-[(6R,8aS)-2-[4-chloro-2-(trifluoromethyl)phenyl]-6-methyl-3-oxo-5,6,8,8a-tetrahydro-1H-imidazo[1,5-a]pyrazin-7-yl]-6-(2-ethoxy-3-pyridinyl)pyridine-2-carboxylic acid

A) methyl (2R)-2-[[(2R)-2-(benzyloxycarbonylamino)-3-hydroxy-propanoyl]amino]propanoate

To a mixture of (2R)-2-(benzyloxycarbonylamino)-3-hydroxy-propanoic acid (5 g, 20.9 mmol) and acetonitrile (40 mL) were added methyl (2R)-2-aminopropanoate hydrochloride (3.5 g, 25.1 mmol), HATU (20 g, 52.3 mmol) and N,N-diisopropylethylamine (11 mL, 62.7 mmol) under an argon atmosphere at room temperature, and the reaction mixture was

stirred at the same temperature for 16 hr. After completion of the reaction, the reaction mixture was concentrated, and the residue was diluted with saturated sodium carbonate aqueous solution and extracted with ethyl acetate. The extract was washed with 1M hydrochloric acid, water and saturated brine, dried over sodium sulfate, and concentrated under reduced pressure. The residue was purified by silica gel column chromatography (ethyl acetate/hexane) to give the title compound (6 g).

$^1$H NMR (400 MHz, DMSO-$d_6$) δ 1.28 (3H, d, J = 7.2 Hz), 3.45-3.55 (1H, m), 3.61 (4H, s), 4.05-4.15 (1H, m), 4.23-4.34 (1H, m), 4.78-4.86 (1H, m), 5.01-5.06 (2H, m), 7.22 (1H, d, J = 8.3 Hz), 7.28-7.39 (5H, m), 8.29 (1H, d, J = 7.1 Hz). MS: 325.3.

B) (3R,6R)-3-(hydroxymethyl)-6-methyl-piperazine-2,5-dione

A mixture of methyl (2R)-2-[[(2R)-2-(benzyloxycarbonylamino)-3-hydroxy-propanoyl]amino]propanoate (6 g, 18.5 mmol) and 10% Pd/C (1.3 g, ca. 50% in water) in methanol (50 mL) was stirred under a hydrogen atmosphere at room temperature for 16 hr. After completion of the reaction, Celite filtration was performed, and the filtrate was concentrated. The residue was used for the next reaction without further purification. A mixture of the obtained residue and methanol (30 mL) was stirred at 80°C for 16 hr and concentrated. The residue was washed with ethyl acetate to give the title compound (2.1 g).
$^1$H NMR (400 MHz, DMSO-$d_6$) δ 1.31 (3H, d, J = 6.9 Hz), 3.63-3.83 (4H, m), 5.06-5.13 (1H, m), 7.88 (1H, s), 8.12 (1H, s).

C) di-tert-butyl (2S,5R)-2-(hydroxymethyl)-5-methyl-piperazine-1,4-dicarboxylate

A mixture of (3R,6R)-3-(hydroxymethyl)-6-methylpiperazine-2,5-dione (2.1 g, 13.3 mmol) borane-tetrahydrofuran complex (1M tetrahydrofuran solution, 100 mL, 100 mmol) was stirred at 70°C for 16 hr. After cooling to 0°C, methanol (60 mL) was added to the reaction mixture, and 5M hydrochloric acid (30 mL) was further added to the reaction mixture. The mixture was stirred at 70°C for 2 hr. The mixture was concentrated, and the residue was washed with tetrahydrofuran. The obtained residue was used for the next reaction without further purification.

To a mixture of the obtained residue in methanol (30 mL) were added triethylamine (10 mL, 73.7 mmol) and di-tert-butyl dicarbonate (6.7 mL, 29.5 mmol) at 0°C. The mixture was stirred at 70°C for 16 hr. After completion of the reaction, the reaction mixture was concentrated, and the residue was dissolved in ethyl acetate. The organic layer was washed with water and saturated brine, dried over sodium sulfate, and concentrated under reduced pressure. The residue was purified by silica gel column chromatography (ethyl acetate/hexane) to give the title compound (1.5 g).
$^1$H NMR (400 MHz, DMSO-$d_6$) δ 1.01 (3H, d, J = 5.8 Hz), 1.35-1.42 (18H, m), 2.74-2.85 (1H, m), 2.90-3.07 (1H, m), 3.36-3.46 (2H, m), 3.50-3.63 (1H, m), 3.79-3.84 (3H, m), 4.73-4.83 (1H, m).

D) tert-butyl (2R,5S)-5-(hydroxymethyl)-2-methyl-piperazine-1-carboxylate

To a mixture of di-tert-butyl (2S,5R)-2-(hydroxymethyl)-5-methyl-piperazine-1,4-dicarboxylate (2.5 g, 7.58 mmol) in ethanol (50 mL) was added an aqueous solution of water (25 mL) and sodium hydroxide (760 mg, 18.9 mmol), and the mixture was heated under reflux for 16 hr. After cooling to 0°C, 1.5M hydrochloric acid was added to adjust to pH9, and the mixture was extracted with dichloromethane. The organic layer was washed with water and saturated brine, dried over sodium sulfate, and concentrated. The residue was purified by silica gel column chromatography (dichloromethane/methanol) to give the title compound (1 g).
$^1$H NMR (400 MHz, $CD_3OD$-$d_4$) 1.20 (3H, dd, J = 15.2, 6.9 Hz), 1.45 (9H, s), 2.62-2.66 (2H, m), 2.79-2.93 (2H, m), 3.47-3.53 (2H, m), 3.85 (1H, d, J = 10.7 Hz), 4.12-4.18 (1H, m).

E) tert-butyl (2R,5S)-4-[[4-chloro-2-(trifluoromethyl)phenyl]carbamoyl]-5-(hydroxymethyl)-2-methylpiperazine-1-carboxylate

To a solution of tert-butyl (2R,5S)-5-(hydroxymethyl)-2-methyl-piperazine-1-carboxylate (1 g, 4.34 mmol) in tetrahydrofuran (20 mL) was added 4-chloro-1-isocyanate-2-(trifluoromethyl)benzene (962 mg, 4.34 mmol) under an argon atmosphere at room temperature. The reaction mixture was stirred at room temperature for 4 hr, and the solvent was evaporated. The residue was purified by silica gel column chromatography (ethyl acetate/hexane) to give the title compound (1.2 g).

$^1$H NMR (400 MHz, DMSO-$d_6$) δ 1.04 (3H, d, J = 5.5 Hz), 1.38 (9H, s), 2.76-2.93 (2H, m), 3.49 (2H, s), 3.88-3.99 (4H, m), 5.69 (1H, s), 7.68 (3H, d, J = 9.8 Hz), 8.73 (1H, s).
MS: 452.1.

F) tert-butyl (6R,8aR)-2-[4-chloro-2-(trifluoromethyl)phenyl]-6-methyl-3-oxo-5,6,8,8a-tetrahydro-1H-imidazo[1,5-a]pyrazine-7-carboxylate

To a mixture of tert-butyl (2R,5S)-4-[[4-chloro-2-(trifluoromethyl)phenyl]carbamoyl]-5-(hydroxymethyl)-2-methylpiperazine-1-carboxylate (1.2 g, 2.66 mmol) in dichloromethane (30 mL) was added dropwise 1,8-diazabicyclo [5.4.0]-7-undecene (3.2 mL, 21.2 mmol) at room temperature under an argon atmosphere, and then methanesulfonyl chloride (0.5 mL, 6.64 mmol) was added dropwise at 0°C. The mixture was stirred at room temperature for 16 hr and added to ice water. The mixture was extracted with ethyl acetate, and the organic layer was washed with water and saturated brine, dried over sodium sulfate, and concentrated. The residue was purified by silica gel column chromatography (hexane/ethyl acetate) to give the title compound (900 mg).

$^1$H NMR (400 MHz, DMSO-$d_6$) δ 1.03-1.26 (3H, m), 1.42 (9H, s), 2.74-3.10 (2H, m), 3.50 (1H, d, J = 13.3 Hz), 3.60-4.29 (5H, m), 7.61 (1H, d, J = 8.3 Hz), 7.82-7.90 (2H, m).
MS: 433.9.

G) (6R,8aS)-2-[4-chloro-2-(trifluoromethyl)phenyl]-6-methyl-1,5,6,7,8,8a-hexahydroimidazo[1,5-a]pyrazin-3-one

To a solution of tert-butyl (6R,8aR)-2-[4-chloro-2-(trifluoromethyl)phenyl]-6-methyl-3-oxo-5,6,8,8a-tetrahydro-1H-imidazo[1,5-a]pyrazine-7-carboxylate (900 mg, 2.07 mmol) in dichloromethane (20 mL) was added trifluoroacetic acid (1.6 mL, 20.7 mmol) at 0°C. The reaction mixture was stirred at room temperature for 16 hr. After completion of the reaction, the reaction mixture was cooled to 0°C and added to ice-cooled saturated aqueous sodium hydrogen carbonate solution. The reaction mixture was extracted with dichloromethane, and the organic layer was washed with water and saturated brine, dried over sodium sulfate, and concentrated. The residue was purified by silica gel column chromatography (methanol/dichloromethane) to give the title compound (650 mg).

$^1$H NMR (400 MHz, DMSO-$d_6$) δ 1.09 (3H, d, J = 6.9 Hz), 2.66-2.75 (1H, m), 2.83-2.97 (1H, m), 3.01-3.13 (2H, m), 3.19-3.27 (1H, m), 3.36-3.44 (1H, m), 3.58-3.78 (2H, m), 3.98-4.08 (1H, m), 7.56 (1H, d, J = 8.3 Hz), 7.79-7.88 (2H, m).
MS: 333.7.

H) 3-[(6R,8aS)-2-[4-chloro-2-(trifluoromethyl)phenyl]-6-methyl-3-oxo-5,6,8,8a-tetrahydro-1H-imidazo[1,5-a]pyrazin-7-yl]-6-bromopyridine-2-carbaldehyde

6-Bromo-3-fluoro-pyridine-2-carbaldehyde (600 mg, 2.92 mmol) and potassium carbonate (810 mg, 5.84 mmol) were added to a mixture of (6R,8aS)-2-[4-chloro-2-(trifluoromethyl)phenyl]-6-methyl-1,5,6,7,8,8a-hexahydroimidazo [1,5-a]pyrazin-3-one (650 mg, 1.95 mmol) in toluene (10 mL) at room temperature. The mixture was heated under reflux for 72 hr. The reaction mixture was cooled to room temperature and ice water was added. The mixture was extracted with ethyl acetate. The obtained organic layer was washed with saturated brine, dried over sodium sulfate, and concentrated. The residue was purified by silica gel column chromatography (hexane/ethyl acetate) to give the title compound (635 mg).

$^1$H NMR (400 MHz, DMSO-$d_6$) δ 1.04 (3H, d, J = 6.6 Hz), 3.24-3.16 (1H, m), 3.43-3.33 (2H, m), 3.49-3.40 (1H, m), 3.57 (1H, d, J = 13.1 Hz), 3.77-3.73 (1H, m), 3.86 (1H, t, J = 8.6 Hz), 4.02-3.98 (1H, m), 7.63 (2H, t, J = 7.9 Hz), 7.75 (1H, d, J = 8.8 Hz), 7.91-7.84 (2H, m), 9.86 (1H, s).
MS: 517.1.

I) 3-[(6R,8aS)-2-[4-chloro-2-(trifluoromethyl)phenyl]-6-methyl-3-oxo-5,6,8,8a-tetrahydro-1H-imidazo[1,5-a]pyrazin-7-yl]-6-(2-ethoxy-3-pyridyl)pyridine-2-carbaldehyde

To a deaerated solution of 3-[(6R,8aS)-2-[4-chloro-2-(trifluoromethyl)phenyl]-6-methyl-3-oxo-5,6,8,8a-tetrahydro-1H-imidazo[1,5-a]pyrazin-7-yl]-6-bromopyridine-2-carbaldehyde (300 mg, 0.58 mmol) in 1,4-dioxane (18 mL) and water (2 mL) were added (2-ethoxy-3-pyridyl)boronic acid (145 mg, 0.87 mmol) and potassium carbonate (240 mg, 1.74 mmol) at room temperature. The reaction mixture was deaerated again, Pd(DtBPF)$Cl_2$ (20 mg, 0.029 mmol) was added under an argon atmosphere, and the mixture was stirred at 100°C for 2 hr. After cooling to room temperature, the mixture was diluted with water and extracted with ethyl acetate. The organic layer was separated, washed with saturated brine, dried over sodium sulfate, and concentrated. The residue was purified by silica gel column chromatography (hexane/ethyl acetate) to give the title compound (195 mg).

$^1$H NMR (400 MHz, DMSO-$d_6$) δ 1.06 (3H, d, J = 6.7 Hz), 1.38 (3H, t, J = 7.0 Hz), 3.31 (3H, d, J = 9.5 Hz), 3.35-3.52 (2H, m), 3.55-3.63 (1H, m), 3.77-3.92 (2H, m), 4.40-4.50 (2H, m), 7.11-7.19 (1H, m), 7.62-7.75 (2H, m), 7.85-7.93 (2H, m),

8.19-8.25 (2H, m), 8.30-8.37 (1H, m), 10.05 (1H, s).
MS: 560.3.

J) 3-[(6R,8aS)-2-[4-chloro-2-(trifluoromethyl)phenyl]-6-methyl-3-oxo-5,6,8,8a-tetrahydro-1H-imidazo[1,5-a]pyrazin-7-yl]-6-(2-ethoxy-3-pyridinyl)pyridine-2-carboxylic acid

To a mixture of 3-[(6R,8aS)-2-[4-chloro-2-(trifluoromethyl)phenyl]-6-methyl-3-oxo-5,6,8,8a-tetrahydro-1H-imidazo[1,5-a]pyrazin-7-yl]-6-(2-ethoxy-3-pyridyl)pyridine-2-carbaldehyde (195 mg, 0.349 mmol) in tert-butanol (2 mL), tetrahydrofuran (2 mL), water (2 mL) and 2-methyl-2-butene (0.4 mL, 3.45 mmol) were added $NaClO_2$ (80 mg, 0.872 mmol) and $KH_2PO_4$ (95 mg, 0.698 mmol) at 0°C. The reaction mixture was stirred at room temperature for 2 hr. To the reaction mixture were added saturated sodium thiosulfate aqueous solution and saturated ammonium chloride aqueous solution. The mixture was extracted with ethyl acetate and the organic layer was separated. The organic layer was washed with 1N hydrochloric acid, water and saturated brine, dried over sodium sulfate, and concentrated. To the obtained residue were added dichloromethane (1 mL) and a solution of 30% ether in pentane solution (10 mL) and the mixture was stirred. The obtained crystal was collected by filtration and washed with pentane to give the title compound (155 mg).
MS: 576.3.

K) 3-[(6R,8aS)-2-[4-chloro-2-(trifluoromethyl)phenyl]-6-methyl-3-oxo-5,6,8,8a-tetrahydro-1H-imidazo[1,5-a]pyrazin-7-yl]-6-(2-ethoxy-3-pyridinyl)-N-[(3R)-1-methyl-5-oxopyrrolidin-3-yl]pyridine-2-carboxamide

To a solution of 3-[(6R,8aS)-2-[4-chloro-2-(trifluoromethyl)phenyl]-6-methyl-3-oxo-5,6,8,8a-tetrahydro-1H-imidazo[1,5-a]pyrazin-7-yl]-6-(2-ethoxy-3-pyridyl)pyridine-2-carboxylic acid (150 mg, 0.26 mmol) in N,N-dimethylformamide (5 mL) were added tetrafluoroboric acid 2-bromo-1-ethylpyridinium (45 mg, 0.391 mmol), N,N-diisopropylethylamine (0.1 mL, 0.521 mmol) and (4R)-4-amino-1-methylpyrrolidin-2-one (45 mg, 0.391 mmol) under an argon atmosphere at room temperature. The reaction mixture was stirred at room temperature for 16 hr and water was added. The mixture was extracted with ethyl acetate and the organic layer was separated. The organic layer was washed with saturated aqueous sodium hydrogen carbonate solution and saturated brine, dried over sodium sulfate, and concentrated. The residue was purified by preparative HPLC to give the title compound (62 mg).

<Preparative HPLC conditions>

Preparative HPLC was performed using an automatic purification device manufactured by Waters. column name: YMC-Triart C18 (100×30 mm, 5 μm), periphery temperature: room temperature, flow rate: 30 mL/min, mobile phase: A=20 mM ammonium bicarbonate aqueous solution, B=acetonitrile.
MS: 672.4.

Example 61

3-[(6R,8aS)-2-[4-chloro-2-(trifluoromethyl)phenyl]-6-methyl-3-oxo-5,6,8,8a-tetrahydro-1H-imidazo[1,5-a]pyrazin-7-yl]-6-(2-methoxy-3-pyridinyl)-N-[(3R)-1-methyl-5-oxopyrrolidin-3-yl]pyridine-2-carboxamide

Example 61a

3-[(6R,8aS)-2-[4-chloro-2-(trifluoromethyl)phenyl]-6-methyl-3-oxo-5,6,8,8a-tetrahydro-1H-imidazo[1,5-a]pyrazin-7-yl]-6-(2-methoxy-3-pyridinyl)pyridine-2-carboxylic acid

A) 3-[(6R,8aS)-2-[4-chloro-2-(trifluoromethyl)phenyl]-6-methyl-3-oxo-5,6,8,8a-tetrahydro-1H-imidazo[1,5-a]pyrazin-7-yl]-6-(2-methoxy-3-pyridyl)pyridine-2-carbaldehyde

To a deaerated solution of 3-[(6R,8aS)-2-[4-chloro-2-(trifluoromethyl)phenyl]-6-methyl-3-oxo-5,6,8,8a-tetrahydro-1H-imidazo[1,5-a]pyrazin-7-yl]-6-bromo-pyridine-2-carbaldehyde (300 mg, 0.58 mmol) in 1,4-dioxane (18 mL) and water (2 mL) were added (2-methoxy-3-pyridyl)boronic acid (135 mg, 0.869 mmol) and potassium carbonate (240 mg, 1.74 mmol) at room temperature. The reaction mixture was deaerated again, $Pd(DtBPF)Cl_2$ (20 mg, 0.029 mmol) was added under an argon atmosphere, and the mixture was stirred at 100°C for 2 hr. After cooling to room temperature, the mixture was diluted with water and extracted with ethyl acetate. The organic layer was separated, washed with saturated brine, dried over sodium sulfate, and concentrated. The residue was purified by silica gel column chromatography (hexane/ethyl acetate) to give the title compound (200 mg).

$^1H$ NMR (400 MHz, DMSO-$d_6$) δ 1.06 (3H, d, J = 6.3 Hz), 3.31 - 3.36 (1H, m), 3.37 - 3.42 (2H, m), 3.44 - 3.52 (1H, m), 3.56 - 3.63 (1H, m), 3.76 - 3.92 (2H, m), 3.95 - 4.00 (3H, m), 4.01 - 4.06 (1H, m), 7.15 - 7.20 (1H, m), 7.63 - 7.73 (2H, m), 7.85 - 7.92 (2H, m), 8.15 (1H, d, J = 8.9 Hz), 8.22 - 8.34 (2H, m),10.05 (1H, s).
MS: 546.2.

B) 3-[(6R,8aS)-2-[4-chloro-2-(trifluoromethyl)phenyl]-6-methyl-3-oxo-5,6,8,8a-tetrahydro-1H-imidazo[1,5-a]pyrazin-7-yl]-6-(2-methoxy-3-pyridinyl)pyridine-2-carboxylic acid

To a mixture of 3-[(6R,8aS)-2-[4-chloro-2-(trifluoromethyl)phenyl]-6-methyl-3-oxo-5,6,8,8a-tetrahydro-1H-imidazo[1,5-a]pyrazin-7-yl]-6-(2-methoxy-3-pyridyl)pyridine-2-carbaldehyde (200 mg, 0.37 mmol) in tert-butanol (2 mL), tetrahydrofuran (2 mL), water (2 mL) and 2-methyl-2-butene (0.4 mL, 3.66 mmol) were added $NaClO_2$ (83 mg, 0.916 mmol) and $KH_2PO_4$ (100 mg, 0.733 mmol) at 0°C. The reaction mixture was stirred at room temperature for 2 hr. To the reaction mixture were added saturated sodium thiosulfate aqueous solution and saturated ammonium chloride aqueous solution. The mixture was extracted with ethyl acetate and the organic layer was separated. The organic layer was washed with 1N hydrochloric acid, water and saturated brine, dried over sodium sulfate, and concentrated. To the obtained residue were added dichloromethane (2 mL) and 20% ether in pentane solution (20 mL) and the mixture was stirred. The obtained crystal was collected by filtration and washed with pentane to give the title compound (155 mg).
MS: 562.1.

C) 3-[(6R,8aS)-2-[4-chloro-2-(trifluoromethyl)phenyl]-6-methyl-3-oxo-5,6,8,8a-tetrahydro-1H-imidazo[1,5-a]pyrazin-7-yl]-6-(2-methoxy-3-pyridinyl)-N-[(3R)-1-methyl-5-oxopyrrolidin-3-yl]pyridine-2-carboxamide

To a solution of 3-[(6R,8aS)-2-[4-chloro-2-(trifluoromethyl)phenyl]-6-methyl-3-oxo-5,6,8,8a-tetrahydro-1H-imidazo[1,5-a]pyrazin-7-yl]-6-(2-methoxy-3-pyridinyl)pyridine-2-carboxylic acid (150 mg, 0.267 mmol) in N,N-dimethylformamide (5 mL) were added tetrafluoroboric acid 2-bromo-1-ethylpyridinium (46 mg, 0.4 mmol), N,N-diisopropylethylamine (0.1 mL, 0.534 mmol) and (4R)-4-amino-1-methyl-pyrrolidin-2-one (46 mg, 0.4 mmol) under an argon atmosphere at room temperature. The reaction mixture was stirred for 16 hr at room temperature, and water was added. The mixture was extracted with ethyl acetate, and the organic layer was separated. The organic layer was washed with saturated aqueous sodium hydrogen carbonate solution and saturated brine, dried over sodium sulfate, and concentrated. The residue was purified by preparative HPLC to give the title compound (131 mg).

<Preparative HPLC conditions>

Preparative HPLC was performed using an automatic purification device manufactured by Waters. column name: YMC-Triart C18 (100×30 mm, 5 μm), periphery temperature: room temperature, flow rate: 30 mL/min, mobile phase: A=20 mM ammonium bicarbonate aqueous solution, B=acetonitrile.

$^1H$ NMR (400 MHz, DMSO-$d_6$) δ1.07 (3H, d, J = 6.6 Hz), 2.39 (1H, dd, J = 16.8, 5.4 Hz), 2.67 (1H, dd, J = 16.6, 8.8 Hz), 2.76 (3H, s), 3.14 (1H, d, J = 3.9 Hz), 3.27-3.44 (4H, m), 3.60 (1H, d, J = 12.8 Hz), 3.74 (1H, dd, J = 9.9, 7.5 Hz), 3.80-3.97 (3H, m), 4.00 (3H, s), 4.56 (1H, d, J = 7.0 Hz), 7.12 (1H, dd, J = 7.4, 4.9 Hz), 7.57 (2H, dd, J = 17.4, 8.8 Hz), 7.82 (2H, d, J = 7.0 Hz), 8.00 (1H, d, J = 8.6 Hz), 8.21 (1H, d, J = 4.8 Hz), 8.28 (1H, d, J = 7.4 Hz), 8.56 (1H, s).
MS: 658.4.

Example 62

3-[(6R,8aS)-6-ethyl-3-oxo-2-[1-(trifluoromethyl)cyclopropyl]-5,6,8,8a-tetrahydro-1H-imidazo[1,5-a]pyrazin-7-yl]-6-(2-ethoxyphenyl)-N-[(3R)-1-methyl-5-oxopyrrolidin-3-yl]pyridine-2-carboxamide

The compound was synthesized according to the method shown in Example 10, Example 38 and Example 60 or a method analogous thereto.
MS: 615.5.

Example 63

3-[(6R,8aS)-2-[4-chloro-2-(trifluoromethyl)phenyl]-6-methyl-3-oxo-5,6,8,8a-tetrahydro-1H-imidazo[1,5-a]pyrazin-7-yl]-6-(2-ethoxyphenyl)-N-[(3R)-1-methyl-5-oxopyrrolidin-3-yl]pyridine-2-carboxamide

Example 63a

3-[(6R,8aS)-2-[4-chloro-2-(trifluoromethyl)phenyl]-6-methyl-3-oxo-5,6,8,8a-tetrahydro-1H-imidazo[1,5-a]pyrazin-7-yl]-6-(2-ethoxyphenyl)pyridine-2-carboxylic acid

A) 3-[(6R,8aS)-2-[4-chloro-2-(trifluoromethyl)phenyl]-6-methyl-3-oxo-5,6,8,8a-tetrahydro-1H-imidazo[1,5-a]pyrazin-7-yl]-6-(2-ethoxyphenyl)pyridine-2-carbaldehyde

To a deaerated solution of 3-[(6R,8aS)-2-[4-chloro-2-(trifluoromethyl)phenyl]-6-methyl-3-oxo-5,6,8,8a-tetrahydro-1H-imidazo[1,5-a]pyrazin-7-yl]-6-bromo-pyridine-2-carbaldehyde (200 mg, 0.483 mmol) in 1,4-dioxane (18 mL) and water (2 mL) were added (2-ethoxyphenyl)boronic acid (71 mg, 0.425 mmol) and potassium carbonate (160 mg, 1.159 mmol) at room temperature. The reaction mixture was deaerated again, Pd(DtBPF)$Cl_2$ (16 mg, 0.019 mmol) was added under an argon atmosphere, and the mixture was stirred at 100°C for 2 hr. After cooling to room temperature, the mixture was diluted with water and extracted with ethyl acetate. The organic layer was separated, washed with saturated brine, dried over sodium sulfate, and concentrated. The residue was purified by silica gel column chromatography (hexane/ethyl acetate) to give the title compound (155 mg).

$^1$H NMR (400 MHz, DMSO-$d_6$) δ 1.02-1.10 (3H, m), 1.36 (3H, t, J = 6.9 Hz), 3.22-3.28 (1H, m), 3.36-3.52 (3H, m), 3.56-3.68 (1H, m), 3.78-3.84 (1H, m), 3.88 (1H, t, J = 8.6 Hz), 3.98-4.08 (1H, m), 4.09-4.19 (2H, m), 7.01-7.18 (2H, m), 7.33-7.43 (1H, m), 7.62-7.70 (2H, m), 7.82-7.92 (3H, m), 8.12 (1H, d, J = 8.8 Hz), 10.06 (1H, s).
MS: 559.0.

B) 3-[(6R,8aS)-2-[4-chloro-2-(trifluoromethyl)phenyl]-6-methyl-3-oxo-5,6,8,8a-tetrahydro-1H-imidazo[1,5-a]pyrazin-7-yl]-6-(2-ethoxyphenyl)pyridine-2-carboxylic acid

To a mixture of 3-[(6R,8aS)-2-[4-chloro-2-(trifluoromethyl)phenyl]-6-methyl-3-oxo-5,6,8,8a-tetrahydro-1H-imidazo[1,5-a]pyrazin-7-yl]-6-(2-ethoxyphenyl)pyridine-2-carbaldehyde (155 mg, 0.277 mmol) in tert-butanol (3 mL), tetrahydrofuran (3 mL), water (3 mL) and 2-methyl-2-butene (0.3 mL, 2.773 mmol) were added $NaClO_2$ (65 mg, 0.693 mmol) and $KH_2PO_4$ (76 mg, 0.555 mmol) at 0°C. The reaction mixture was stirred at room temperature for 2 hr. To the reaction mixture were added saturated sodium thiosulfate aqueous solution and saturated ammonium chloride aqueous solution. The mixture was extracted with ethyl acetate and the organic layer was separated. The organic layer was washed with 1N hydrochloric acid, water and saturated brine, dried over sodium sulfate, and concentrated. To the obtained residue were added dichloromethane (1 mL) and 30% ether in pentane solution (10 mL) and the mixture was stirred. The obtained crystal was collected by filtration and washed with pentane to give the title compound (150 mg).

$^1$H NMR (400 MHz, DMSO-$d_6$) δ 1.02 (3H, d, J = 6.7 Hz), 1.12-1.26 (3H, m), 1.36 (3H, t, J = 7.0 Hz), 3.08-3.16 (1H, m), 3.59 (1H, d, J = 12.9 Hz), 3.68-3.99 (3H, m), 4.07-4.17 (2H, m), 7.00-7.08 (1H, m), 7.12 (1H, d, J = 8.3 Hz), 7.32-7.40 (1H, m), 7.57 (1H, d, J = 8.7 Hz), 7.64 (1H, d, J = 8.3 Hz), 7.78 (1H, d, J = 7.5 Hz), 7.88 (2H, d, J = 11.1 Hz), 8.01 (1H, d, J = 8.8 Hz), 12.28-13.57 (1H, m).
MS: 575.3.

C) 3-[(6R,8aS)-2-[4-chloro-2-(trifluoromethyl)phenyl]-6-methyl-3-oxo-5,6,8,8a-tetrahydro-1H-imidazo[1,5-a]pyrazin-7-yl]-6-(2-ethoxyphenyl)-N-[(3R)-1-methyl-5-oxopyrrolidin-3-yl]pyridine-2-carboxamide

To a solution of 3-[(6R,8aS)-2-[4-chloro-2-(trifluoromethyl)phenyl]-6-methyl-3-oxo-5,6,8,8a-tetrahydro-1H-imidazo[1,5-a]pyrazin-7-yl]-6-(2-ethoxyphenyl)pyridine-2-carboxylic acid (150 mg, 0.261 mmol) in N,N-dimethylformamide (5 mL) were added tetrafluoroboric acid 2-bromo-1-ethylpyridinium (45 mg, 0.391 mmol), N,N-diisopropylethylamine (0.1 mL, 0.522 mmol) and (4R)-4-amino-1-methylpyrrolidin-2-one (45 mg, 0.39 mmol) under an argon atmosphere at room temperature. The reaction mixture was stirred at room temperature for 16 hr and water was added. The mixture was extracted with ethyl acetate and the organic layer was separated. The organic layer was washed with saturated aqueous sodium hydrogen carbonate solution and saturated brine, dried over sodium sulfate, and concentrated. The residue was purified by Preparative HPLC to give the title compound (45 mg).

<Preparative HPLC conditions>

Preparative HPLC was performed using an automatic purification device manufactured by Waters. column name: YMC-Triart C18 (100×30 mm, 5 μm), periphery temperature: room temperature, flow rate: 30 mL/min, mobile phase: A=20 mM ammonium bicarbonate aqueous solution, B=acetonitrile.

$^1$H NMR (400 MHz, DMSO-$d_6$) δ1.08 (3H, d, J = 6.7 Hz), 1.36 (3H, t, J = 6.9 Hz), 2.39 (1H, dd, J = 16.8, 5.4 Hz), 2.67

(1H, dd, J = 16.6, 8.7 Hz), 2.76 (3H, s), 3.15 (1H, dd, J = 11.9, 4.1 Hz), 3.28 - 3.45 (4H, m), 3.60 (1H, d, J = 12.8 Hz), 3.74 (1H, dd, J = 10.0, 7.4 Hz), 3.84 - 4.01 (3H, m), 4.14 (2H, q, J = 6.9 Hz), 4.54 - 4.59 (1H, m), 7.05 (1H, t, J = 7.5 Hz), 7.11 (1H, d, J = 8.3 Hz), 7.30 - 7.39 (1H, m), 7.52 (1H, d, J = 8.6 Hz), 7.59 (1H, d, J = 8.7 Hz), 7.78 - 7.85 (3H, m), 7.94 (1H, d, J = 8.6 Hz), 8.48 (1H, d, J = 6.8 Hz).
MS: 671.7.

Example 64

3-[(6R,8aS)-2-[4-chloro-2-(trifluoromethyl)phenyl]-6-methyl-3-oxo-5,6,8,8a-tetrahydro-1H-imidazo[1,5-a]pyrazin-7-yl]-6-(2-methoxyphenyl)-N-[(3R)-1-methyl-5-oxopyrrolidin-3-yl]pyridine-2-carboxamide

Example 64a

3-[(6R,8aS)-2-[4-chloro-2-(trifluoromethyl)phenyl]-6-methyl-3-oxo-5,6,8,8a-tetrahydro-1H-imidazo[1,5-a]pyrazin-7-yl]-6-(2-methoxyphenyl)pyridine-2-carboxylic acid

A) 3-[(6R,8aS)-2-[4-chloro-2-(trifluoromethyl)phenyl]-6-methyl-3-oxo-5,6,8,8a-tetrahydro-1H-imidazo[1,5-a]pyrazin-7-yl]-6-(2-methoxyphenyl)pyridine-2-carbaldehyde

To a deaerated solution of 3-[(6R,8aS)-2-[4-chloro-2-(trifluoromethyl)phenyl]-6-methyl-3-oxo-5,6,8,8a-tetrahydro-1H-imidazo[1,5-a]pyrazin-7-yl]-6-bromopyridine-2-carbaldehyde (100 mg, 0.193 mmol) in 1,4-dioxane (9 mL) and water (1 mL) were added (2-methoxyphenyl)boronic acid (32.3 mg, 0.212 mmol) and potassium carbonate (80 mg, 0.579 mmol) at room temperature. The reaction mixture was deaerated again, Pd(DtBPF)$Cl_2$ (8 mg, 0.01 mmol) was added under an argon atmosphere, and the mixture was stirred at 100°C for 2 hr. After cooling to room temperature, the mixture was diluted with water and extracted with ethyl acetate. The organic layer was separated, washed with saturated brine, dried over sodium sulfate, and concentrated. The residue was purified by silica gel column chromatography (hexane/ethyl acetate) to give the title compound (200 mg).
MS: 545.2.

B) 3-[(6R,8aS)-2-[4-chloro-2-(trifluoromethyl)phenyl]-6-methyl-3-oxo-5,6,8,8a-tetrahydro-1H-imidazo[1,5-a]pyrazin-7-yl]-6-(2-methoxyphenyl)pyridine-2-carboxylic acid

To a mixture of 3-[(6R,8aS)-2-[4-chloro-2-(trifluoromethyl)phenyl]-6-methyl-3-oxo-5,6,8,8a-tetrahydro-1H-imidazo[1,5-a]pyrazin-7-yl]-6-(2-methoxyphenyl)pyridine-2-carbaldehyde (75 mg, 0.138 mmol) in tert-butanol (1 mL), tetrahydrofuran (1 mL), water (1 mL) and 2-methyl-2-butene (0.15 mL, 1.38 mmol) were added $NaClO_2$ (32 mg, 0.344 mmol) and $KH_2PO_4$ (40 mg, 0.275 mmol) at 0°C. The reaction mixture was stirred at room temperature for 2 hr. To the reaction mixture were added saturated sodium thiosulfate aqueous solution and saturated ammonium chloride aqueous solution. The mixture was extracted with ethyl acetate and the organic layer was separated. The organic layer was washed with 1N hydrochloric acid, water and saturated brine, dried over sodium sulfate, and concentrated. To the obtained residue were added dichloromethane (1 mL) and 30% ether in pentane solution (10 mL) and the mixture was stirred. The obtained crystal was collected by filtration and washed with pentane to give the title compound (60 mg).
MS: 561.2.

C) 3-[(6R,8aS)-2-[4-chloro-2-(trifluoromethyl)phenyl]-6-methyl-3-oxo-5,6,8,8a-tetrahydro-1H-imidazo[1,5-a]pyrazin-7-yl]-6-(2-methoxyphenyl)-N-[(3R)-1-methyl-5-oxopyrrolidin-3-yl]pyridine-2-carboxamide

To a solution of 3-[(6R,8aS)-2-[4-chloro-2-(trifluoromethyl)phenyl]-6-methyl-3-oxo-5,6,8,8a-tetrahydro-1H-imidazo[1,5-a]pyrazin-7-yl]-6-(2-methoxyphenyl)pyridine-2-carboxylic acid (60 mg, 0.107 mmol) in N,N-dimethylformamide (5 mL) solution were added tetrafluoroboric acid 2-bromo-1-ethylpyridinium (20 mg, 0.16 mmol), N,N-diisopropylethylamine (0.05 mL, 0.214 mmol) and (4R)-4-amino-1-methylpyrrolidin-2-one (20 mg, 0.16 mmol) under an argon atmosphere at room temperature. The reaction mixture was stirred at room temperature for 16 hr and water was added. The mixture was extracted with ethyl acetate and the organic layer was separated. The organic layer was washed with saturated aqueous sodium hydrogen carbonate solution and saturated brine, dried over sodium sulfate, and concentrated. The residue was purified by Preparative HPLC to give the title compound (1.5 mg).

<Preparative HPLC conditions>

Preparative HPLC was performed using an automatic purification device manufactured by Waters. column

name: YMC-Triart C18 (100×30 mm, 5 μm), periphery temperature: room temperature, flow rate: 30 mL/min, mobile phase: A=20 mM ammonium bicarbonate aqueous solution, B=acetonitrile.
MS: 657.6.

Example 65

3-[(6R,8aS)-6-ethyl-3-oxo-2-[1-(trifluoromethyl)cyclobutyl]-5,6,8,8a-tetrahydro-1H-imidazo[1,5-a]pyrazin-7-yl]-6-(2-ethoxy-3-pyridinyl)-N-[(3R)-1-methyl-5-oxopyrrolidin-3-yl]pyridine-2-carboxamide

A) tert-butyl (2R,5S)-2-ethyl-5-(hydroxymethyl)-4-[[1-(trifluoromethyl)cyclobutyl]carbamoyl]piperazine-1-carboxylate

To a mixture of dichloromethane (25 mL), 1-(trifluoromethyl)cyclobutaneamine (770 mg, 5.525 mmol) and tritylamine (1.7 mL, 12.3 mmol) was added triphosgene (606 mg, 2.046 mmol) under an argon atmosphere at 0°C, and the reaction mixture was stirred at 25°C for 16 hr. To the reaction mixture was added a solution of tert-butyl (2R,5S)-2-ethyl-5-(hydroxymethyl)piperazine-1-carboxylate (1 g, 4.093 mmol) in dichloromethane (15 mL), and triethylamine (1.7 ml, 12.3 mmol) was successively added at room temperature, and the reaction mixture was stirred at room temperature for 4 hr. After completion of the reaction, the reaction mixture was filtered, and the filtrate was washed with dichloromethane. The filtrate was washed with saturated brine, dried over sodium sulfate, and concentrated under reduced pressure. The obtained residue was purified by silica gel column chromatography (ethyl acetate/hexane) to give the title compound (1.25 g).
$^1$H NMR (400 MHz, DMSO-$d_6$) δ 0.83 (3H, t, J = 7.5 Hz), 1.38 (11H, s), 1.71-1.98 (2H, m), 2.31-2.46 (4H, m), 2.65-2.85 (2H, m), 3.41 (2H, t, J = 4.9 Hz), 3.73-3.77 (1H, m), 3.79-4.05 (3H, m), 5.15-5.21 (1H, m), 6.87 (1H, s).

B) tert-butyl (6R,8aR)-6-ethyl-3-oxo-2-[1-(trifluoromethyl)cyclobutyl]-5,6,8,8a-tetrahydro-1H-imidazo[1,5-a]pyrazine-7-carboxylate

To a solution of tert-butyl (2R,5S)-2-ethyl-5-(hydroxymethyl)-4-[[1-(trifluoromethyl)cyclobutyl]carbamoyl]piperazine-1-carboxylate (1.25 g, 3.05 mmol) in dichloromethane (25 mL) was added 1,8-diazabicyclo[5.4.0]-7-undecene (3.65 mL, 24.4 mmol) under an argon atmosphere at room temperature, and methanesulfonyl chloride (0.6 mL, 7.632 mmol) was further added at 0°C. The reaction mixture was stirred at room temperature for 16 hr. After completion of the reaction, the reaction mixture was added to ice-cold water. The aqueous layer was extracted with ethyl acetate, and the organic layer was washed with water and saturated brine, dried over sodium sulfate, filtered, and the filtrate was concentrated under reduced pressure. The obtained residue was purified by silica gel column chromatography (ethyl acetate/hexane) to give the title compound (635 mg).
$^1$H NMR (400 MHz, DMSO-$d_6$) δ 0.73-0.86 (3H, m), 1.40 (11H, s), 1.84-1.96 (2H, m), 2.28-2.37 (2H, m), 2.56-2.69 (2H, m), 2.81-2.92 (1H, m), 3.01-3.08 (1H, m), 3.42-3.54 (3H, m), 3.78-4.07 (2H, m).

C) (6R,8aS)-6-ethyl-2-[1-(trifluoromethyl)cyclobutyl]-1,5,6,7,8,8a-hexahydroimidazo[1,5-a]pyrazin-3-one

To a stirred mixture of dichloromethane (30 mL) and tert-butyl (6R,8aR)-6-ethyl-3-oxo-2-[1-(trifluoromethyl)cyclobutyl]-5,6,8,8a-tetrahydro-1H-imidazo[1,5-a]pyrazine-7-carboxylate (600 mg, 1.533 mmol) was added trifluoroacetic acid (1.45 mL, 18.4 mmol) at 0°C, and the reaction mixture was stirred at room temperature for 16 hr. After completion of the reaction, the reaction mixture was cooled to 0°C and poured into sodium hydrogen carbonate aqueous solution under ice-cooling. The aqueous layer was extracted with dichloromethane, and the combined organic layer was washed with water and saturated brine, dried over sodium sulfate, filtered, and the filtrate was concentrated under reduced pressure. The obtained residue was purified by silica gel column chromatography (dichloromethane/methanol) to give the title compound (400 mg). $^1$H NMR (400 MHz, DMSO-$d_6$) δ 0.83 (3H, t, J = 7.4 Hz), 1.32 - 1.52 (2H, m), 1.83 - 1.96 (2H, m), 2.26 - 2.36 (2H, m), 2.59 (3H, d, J = 6.3 Hz), 2.83 - 2.93 (2H, m), 3.41 - 3.54 (3H, m), 3.73 - 3.97 (1H, m).

D) 3-[(6R,8aS)-6-ethyl-3-oxo-2-[1-(trifluoromethyl)cyclobutyl]-5,6,8,8a-tetrahydro-1H-imidazo[1,5-a]pyrazin-7-yl]-6-bromopyridine-2-carbaldehyde

To a mixture of (6R,8aS)-6-ethyl-2-[1-(trifluoromethyl)cyclobutyl]-1,5,6,7,8,8a-hexahydroimidazo[1,5-a]pyrazin-3-one (400 mg, 1.545 mmol) in toluene (15 mL) was added 6-bromo-3-fluoro-pyridine-2-carbaldehyde (475 mg, 2.317 mmol), and then potassium carbonate (640 mg, 4.63 mmol) was added at room temperature. The reaction mixture was heated under reflux for 3 days. The reaction mixture was cooled to room temperature, ice-cold water (50 mL) was added, and the aqueous layer was extracted with ethyl acetate (2x100 mL). The combined organic layer was washed with saturated brine, dried over sodium sulfate, filtered, and the filtrate was concentrated under reduced pressure. The

obtained residue was purified by silica gel column chromatography (ethyl acetate/hexane) to give the title compound (550 mg).
MS: 475.2.

E) 3-[(6R,8aS)-6-ethyl-3-oxo-2-[1-(trifluoromethyl)cyclobutyl]-5,6,8,8a-tetrahydro-1H-imidazo[1,5-a]pyrazin-7-yl]-6-(2-ethoxy-3-pyridyl)pyridine-2-carbaldehyde

3-[(6R,8aS)-6-ethyl-3-oxo-2-[1-(trifluoromethyl)cyclobutyl]-5,6,8,8a-tetrahydro-1H-imidazo[1,5-a]pyrazin-7-yl]-6-bromo-pyridine-2-carbaldehyde (200 mg, 0.421 mmol) was dissolved in a mixture of 1,4-dioxane (9 mL) and water (1 mL), and the mixture was subjected to a deaeration treatment. (2-Ethoxypyridin-3-yl)boronic acid (106 mg, 0.631 mmol) and potassium carbonate (175 mg, 1.262 mmol) were added at room temperature, and the mixture was deaerated again. To this mixture was added Pd(DtBPF)$Cl_2$ (15 mg, 0.021 mmol) under an argon atmosphere, and the reaction mixture was heated at 100°C for 2 hr. After completion of the reaction, the reaction mixture was cooled to room temperature and water (100 mL) was added. The aqueous layer was extracted with ethyl acetate (2x100mL). The combined organic layer was washed with saturated brine, dried over anhydrous sodium sulfate, filtered, and the filtrate was concentrated under reduced pressure. The obtained residue was purified by silica gel column chromatography (ethyl acetate/hexane) to give the title compound (200 mg).
MS: 518.2.

F) 3-[(6R,8aS)-6-ethyl-3-oxo-2-[1-(trifluoromethyl)cyclobutyl]-5,6,8,8a-tetrahydro-1H-imidazo[1,5-a]pyrazin-7-yl]-6-(2-ethoxy-3-pyridyl)pyridine-2-carboxylic acid

To a mixture of 3-[(6R,8aS)-6-ethyl-3-oxo-2-[1-(trifluoromethyl)cyclobutyl]-5,6,8,8a-tetrahydro-1H-imidazo[1,5-a]pyrazin-7-yl]-6-(2-ethoxy-3-pyridyl)pyridine-2-carbaldehyde (200 mg, 0.386 mmol) in tert-butanol (3 mL), tetrahydrofuran (3 mL), water (3 mL) and 2-methyl-2-butene (0.4 mL, 3.86 mmol) were added $NaClO_2$ (88 mg, 0.966 mmol) and $KH_2PO_4$ (106 mg, 0.773 mmol) at 0°C. The reaction mixture was stirred at room temperature for 2 hr. To the reaction mixture were added saturated sodium thiosulfate aqueous solution and saturated ammonium chloride aqueous solution. The mixture was extracted with ethyl acetate and the organic layer was separated. The organic layer was washed with 1N hydrochloric acid, water and saturated brine, dried over sodium sulfate, and concentrated. To the obtained residue were added dichloromethane (1 mL) and 30% ether in pentane solution (10 mL) and the mixture was stirred. The obtained crystal was collected by filtration and washed with pentane to give the title compound (150 mg).
MS: 534.1.

G) 3-[(6R,8aS)-6-ethyl-3-oxo-2-[1-(trifluoromethyl)cyclobutyl]-5,6,8,8a-tetrahydro-1H-imidazo[1,5-a]pyrazin-7-yl]-6-(2-ethoxy-3-pyridinyl)-N-[(3R)-1-methyl-5-oxopyrrolidin-3-yl]pyridine-2-carboxamide

To a solution of 3-[(6R,8aS)-6-ethyl-3-oxo-2-[1-(trifluoromethyl)cyclobutyl]-5,6,8,8a-tetrahydro-1H-imidazo[1,5-a]pyrazin-7-yl]-6-(2-ethoxy-3-pyridyl)pyridine-2-carboxylic acid (150 mg, 0.281 mmol) in N,N-dimethylformamide (5 mL) were added tetrafluoroboric acid 2-bromo-1-ethylpyridinium (50 mg, 0.422 mmol), N,N-diisopropyl ethylamine (0.1 mL, 0.562 mmol) and (4R)-4-amino-1-methylpyrrolidin-2-one (50 mg) under an argon atmosphere at room temperature. The reaction mixture was stirred at room temperature for 16 hr and water was added. The mixture was extracted with ethyl acetate and the organic layer was separated. The organic layer was washed with saturated aqueous sodium hydrogen carbonate solution and saturated brine, dried over sodium sulfate, and concentrated. The residue was purified by Preparative HPLC to give the title compound (36 mg).

<Preparative HPLC conditions>

Preparative HPLC was performed using an automatic purification device manufactured by Waters. column name: YMC-Triart C18 (100×30 mm, 5 μm), periphery temperature: room temperature, flow rate: 30 mL/min, mobile phase: A=20 mM ammonium bicarbonate aqueous solution, B=acetonitrile.

$^1$H NMR (400 MHz, DMSO-$d_6$) δ 0.80 (3H, t, J = 7.3 Hz), 1.36-1.41 (3H, m), 1.41-1.51 (1H, m), 1.52-1.66 (1H, m), 1.98 (2H, q, J = 8.8 Hz), 2.33-2.45 (3H, m), 2.66 (2H, dd, J = 17.1, 8.6 Hz), 2.76 (3H, s), 3.05-3.16 (3H, m), 3.17-3.23 (1H, m), 3.32 (1H, dd, J = 10.0, 4.5 Hz), 3.46-3.53 (1H, m), 3.56-3.82 (4H, m), 4.49 (2H, q, J = 7.0 Hz), 4.53-4.58 (1H, m), 7.05-7.13 (1H, m), 7.52 (1H, d, J = 8.9 Hz), 8.03 (1H, dd, J = 8.7, 1.6 Hz), 8.16 (1H, dd, J = 4.8, 2.0 Hz), 8.29 (1H, dd, J = 7.5, 2.0 Hz), 8.50 (1H, d, J = 6.6 Hz).
MS: 630.7.

Example 66

3-[(6R,8aS)-6-ethyl-3-oxo-2-[1-(trifluoromethyl)cyclobutyl]-5,6,8,8a-tetrahydro-1H-imidazo[1,5-a]pyrazin-7-yl]-6-(2-ethoxyphenyl)-N-[(3R)-1-methyl-5-oxopyrrolidin-3-yl]pyridine-2-carboxamide

A) 3-[(6R,8aS)-6-ethyl-3-oxo-2-[1-(trifluoromethyl)cyclobutyl]-5,6,8,8a-tetrahydro-1H-imidazo[1,5-a]pyrazin-7-yl]-6-(2-ethoxyphenyl)pyridine-2-carbaldehyde

3-[(6R,8aS)-6-ethyl-3-oxo-2-[1-(trifluoromethyl)cyclobutyl]-5,6,8,8a-tetrahydro-1H-imidazo[1,5-a]pyrazin-7-yl]-6-bromopyridine-2-carbaldehyde (200 mg, 0.421 mmol) was dissolved in a mixture of 1,4-dioxane (9 mL) and water (1 mL), a deaeration treatment was performed, (2-ethoxyphenyl)boronic acid (106 mg, 0.631 mmol) and potassium carbonate (175 mg, 1.262 mmol) were added at 25°C, and the mixture was deaerated again. To this mixture was added Pd(DtBPF)$Cl_2$ (15 mg, 0.021 mmol) under an argon atmosphere, and the reaction mixture was heated at 100°C for 2 hr. After completion of the reaction, the reaction mixture was cooled to room temperature and water (100 mL) was added. The aqueous layer was extracted with ethyl acetate (2x100 mL). The organic layer was washed with saturated brine, dried over anhydrous sodium sulfate, filtered, and the filtrate was concentrated under reduced pressure. The obtained residue was purified by silica gel column chromatography (ethyl acetate/hexane) to give the title compound (200 mg).
MS: 517.1.

B) 3-[(6R,8aS)-6-ethyl-3-oxo-2-[1-(trifluoromethyl)cyclobutyl]-5,6,8,8a-tetrahydro-1H-imidazo[1,5-a]pyrazin-7-yl]-6-(2-ethoxyphenyl)pyridine-2-carboxylic acid

To a mixture of 3-[(6R,8aS)-6-ethyl-3-oxo-2-[1-(trifluoromethyl)cyclobutyl]-5,6,8,8a-tetrahydro-1H-imidazo[1,5-a]pyrazin-7-yl]-6-(2-ethoxyphenyl)pyridine-2-carbaldehyde (200 mg, 0.387 mmol) in tert-butanol (2 mL), tetrahydrofuran (2 mL), water (2 mL) and 2-methyl-2-butene (0.3 mL, 2.78 mmol) were added $NaClO_2$ (88 mg, 0.968 mmol) and $KH_2PO_4$ (106 mg, 0.774 mmol) at 0°C. The reaction mixture was stirred at room temperature for 2 hr. To the reaction mixture were added saturated sodium thiosulfate aqueous solution and saturated ammonium chloride aqueous solution. The mixture was extracted with ethyl acetate and the organic layer was separated. The organic layer was washed with 1N hydrochloric acid, water and saturated brine, dried over sodium sulfate, and concentrated. To the obtained residue were added dichloromethane (1 mL) and 30% ether in pentane solution (10 mL) and the mixture was stirred. The obtained crystal was collected by filtration and washed with pentane to give the title compound (150 mg).
MS: 533.2.

C) 3-[(6R,8aS)-6-ethyl-3-oxo-2-[1-(trifluoromethyl)cyclobutyl]-5,6,8,8a-tetrahydro-1H-imidazo[1,5-a]pyrazin-7-yl]-6-(2-ethoxyphenyl)-N-[(3R)-1-methyl-5-oxopyrrolidin-3-yl]pyridine-2-carboxamide

To a solution of 3-[(6R,8aS)-6-ethyl-3-oxo-2-[1-(trifluoromethyl)cyclobutyl]-5,6,8,8a-tetrahydro-1H-imidazo[1,5-a]pyrazin-7-yl]-6-(2-ethoxyphenyl)pyridine-2-carboxylic acid (150 mg, 0.282 mmol) in N,N-dimethylformamide (5 mL) were added tetrafluoroboric acid 2-bromo-1-ethylpyridinium (50 mg, 0.422 mmol), N,N-diisopropylethylamine (0.1 mL, 0.563 mmol) and (4R)-4-amino-1-methyl-pyrrolidin-2-one (50 mg) under an argon atmosphere at room temperature. The reaction mixture was stirred for 16 hr at room temperature and saturated ammonium chloride aqueous solution was added. The mixture was extracted with ethyl acetate and the organic layer was separated. The organic layer was washed with 1N hydrochloric acid, water and saturated brine, dried over sodium sulfate, and concentrated. The residue was purified by Preparative HPLC to give the title compound (62 mg).

<Preparative HPLC conditions>

Preparative HPLC was performed using an automatic purification device manufactured by Waters. column name: YMC-Triart C18 (100×30 mm, 5 μm), periphery temperature: room temperature, flow rate: 30 mL/min, mobile phase: A=20 mM ammonium bicarbonate aqueous solution, B=acetonitrile.

$^1$H NMR (400 MHz, DMSO-$d_6$) δ 0.80 (3H, t, J = 7.3 Hz), 1.35 (3H, t, J = 6.9 Hz), 1.39-1.50 (1H, m), 1.50-1.64 (1H, m), 1.98 (2H, q, J = 8.7 Hz), 2.32-2.45 (3H, m), 2.60-2.71 (3H, m), 2.75 (3H, s), 3.04-3.16 (3H, m), 3.17-3.27 (1H, m), 3.31 (1H, dd, J = 10.0, 4.5 Hz), 3.44-3.51 (1H, m), 3.56-3.79 (4H, m), 4.13 (2H, q, J = 6.9 Hz), 4.52-4.57 (1H, m), 7.04 (1H, td, J = 7.4, 1.2 Hz), 7.10 (1H, d, J = 8.3 Hz), 7.29-7.37 (1H, m), 7.48 (1H, d, J = 8.7 Hz), 7.82 (1H, dd, J = 7.7, 1.8 Hz), 7.91 (1H, d, J = 8.7 Hz), 8.46 (1H, d, J = 6.4 Hz).
MS: 629.7.

Example 67

3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-(2-ethoxyphenyl)-N-[(3R)-1-methyl-5-oxopyrrolidin-3-yl]pyridine-2-carboxamide

A) 3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-(2-ethoxyphenyl)pyridine-2-carbaldehyde

To a mixture of 3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-bromo-pyridine-2-carbaldehyde (200 mg, 0.376 mmol), (2-ethoxyphenyl)boronic acid (68.7 mg, 0.414 mmol), 2M sodium carbonate aqueous solution (0.564 mL) and toluene (8 mL) was added $PdCl_2$(dppf)-$CH_2Cl_2$ (30.7 mg, 0.0376 mmol) at room temperature. The reaction mixture was stirred at 90°C for 24 hr. After completion of the reaction, the reaction mixture was cooled to 25°C, water and ethyl acetate were added, and the precipitate was filtered. The filtrate was extracted with ethyl acetate, and the obtained organic layer was washed with saturated brine, dried over anhydrous magnesium sulfate, filtered, and the filtrate was concentrated under reduced pressure. The obtained residue was purified by silica gel column chromatography (ethyl acetate/hexane) to give the title compound (80.6 mg).
MS: 573.9.

B) 3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-(2-ethoxyphenyl)-N-[(3R)-1-methyl-5-oxopyrrolidin-3-yl]pyridine-2-carboxamide

To a mixture of 3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-(2-ethoxyphenyl)pyridine-2-carbaldehyde (80.6 mg, 0.141 mmol), sodium phosphate (67.5 mg, 0.563 mmol), 2-methyl-2-butene (0.104 mL, 0.985 mmol), tert-butanol (1 mL), tetrahydrofuran (0.5 mL) and water (0.5 mL) was added $NaClO_2$ (25 mg, 0.281 mmol) at room temperature. The reaction mixture was stirred at room temperature for 3 hr. To the reaction mixture were added saturated sodium thiosulfate aqueous solution and saturated ammonium chloride aqueous solution. The mixture was extracted with ethyl acetate and the organic layer was separated. To the aqueous layer was added sodium chloride, and the mixture was extracted with ethyl acetate again. The combined organic layer was dried over magnesium sulfate and concentrated. The obtained residue was used for the next reaction without further purification.
To a solution of the obtained residue (83.4 mg), N,N-diisopropylethylamine (0.0485 mL, 0.283 mmol), HATU (64.6 mg, 0.17 mmol) in N,N-dimethylformamide (2 mL) was added (4R)-4-amino-1-methyl-pyrrolidin-2-one (20 mg, 0.177 mmol). The reaction mixture was stirred at room temperature for 18 hr and water was added. The mixture was extracted with ethyl acetate and the organic layer was separated. The organic layer was washed with saturated brine, dried over magnesium sulfate, and concentrated. The obtained residue was purified by silica gel column chromatography (ethyl acetate/methanol) and Preparative HPLC to give the title compound (58.4 mg).

$^1$H NMR (300 MHz, DMSO-$d_6$) $\delta$ 0.77 (3H, t, J = 7.3 Hz), 1.30-1.68 (5H, m), 2.36 (1H, dd, J = 16.6, 5.2 Hz), 2.59-2.69 (1H, m), 2.73 (3H, s), 3.02-3.30 (4H, m), 3.35-3.50 (2H, m), 3.66-3.77 (2H, m), 3.79-3.96 (2H, m), 4.06-4.18 (2H, m), 4.36-4.66 (1H, m), 6.97-7.19 (2H, m), 7.28-7.43 (1H, m), 7.50 (1H, d, J = 8.8 Hz), 7.63 (1H, d, J = 7.9 Hz), 7.79-7.91 (3H, m), 7.95 (1H, d, J = 8.7 Hz), 8.82 (1H, d, J = 6.6 Hz).
MS: 685.1.

Preparative HPLC conditions

column: L-Column2 ODS (20 mmI.D.×150 mm, 5 μm)

mobile phase: A) $H_2O$/TFA = 1000/1 (v/v): B) MeCN/TFA = 1000/1 (v/v)
flow rate: 20 ml/min
periphery temperature: room temperature

Example 67a

3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-(2-ethoxyphenyl)pyridine-2-carboxylic acid

The compound was synthesized according to the method shown in Example 10 and Example 38 or a method analogous thereto.

MS:589.2.

Example 68

3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-(2-methoxyphenyl)-N-[(3R)-1-methyl-5-oxopyrrolidin-3-yl]pyridine-2-carboxamide

To a mixture of 3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-bromo-pyridine-2-carbaldehyde (200 mg, 0.376 mmol), (2-methoxyphenyl)boronic acid (62.9 mg, 0.414 mmol), 2M sodium carbonate aqueous solution (0.564 mL) and toluene (8 mL) was added $PdCl_2$(dppf)-$CH_2Cl_2$ (30.7 mg, 0.0376 mmol) at room temperature. The reaction mixture was stirred at 90°C for 24 hr. After completion of the reaction, the reaction mixture was cooled to 25°C, water and ethyl acetate were added, and the precipitate was filtered. The filtrate was extracted with ethyl acetate, and the obtained organic layer was washed with saturated brine, dried over anhydrous magnesium sulfate, filtered, and the filtrate was concentrated under reduced pressure. The obtained residue was purified by silica gel column chromatography (ethyl acetate/hexane). The obtained residue was used for the next reaction without further purification.

To a mixture of the obtained residue (117 mg), sodium phosphate (101 mg, 0.839 mmol), 2-methyl-2-butene (0.156 mL, 1.47 mmol), tert-butanol (1 mL), tetrahydrofuran (0.5 mL) and water (0.5 mL) was added $NaClO_2$ (38 mg, 0.42 mmol) at room temperature. The reaction mixture was stirred at room temperature for 3 hr. To the reaction mixture were added saturated sodium thiosulfate aqueous solution and saturated ammonium chloride aqueous solution. The mixture was extracted with ethyl acetate and the organic layer was separated. To the aqueous layer was added sodium chloride, and the mixture was extracted with ethyl acetate again. The combined organic layer was dried over magnesium sulfate and concentrated. The obtained residue was used for the next reaction without further purification.

To a solution of the obtained residue (122 mg), N,N-diisopropylethylamine (0.0728 mL, 0.425 mmol), HATU (97 mg, 0.255 mmol) in N,N-dimethylformamide (2 mL) was added (4R)-4-amino-1-methylpyrrolidin-2-one (30 mg, 0.266 mmol). The reaction mixture was stirred at room temperature for 18 hr and water was added. The mixture was extracted with ethyl acetate and the organic layer was separated. The organic layer was washed with saturated brine, dried over magnesium sulfate, and concentrated. The obtained residue was purified by silica gel column chromatography (ethyl acetate/methanol) and Preparative HPLC to give the title compound (16 mg).

$^1$H NMR (300 MHz, DMSO-$d_6$) $\delta$ 0.77 (3H, t, J = 7.1 Hz), 1.32-1.67 (2H, m), 2.23-2.41 (2H, m), 2.59-2.69 (1H, m), 2.72 (3H, s), 3.02-3.25 (3H, m), 3.36-3.51 (2H, m), 3.64-3.78 (2H, m), 3.79-3.94 (5H, m), 4.41-4.75 (1H, m), 7.00-7.21 (2H, m), 7.30-7.42 (1H, m), 7.50 (1H, d, J = 8.9 Hz), 7.63 (1H, d, J = 8.2 Hz), 7.74-7.99 (4H, m), 8.73-8.94 (1H, m).
MS: 671.1.

Preparative HPLC conditions

column: L-Column2 ODS (20 mmI.D.×150 mm, 5 μm)

mobile phase: A) $H_2O$/TFA = 1000/1 (v/v): B) MeCN/TFA = 1000/1 (v/v)
flow rate: 20 ml/min
periphery temperature: room temperature

Example 68a

3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-(2-methoxyphenyl)pyridine-2-carboxylic acid

The compound was synthesized according to the method shown in Example 10 and Example 38 or a method analogous thereto.
MS:575.9.

Example 69

3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-(2-methoxy-3-pyridinyl)-N-[(3R)-1-methyl-5-oxopyrrolidin-3-yl]pyridine-2-carboxamide

To a mixture of 3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-bromo-pyridine-2-carbaldehyde (200 mg, 0.38 mmol), (2-methoxy-3-pyridyl)boronic acid (63.3 mg, 0.414 mmol), 2M sodium carbonate aqueous solution (0.564 mL) and toluene (8 mL) was added $PdCl_2(dppf)-CH_2Cl_2$ (30.7 mg, 0.0376 mmol) at room temperature. The reaction mixture was stirred at 90°C for 24 hr. After completion of the reaction, the reaction mixture was cooled to 25°C, water and ethyl acetate were added, and the precipitate was filtered. The filtrate was extracted with ethyl acetate, and the obtained organic layer was washed with saturated brine, dried over anhydrous sodium sulfate, filtered, and the filtrate was concentrated under reduced pressure. The obtained residue was purified by silica gel column chromatography (ethyl acetate/hexane), and the obtained residue was used for the next reaction without further purification.

To a mixture of the obtained residue (116 mg), sodium phosphate (99.4 mg, 0.829 mmol), 2-methyl-2-butene (0.154 mL, 1.45 mmol), tert-butanol (1 mL), tetrahydrofuran (0.5 mL) and water (0.5 mL) was added $NaClO_2$ (38 mg, 0.41 mmol) at room temperature. The reaction mixture was stirred at room temperature for 3 hr. To the reaction mixture were added saturated sodium thiosulfate aqueous solution and saturated ammonium chloride aqueous solution. The mixture was extracted with ethyl acetate and the organic layer was separated. To the obtained aqueous layer was added sodium chloride, and the mixture was extracted with ethyl acetate again. The combined organic layer was dried over magnesium sulfate and concentrated. The obtained residue was used for the next reaction without further purification.

To a solution of the obtained residue (119 mg), N,N-diisopropylethylamine (0.0707 mL, 0.413 mmol), HATU (94 mg, 0.248 mmol) in N,N-dimethylformamide (2 mL) was added (4R)-4-amino-1-methyl-pyrrolidin-2-one (30 mg, 0.259 mmol). The reaction mixture was stirred at room temperature for 18 hr and water was added. The mixture was extracted with ethyl acetate and the organic layer was separated. The organic layer was washed with saturated brine, dried over magnesium sulfate, and concentrated. The obtained residue was purified by silica gel column chromatography (ethyl acetate/methanol) and Preparative HPLC to give the title compound (56.6 mg).

$^1H$ NMR (300 MHz, DMSO-$d_6$) δ 0.77 (3H, t, J = 7.3 Hz), 1.33-1.76 (2H, m), 2.37 (1H, dd, J = 16.6, 5.0 Hz), 2.67 (1H, dd, J = 17.0, 8.7 Hz), 2.73 (3H, s), 3.12-3.31 (4H, m), 3.36 (1H, br d, J = 6.3 Hz), 3.42-3.52 (1H, m), 3.65-3.94 (4H, m), 3.96 (3H, s), 4.44-4.66 (1H, m), 7.15 (1H, dd, J = 7.5, 4.9 Hz), 7.54 (1H, d, J = 8.8 Hz), 7.63 (1H, d, J = 8.0 Hz), 7.84-7.93 (2H, m), 8.00 (1H, d, J = 8. 7 Hz), 8.21 (1H, dd, J = 4.9, 2. 0 Hz), 8.30 (1H, dd, J = 7.4, 1.9 Hz), 8.86 (1H, d, J = 6.6 Hz). MS: 672.2.

Preparative HPLC conditions

column: L-Column2 ODS (20 mmI.D.×150 mm, 5 μm)
mobile phase: A) $H_2O$/TFA = 1000/1 (v/v): B) MeCN/TFA = 1000/1 (v/v)
flow rate: 20 ml/min
periphery temperature: room temperature

Example 69a

3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-(2-methoxy-3-pyridinyl)pyridine-2-carboxylic acid

The compound was synthesized according to the method shown in Example 10 and Example 38 or a method analogous thereto.
MS:576.1.

Example compounds described above are shown in the following Tables 2-1 to Table 2-4. MS in the Tables indicates actual values.

[Table 2-1]

| | | | |
|---|---|---|---|
| Example 59 | 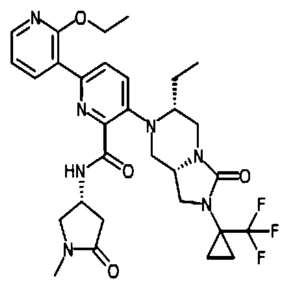 | 3-[(6R,8aS)-6-ethyl-3-oxo-2-[1-(trifluoromethyl)cyclopropyl]-5, 6, 8, 8a-tetrahydro-1H-imidazo[1, 5-a]pyrazin-7-yl]-6-(2-ethoxy-3-pyridinyl)-N-[(3R)-1-methyl-5-oxopyrrol idin-3-yl]pyridine-2-carboxamide | 616.4 |
| Example 60 | 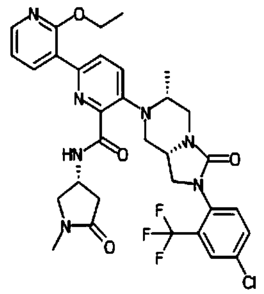 | 3-[(6R,8aS)-2-[4-chloro-2-(trifluoromethyl)phenyl]-6-methyl-3-oxo-5, 6, 8, 8a-tetrahydro-1H-imidazo[1,5-a]pyrazin-7-yl]-6-(2-ethoxy-3-pyridinyl)-N-[(3R)-1-methyl-5-oxopyrrolidin-3-yl]pyridine-2-carboxamide | 672.4 |
| Example 60a | 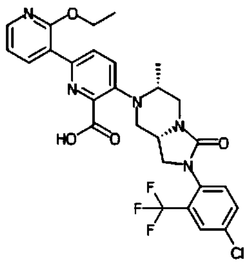 | 3-[(6R,8aS)-2-[4-chloro-2-(trifluoromethyl)phenyl]-6-methyl-3-oxo-5, 6, 8, 8a-tetrahydro-1H-imidazo[1,5-a]pyrazin-7-yl]-6-(2-ethoxy-3-pyridinyl)pyridine-2-carboxylic acid | 576.3 |
| Example 61 | 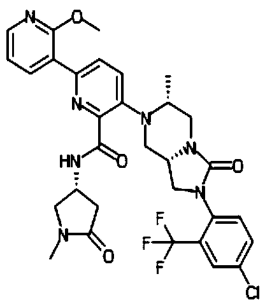 | 3-[(6R,8aS)-2-[4-chloro-2-(trifluoromethyl)phenyl]-6-methyl-3-oxo-5, 6, 8, 8a-tetrahydro-1H-imidazo[1, 5-a]pyrazin-7-yl]-6-(2-methoxy-3-pyridinyl)-N-[(3R)-1-methyl-5-oxopyrrolidin-3-yl]pyridine-2-carboxamide | 658.4 |
| Example 61a | 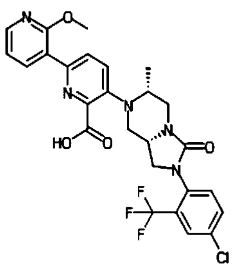 | 3-[(6R,8aS)-2-[4-chloro-2-(trifluoromethyl)phenyl]-6-methyl-3-oxo-5, 6, 8, 8a-tetrahydro-1H-imidazo[1, 5-a]pyrazin-7-yl]-6-(2-methoxy-3-pyridyl)pyridine-2-caboxylic acid | 562.1 |

[Table 2-2]

| | | | |
|---|---|---|---|
| Example 62 | 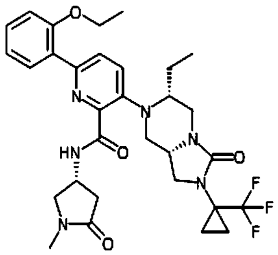 | 3-[(6R,8aS)-6-ethyl-3-oxo-2-[1-(trifluoromethyl)cyclopropyl]-5, 6, 8, 8a-tetrahydro-1H-imidazo[1, 5-a]pyrazin-7-yl]-6-(2-ethoxyphenyl)-N-[(3R)-1-methyl-5-oxopyrrol idin-3-yl]pyridine-2-carboxamide | 615.5 |
| Example 63 | 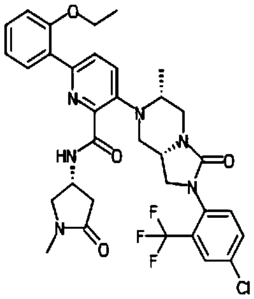 | 3-[(6R,8aS)-2-[4-chloro-2-(trifluoromethyl)phenyl]-6-methyl-3-oxo-5, 6, 8, 8a-tetrahydro-1H-imidazo[1,5-a]pyrazin-7-yl]-6-(2-ethoxyphenyl)-N-[(3R)-1-methyl-5-oxopyrrol idin-3-yl]pyridine-2-carboxamide | 671.7 |
| Example 63a | 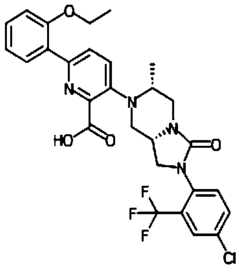 | 3-[(6R,8aS)-2-[4-chloro-2-(trifluoromethyl)phenyl]-6-methyl-3-oxo-5, 6, 8, 8a-tetrahydro-1H-imidazo[1,5-a]pyrazin-7-yl]-6-(2-ethoxyphenyl)pyridine-2-carboxylic acid | 575.3 |
| Example 64 | 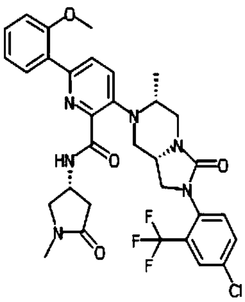 | 3-[(6R,8aS)-2-[4-chloro-2-(trifluoromethyl)phenyl]-6-methyl-3-oxo-5,6,8,8a-tetrahydro-1H-imidazo[1, 5-a]pyrazin-7-yl]-6-(2-methoxyphenyl)-N-[(3R)-1-methyl-5-oxopyrrolidin-3-yl]pyridine-2-carboxamide | 657.6 |
| Example 64a | 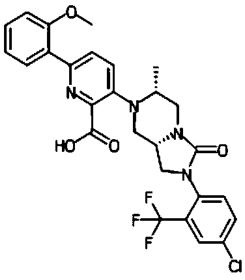 | 3-[(6R,8aS)-2-[4-chloro-2-(trifluoromethyl)phenyl]-6-methyl-3-oxo-5, 6, 8, 8a-tetrahydro-1H-imidazo[1, 5-a]pyrazin-7-yl]-6-(2-methoxyphenyl)pyridine-2-carboxylic acid | 561.2 |

[Table 2 -3]

| | | | |
|---|---|---|---|
| Example 65 | 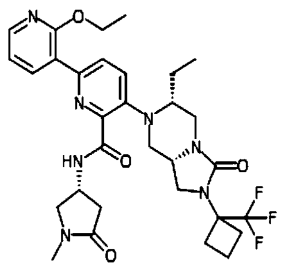 | 3-[(6R,8aS)-6-ethyl-3-oxo-2-[1-(trifluoromethyl)cyclobutyl]-5, 6, 8, 8a-tetrahydro-1H-imidazo[1,5-a]pyrazin-7-yl]-6-(2-ethoxy-3-pyridinyl)-N-[(3R)-1-methyl-5-oxopyrrolidin-3-yl]pyridine-2-carboxamide | 630.7 |
| Example 66 | 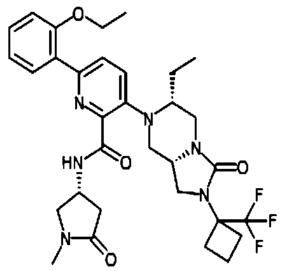 | 3-[(6R,8aS)-6-ethyl-3-oxo-2-[1-(trifluoromethyl)cyclobutyl]-5, 6, 8, 8a-tetrahydro-1H-imidazo[1, 5-a]pyrazin-7-yl]-6-(2-ethoxyphenyl)-N-[(3R)-1-methyl-5-oxopyrrol idin-3-yl]pyridine-2-carboxamide | 629.7 |
| Example 67 | 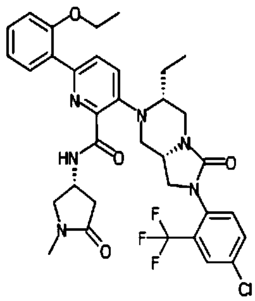 | 3-[(6R,8aS)-2-[4-chloro-2-(trifluoromethyl)phenyl]-6-ethyl-3-oxo-5,6,8,8a-tetrahydro-1H-imidazo[1, 5-a]pyrazin-7-yl]-6-(2-ethoxyphenyl)-N-[(3R)-1-methyl-5-oxopyrrol idin-3-yl]pyridine-2-carboxamide | 685.1 |
| Example 67a | 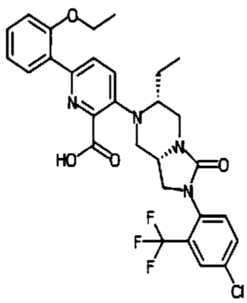 | 3-[(6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-(2-ethoxyphenyl)pyridine-2-carboxylic acid | 589.2 |

[Table 2 -4]

| | | | |
|---|---|---|---|
| Example 68 | 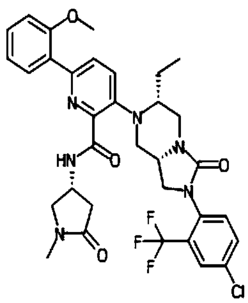 | 3-[(6R,8aS)-2-[4-chloro-2-(trifluoromethyl)phenyl]-6-ethyl-3-oxo-5, 6, 8, 8a-tetrahydro-1H-imidazo[1,5-a]pyrazin-7-yl]-6-(2-methoxyphenyl)-N-[(3R)-1-methyl-5-oxopyrrol idin-3-yl]pyridine-2-carboxamide | 671.1 |

(continued)

| | | | |
|---|---|---|---|
| Example 68a | 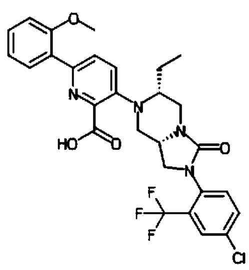 | 3-[(6R,8aS)-2-[4-chloro-2-(trifluoromethyl)phenyl]-6-ethyl-3-oxo-5, 6, 8, 8a-tetrahydro-1H-imidazo[1,5-a]pyrazin-7-yl]-6-(2-methoxyphenyl)pyridine-2-carboxylic acid | 575.9 |
| Example 69 | 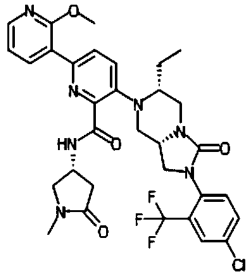 | 3-[(6R, 8aS)-2-[4-chloro-2-(trifluoromethyl)phenyl]-6-ethyl-3-oxo-5, 6, 8, 8a-tetrahydro-1H-imidazo[1, 5-a]pyrazin-7-yl]-6-(2-methoxy-3-pyridinyl)-N-[(3R)-1-methyl-5-oxopyrrolidin-3-yl] pyridine-2-carboxamide | 672.2 |
| Example 69a | 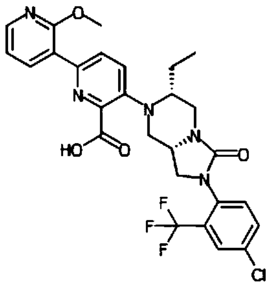 | 3-[(6R, 8aS)-2-[4-chloro-2-(trifluoromethyl)phenyl]-6-ethyl-3-oxo-5, 6, 8, 8a-tetrahydro-1H-imidazo[1,5-a]pyrazin-7-yl]-6-(2-methoxy-3-pyridinyl)pyridine-2-carboxyl ic acid | 576.1 |

Experimental Example 1: Measurement of human MC2R inhibitory activity using intracellular cAMP concentration as an indicator

GeneBLAzer MC2R-CRE-bla CHO-K1 cells (Thermo Fisher Scientific) that had been cryopreserved in advance were thawed and suspended in a medium [Dulbecco's Modified Eagle Medium (Thermo Fisher Scientific) containing fetal bovine serum (10% (v/v), HyClone Laboratories), Non-Essential Amino Acids Solution (1/100 (w/w), Thermo Fisher Scientific), HEPES (25 mM, Thermo Fisher Scientific), Penicillin/Streptomycin (1/100 (w/w), Fujifilm Wako)]. After centrifuging the cell suspension, the supernatant was removed, the cells were suspended in D-PBS(-) (Fujifilm Wako), and the supernatant was removed again after centrifugation. The cells were resuspended in an assay buffer [Hank's Balanced Salt Solution (Thermo Fisher Scientific) containing HEPES pH 7.5 (10 mM, Thermo Fisher Scientific), fatty acid-free bovine serum albumin (0.01% (w/v), Sigma), IBMX (500 μM, Fujifilm Wako)] and added to a 384-well white plate (Greiner) at 1,500 cells/well. Then, the test compound prepared using the assay buffer was added, and after standing for 20 min, an assay buffer containing ACTH (Human 1-24) (Peptide Institute) was added (ACTH final concentration, 3 pM). After standing at room temperature for 30 min, the intracellular cAMP level was measured using cAMP Gs HiRange kit HTRF (PerkinElmer). The inhibition rate (%) of the compound against MC2R was calculated using the following formula.

$$\text{Inhibition rate (\%)} = (C-B)/(A-B) \times 100$$

A: cAMP concentration calculated from wells containing 3 pM ACTH (1-24)
B: cAMP concentration calculated from wells without addition of 3 pM ACTH (1-24)
C: cAMP concentration calculated from wells containing 3 pM ACTH (1-24) and test compound at concentration determined in advance

The inhibition rate (%) of the test compound against MC2R at 1 μM is shown in Table 3-1 to Table 3-4.

[Table 3-1]

| test compound (Ex. No.) | MC2R inhibition rate (% of inhibition) |
|---|---|
| 1 | 100 |
| 2 | 99 |
| 3 | 81 |
| 4 | 100 |
| 5 | 100 |
| 6 | 100 |
| 7 | 91 |
| 8 | 90 |
| 9 | 99 |
| 10 | 100 |
| 11 | 97 |
| 12 | 100 |
| 13 | 99 |
| 14 | 100 |
| 15 | 100 |
| 16 | 100 |
| 17 | 91 |
| 18 | 100 |
| 19 | 100 |
| 20 | 99 |

[Table 3-2]

| | |
|---|---|
| 21 | 82 |
| 22 | 100 |
| 23 | 100 |
| 24 | 100 |
| 25 | 99 |
| 26 | 100 |
| 27 | 100 |
| 28 | 100 |
| 29 | 100 |
| 30 | 100 |
| 31 | 100 |
| 32 | 100 |
| 33 | 100 |
| 34 | 96 |
| 35 | 100 |
| 36 | 100 |
| 37 | 100 |

(continued)

| | |
|---|---|
| 38 | 100 |
| 38a | 91 |
| 39 | 100 |

[Table 3-3]

| | |
|---|---|
| 40 | 100 |
| 41 | 100 |
| 42 | 100 |
| 43 | 100 |
| 44 | 100 |
| 45 | 100 |
| 46 | 100 |
| 47 | 100 |
| 48 | 100 |
| 49 | 100 |
| 50 | 100 |
| 51 | 100 |
| 52 | 100 |
| 53 | 100 |
| 54 | 100 |
| 55 | 100 |
| 56 | 100 |
| 57 | 100 |
| 58 | 100 |
| 59 | 87 |

[Table 3-4]

| | |
|---|---|
| 60 | 97 |
| 61 | 97 |
| 62 | 98 |
| 63 | 100 |
| 64 | 95 |
| 65 | 89 |
| 66 | 97 |
| 67 | 94 |
| 68 | 96 |
| 69 | 92 |

As described above, it was shown that the present compound has a superior MC2R inhibitory action.

Experimental Example 2: Suppressive action of ACTH-induced corticosterone secretion in mice

Nine-week-old male ICR mice (CHARLES RIVER LABORATORIES JAPAN, INC.) were used in the test.

The mice were assigned to each group based on body weight, with 5 mice per group. Test compounds were suspended in 0.5% (w/v) methylcellulose solution. ACTH (1-24) (A0948, LKT Laboratories, Inc.) was dissolved in saline. The test compound (10 mg/kg) or vehicle was orally administered to mice, and 1 hr later, ACTH solution (100 μg/kg) was administered subcutaneously. Blood was collected from the tail vein before ACTH administration and 0.5, 1, and 2 hr after administration, and corticosterone concentration in plasma was measured using an ELISA kit (EC3001-1, Assaypro). Using the plasma corticosterone concentration at each time point, the area under the blood concentration-time curve up to 2 hr after administration ($AUC_{0-2h}$) was calculated.

The ratio of $AUC_{0-2h}$ in the test compound group to $AUC_{0-2h}$ in the vehicle administration group is shown in Table 4.

[Table 4]

| Example No. (10 mg/kg, *p.o.*) | plasma corticosterone $AUC_{0-2h}$ (% of vehicle, mean±S.D.) |
|---|---|
| 30 | 8.6 ± 2.4 |
| 31 | 61.1 ± 7.3 |
| 34 | 15.6 ± 4.9 |
| 37 | 14.0 ± 1.8 |
| 38 | 14.2 ± 4.3 |
| 47 | 17.5 ± 4.4 |
| 49 | 7.7 ± 2.5 |

As mentioned above, it was shown that the present compound has a superior suppressive action on ACTH-induced corticosteron secretion.

The Formulation Examples containing the compound of Example 1 are shown below to explain the preparations of the present compound. However, the embodiment of the preparation of the present compound is not limited to these.

Formulation Example 1 (production of capsule)

| | | | |
|---|---|---|---|
| 1) | compound of Example 1 | | 30 mg |
| 2) | finely-powdered cellulose | | 10 mg |
| 3) | lactose | | 19 mg |
| 4) | magnesium stearate | | 1 mg |
| | | total | 60 mg |

1), 2), 3) and 4) are mixed and filled in a gelatin capsule.

Formulation Example 2 (production of tablets)

| | | | |
|---|---|---|---|
| 1) | compound of Example 1 | | 30 g |
| 2) | lactose | | 50 g |
| 3) | cornstarch | | 15 g |
| 4) | calcium carboxymethylcellulosese | | 44 g |
| 5) | magnesium stearate | | 1 g |
| | 1000 tablets | total | 140 g |

The total amount of 1), 2) and 3) and 30 g of 4) is kneaded with water, vacuum dried, and sieved. The sieved

powder is mixed with 4) (14 g) and 5) (1 g), and the mixture is punched by a tableting machine, whereby 1000 tablets containing 30 mg of the compound of Example 1 per tablet are obtained.

[Industrial Applicability]

The present invention provides, as one embodiment thereof, a compound having a superior MC2R antagonist action and useful in the pharmaceutical field as a prophylactic or therapeutic agent for diseases involving ACTH signal abnormalities, particularly, endocrine diseases (e.g., Cushing's disease, ectopic ACTH syndrome, ectopic CRH syndrome, congenital adrenal hyperplasia, or polycystic ovary syndrome), metabolic diseases (e.g., obesity, type 2 diabetes, dyslipidemia, osteoporosis, or sarcopenia), cardiovascular diseases (e.g., hypertension, arteriosclerosis), central nervous system diseases (e.g., depression, dementia, insomnia), eye diseases (e.g., cataract, glaucoma), and infectious diseases.

This application is based on a patent application No. 2023-80580 filed in Japan (filing date: May 16, 2023), the contents of which are incorporated in full herein.

## Claims

1. A compound represented by the following formula (I):

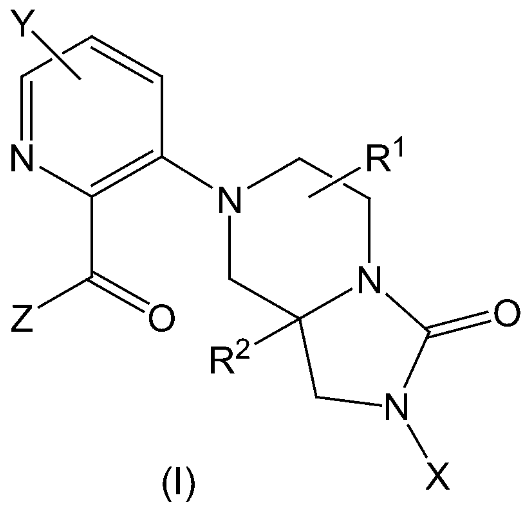

(I)

wherein

$R^1$ is a hydrogen atom or an optionally substituted $C_{1-6}$ alkyl group,
$R^2$ is a hydrogen atom or an optionally substituted $C_{1-6}$ alkyl group,
X is an optionally substituted aryl group, an optionally substituted aromatic heterocyclic group, or an optionally substituted saturated hydrocarbon ring group,
Y is an optionally substituted aromatic heterocyclic group or an optionally substituted aryl group, and
Z is a hydrogen atom, a hydroxy group, $NH_2$, or $NHR^3$
wherein $R^3$ is an optionally substituted $C_{1-6}$ alkyl group or an optionally substituted saturated ring group,
or a salt thereof.

2. The compound according to claim 1, wherein X is an optionally substituted aryl group, an optionally substituted nitrogen-containing aromatic heterocyclic group, or an optionally substituted saturated hydrocarbon ring group, and Y is an optionally substituted nitrogen-containing aromatic heterocyclic group or an optionally substituted aryl group, or a salt thereof.

3. The compound according to claim 2, wherein X is an optionally substituted $C_{6-14}$ aryl group, an optionally substituted 5- to 14-membered nitrogen-containing aromatic heterocyclic group, or an optionally substituted saturated hydrocarbon ring group, and

Y is an optionally substituted 5- to 14-membered nitrogen-containing aromatic heterocyclic group or an optionally

substituted $C_{6-14}$ aryl group,
or a salt thereof.

4. The compound according to claim 3, wherein X is an optionally substituted phenyl group, an optionally substituted pyridyl group, or an optionally substituted saturated hydrocarbon ring group, and

Y is an optionally substituted pyridyl group or an optionally substituted phenyl group,
or a salt thereof.

5. The compound according to claim 4, wherein X is an optionally substituted phenyl group or an optionally substituted saturated hydrocarbon ring group, and

Y is an optionally substituted pyridyl group or an optionally substituted phenyl group,
or a salt thereof.

6. The compound according to any one of claims 3 to 5,
wherein the "optionally substituted saturated hydrocarbon ring group" for X is an "optionally substituted $C_{3-10}$ cycloalkyl group", or a salt thereof.

7. The compound according to claim 5, wherein X is an optionally substituted phenyl group or an optionally substituted $C_{3-10}$ cycloalkyl group,
or a salt thereof.

8. The compound according to claim 1, wherein Z is a hydrogen atom, a hydroxy group, $NH_2$, or $NHR^3$

wherein $R^3$ is an optionally substituted $C_{1-6}$ alkyl group or an optionally substituted 3- to 8-membered saturated ring group,
or a salt thereof.

9. The compound according to claim 1, wherein $R^1$ is a hydrogen atom or a $C_{1-6}$ alkyl group,

$R^2$ is a hydrogen atom or a $C_{1-6}$ alkyl group,
X is a phenyl group optionally substituted by 1 to 3 substituents selected from 1) a halogen atom, 2) a $C_{1-6}$ alkyl group optionally substituted by 1 to 3 halogen atoms, and 3) a cyano group, or
a $C_{3-10}$ cycloalkyl group optionally substituted by 1 to 3 $C_{1-6}$ alkyl groups optionally substituted by 1 to 3 halogen atoms,
Y is a pyridyl group optionally substituted by 1 to 3 $C_{1-6}$ alkoxy groups, or a phenyl group optionally substituted by 1 to 3 $C_{1-6}$ alkoxy groups, and
Z is a hydrogen atom, a hydroxy group, $NH_2$, or
$NHR^3$
wherein $R^3$ is

(i) a $C_{1-6}$ alkyl group optionally substituted by 1 to 3 substituents selected from

a hydroxy group,
a $C_{1-6}$ alkoxy group,
a (mono- or di-$C_{1-6}$ alkyl)amino group,
a $C_{3-10}$ cycloalkyl group,
a 5- or 6-membered monocyclic aromatic heterocyclic group,
a 3- to 8-membered monocyclic non-aromatic heterocyclic group optionally substituted by a $C_{1-6}$ alkoxy-carbonyl group,
a $C_{1-6}$ alkyl-carbonylamino group,
a (mono- or di-$C_{1-6}$ alkylamino)carbonyl group, and
a $C_{1-6}$ alkyl-sulfonyl group, or

(ii) a 3- to 8-membered monocyclic saturated ring group optionally substituted by 1 to 3 substituents selected from

a $C_{1-6}$ alkyl group,
a $C_{1-6}$ alkoxy-carbonyl group, and
an oxo group,
or a salt thereof.

**10.** The compound according to claim 9, wherein X is a phenyl group optionally substituted by 1 to 3 substituents selected from 1) a halogen atom, 2) a $C_{1-6}$ alkyl group optionally substituted by 1 to 3 halogen atoms, and 3) a cyano group, and

Y is a pyridyl group optionally substituted by 1 to 3 $C_{1-6}$ alkoxy groups,
or a salt thereof.

**11.** The compound according to claim 9, wherein $R^1$ is a $C_{1-6}$ alkyl group, and

$R^2$ is a hydrogen atom,
or a salt thereof.

**12.** The compound according to claim 9, wherein Z is a hydrogen atom, a hydroxy group, $NH_2$, or

$NHR^3$
wherein $R^3$ is

(i) a $C_{1-6}$ alkyl group optionally substituted by 1 to 3 substituents selected from

a hydroxy group,
a $C_{1-6}$ alkoxy group,
a (mono- or di-$C_{1-6}$ alkyl)amino group,
a $C_{3-10}$ cycloalkyl group,
a 5- or 6-membered monocyclic nitrogen-containing aromatic heterocyclic group,
a 3- to 8-membered monocyclic oxygen-containing non-aromatic heterocyclic group,
a 3- to 8-membered monocyclic nitrogen-containing non-aromatic heterocyclic group optionally substituted by a $C_{1-6}$ alkoxy-carbonyl group,
a $C_{1-6}$ alkyl-carbonylamino group,
a (mono- or di-$C_{1-6}$ alkylamino)carbonyl group, and
a $C_{1-6}$ alkyl-sulfonyl group, or

(ii)

(a) a 3- to 8-membered monocyclic oxygen-containing saturated heterocyclic group, or
(b) a 3- to 8-membered monocyclic nitrogen-containing saturated heterocyclic group optionally substituted by 1 to 3 substituents selected from

a $C_{1-6}$ alkyl group,
a $C_{1-6}$ alkoxy-carbonyl group, and
an oxo group,
or a salt thereof.

**13.** The compound according to claim 12, wherein Z is a hydroxy group or $NHR^3$
wherein $R^3$ is

(a) a 3- to 8-membered monocyclic oxygen containing saturated heterocyclic group, or
(b) a 3- to 8-membered nitrogen-containing saturated heterocyclic group optionally substituted 1 to 3 substituents selected from

a $C_{1-6}$ alkyl group,
a $C_{1-6}$ alkoxy-carbonyl group, and
an oxo group,
or a salt thereof.

14. The compound according to claim 9, wherein $R^1$ is a $C_{1-6}$ alkyl group,

$R^2$ is a hydrogen atom,
X is a phenyl group optionally substituted by 1 to 3 substituents selected from 1) a halogen atom, 2) a $C_{1-6}$ alkyl group optionally substituted by 1 to 3 halogen atoms, and 3) a cyano group,
Y is a pyridyl group optionally substituted by 1 to 3 $C_{1-6}$ alkoxy groups, and
Z is a hydroxy group, or $NHR^3$
wherein $R^3$ is a pyrrolidinyl group optionally substituted by 1 to 3 substituents selected from a $C_{1-6}$ alkyl group and an oxo group,
or a salt thereof.

15. The compound according to any one of claims 1 to 5 and 7 to 14, wherein compound (I) has a structure represented by the following formula (Ia):

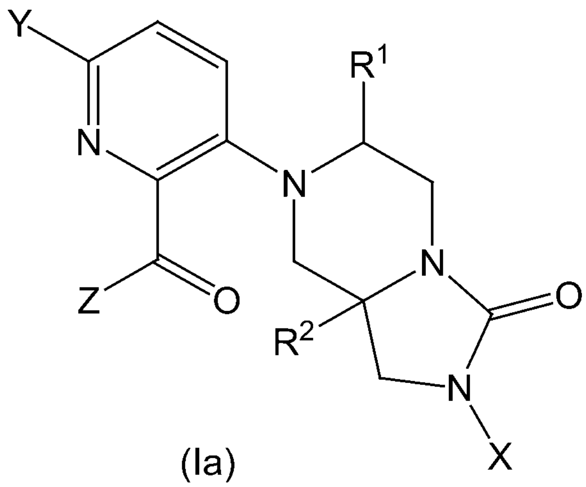

(Ia)

,

or a salt thereof.

16. The compound according to claim 1 or a salt thereof, which is selected from

3-[ (6R,8aS)-2-[4-chloro-2-(trifluoromethyl)phenyl]-6-ethyl-3-oxo-5,6,8,8a-tetrahydro-1H-imidazo[1,5-a]pyrazin-7-yl]-6-(2-ethoxypyridin-3-yl)-N-[(3R)-1-methyl-5-oxopyrrolidin-3-yl]pyridine-2-carboxamide,
3-[ (6R,8aS)-6-ethyl-2-[4-fluoro-2-(trifluoromethyl)phenyl]-3-oxo-5,6,8,8a-tetrahydro-1H-imidazo[1,5-a]pyrazin-7-yl]-6-(2-ethoxypyridin-3-yl)-N-[(3R)-1-methyl-5-oxopyrrolidin-3-yl]pyridine-2-carboxamide,
3-[(6R,8aS)-2-(4-chloro-2-cyanophenyl)-6-ethyl-3-oxo-5,6,8,8a-tetrahydro-1H-imidazo[1,5-a]pyrazin-7-yl]-6-(2-ethoxypyridin-3-yl)-N-[ (3R)-1-methyl-5-oxopyrrolidin-3-yl]pyridine-2-carboxamide,
3-[ (6R,8aS)-2-[4-chloro-2-(trifluoromethyl)phenyl]-6-methyl-3-oxo-5,6,8,8a-tetrahydro-1H-imidazo[1,5-a]pyrazin-7-yl]-6-(2-methoxy-3-pyridinyl)-N-[(3R)-1-methyl-5-oxopyrrolidin-3-yl]pyridine-2-carboxamide,
3-[ (6R,8aS)-2-[4-chloro-2-(trifluoromethyl)phenyl]-6-methyl-3-oxo-5,6,8,8a-tetrahydro-1H-imidazo[1,5-a]pyrazin-7-yl]-6-(2-methoxyphenyl)-N-[ (3R)-1-methyl-5-oxopyrrolidin-3-yl]pyridine-2-carboxamide, or
a salt of each compound mentioned above.

17. A medicament comprising the compound according to claim 1, or a salt thereof as an active ingredient.

18. The medicament according to claim 17, which is an MC2R antagonist.

19. The medicament according to claim 17 or 18, which is a prophylactic or therapeutic agent for endocrine disease, metabolic disease, cardiovascular disease, central nervous system disease, eye disease, and/or infectious disease.

20. The medicament according to claim 19, which is a prophylactic or therapeutic agent for Cushing's disease, ectopic ACTH syndrome, ectopic CRH syndrome, congenital adrenal hyperplasia, polycystic ovary syndrome, obesity, type 2 diabetes, dyslipidemia, osteoporosis, sarcopenia, hypertension, arteriosclerosis, depression, dementia, insomnia, cataract, glaucoma and/or infectious disease.

**21.** A method for the prophylaxis and/or treatment of endocrine disease, metabolic disease, cardiovascular disease, central nervous system disease, eye disease, and/or infectious disease, comprising administering an effective amount of the compound according to claim 1, or a salt thereof to a mammal in need of the administration.

**22.** The compound according to claim 1 or a salt thereof, which is for use for the prophylaxis and/or treatment of endocrine disease, metabolic disease, cardiovascular disease, central nervous system disease, eye disease, and/or infectious disease.

**23.** Use of the compound according to claim 1 or a salt thereof in the production of a medicament for the prophylaxis and/or treatment of endocrine disease, metabolic disease, cardiovascular disease, central nervous system disease, eye disease, and/or infectious disease.

## INTERNATIONAL SEARCH REPORT

International application No.
**PCT/JP2024/017921**

### A. CLASSIFICATION OF SUBJECT MATTER

***C07D 487/04***(2006.01)i; ***A61K 31/4985***(2006.01)i; ***A61P 3/00***(2006.01)i; ***A61P 3/04***(2006.01)i; ***A61P 3/10***(2006.01)i; ***A61P 5/00***(2006.01)i; ***A61P 5/38***(2006.01)i; ***A61P 9/00***(2006.01)i; ***A61P 9/10***(2006.01)i; ***A61P 9/12***(2006.01)i; ***A61P 15/00***(2006.01)i; ***A61P 19/10***(2006.01)i; ***A61P 25/00***(2006.01)i; ***A61P 25/24***(2006.01)i; ***A61P 27/02***(2006.01)i; ***A61P 27/06***(2006.01)i; ***A61P 27/12***(2006.01)i; ***A61P 31/00***(2006.01)i; ***A61P 43/00***(2006.01)i
FI: C07D487/04 144; A61P5/38; A61P27/06; A61P27/12; A61P25/24; A61P9/10 101; A61P9/12; A61P19/10; A61P3/10; A61P3/04; A61P15/00; A61P31/00; A61P27/02; A61P25/00; A61P9/00; A61P3/00; A61P5/00; A61P43/00 111; A61K31/4985; C07D487/04 CSP

According to International Patent Classification (IPC) or to both national classification and IPC

### B. FIELDS SEARCHED

Minimum documentation searched (classification system followed by classification symbols)

C07D487/04; A61K31/4985; A61P3/00; A61P3/04; A61P3/10; A61P5/00; A61P5/38; A61P9/00; A61P9/10; A61P9/12; A61P15/00; A61P19/10; A61P25/00; A61P25/24; A61P27/02; A61P27/06; A61P27/12; A61P31/00; A61P43/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY (STN)

### C. DOCUMENTS CONSIDERED TO BE RELEVANT

| Category* | Citation of document, with indication, where appropriate, of the relevant passages | Relevant to claim No. |
|---|---|---|
| A | JP 2021-528375 A (CRINETICS PHARMACEUTICALS, INC.) 21 October 2021 (2021-10-21)<br>claims, examples | 1-23 |
| A | JP 2022-553832 A (CRINETICS PHARMACEUTICALS, INC.) 26 December 2022 (2022-12-26)<br>claims, examples | 1-23 |
| A | JP 2004-501917 A (PFIZER PRODUCTS INC.) 22 January 2004 (2004-01-22)<br>claims, examples, scheme 8 | 1-23 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **08 July 2024** | **23 July 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**

International application No.
**PCT/JP2024/017921**

**C. DOCUMENTS CONSIDERED TO BE RELEVANT**

| Category* | Citation of document, with indication, where appropriate, of the relevant passages | Relevant to claim No. |
|---|---|---|
| P, A | KIM, Sun Hee et al. Discovery of CRN04894: A Novel Potent Selective MC2R Antagonist, ACS Medicinal Chemistry Letters, 2024, 15(4), pp. 478-485, DOI: 10.1021/acsmedchemlett.3c00514<br>entire text | 1-23 |

Form PCT/ISA/210 (second sheet) (July 2022)

**INTERNATIONAL SEARCH REPORT**
**Information on patent family members**

International application No.
**PCT/JP2024/017921**

| Patent document cited in search report | Publication date (day/month/year) | Patent family member(s) | Publication date (day/month/year) |
|---|---|---|---|
| JP 2021-528375 A | 21 October 2021 | WO 2019/236699 A1<br>claims, examples<br>JP 2024-9837 A<br>US 2019/0367481 A1<br>US 2020/0010452 A1<br>US 2020/0216415 A1<br>US 2021/0238164 A1<br>US 2021/0002254 A1<br>US 2024/0109866 A1<br>EP 3802500 A1<br>KR 10-2021-0005995 A<br>CN 112533904 A<br>TW 202016089 A<br>CA 3101644 A<br>AU 2019280689 A<br>SG 11202012071T A<br>IL 279152 A<br>BR 112020024577 A<br>MA 52806 A<br>MX 2020013085 A<br>ZA 202007343 B<br>EA 202092699 A | |
| JP 2022-553832 A | 26 December 2022 | US 2023/0015914 A1<br>WO 2021/091788 A1<br>EP 4054583 A1 | |
| JP 2004-501917 A | 22 January 2004 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2019236699 A
- JP 2023080580 A


### Non-patent literature cited in the description


- *Front Endocrinol*, 05 August 2016, vol. 7, 101
- Courses in Experimental Chemistry. Chemical Society of Japan, vol. 13-19
- **L. F. TIETZE** ; **TH. EICHER** ; **NANKODO**. Fine Organic Chemistry rev.
- **HIDEO TOGO** ; **KODANSHA**. rev. Organic Name Reactions, Their Mechanism and Essence
- ORGANIC SYNTHESES Collective. John Wiley & Sons Inc, vol. 1-7
- **JIE JACK LI**. Modern Organic Synthesis in the Laboratory, A Collection of Standard Experimental Procedures. OXFORD UNIVERSITY
- Comprehensive Heterocyclic Chemistry III. Elsevier Japan KK, vol. 1-14
- **KIYOSHI TOMIOKA** ; **KAGAKUDOJIN**. Strategic Applications of Named Reactions in Organic Synthesis
- Comprehensive Organic Transformations. VCH Publishers Inc., 1989
- **THEODORA W. GREENE** ; **PETER G. M. WUTS**. Protective Groups in Organic Synthesis. Wiley-Interscience, 2007
- **P. J. KOCIENSKI**. Protecting Groups. Thieme, 2004
- Courses in Experimental Chemistry. Chemical Society of Japan, vol. 14, 251-253
- Jikken Kagaku Kouza. Chemical Society of Japan, vol. 19, 273-274
- Development of Pharmaceuticals. Design of Molecules. HIROKAWA SHOTEN, 1990, vol. 7, 163-198